# EUROPEAN PATENT SPECIFICATION

(11) **EP 0 420 194 B1**
(45) Date of publication and mention of the grant of the patent: **29.05.1996**
(21) Application number: 90118479.6
(22) Date of filing: 26.09.1990
(51) Int. Cl.: C07D 413/04, C07D 417/04, C07D 403/04, A01N 43/84, A01N 43/76, A01N 43/78, A01N 43/60

(54) **Uracil derivatives and their production and use**
Uracilderivate, ihre Herstellung und ihre Verwendung
Dérivés d'uraciles, leur préparation et leur utilisation

(30) Priority: 26.09.1989 JP 250320/89; 02.10.1989 JP 258261/89; 24.05.1990 JP 136498/90; 06.07.1990 JP 180091/90
(43) Date of publication of application: 03.04.1991
(73) Proprietor: SUMITOMO CHEMICAL COMPANY, LIMITED, Chuo-ku Osaka 541 (JP)
(72) Inventor: Enomoto, Masayuki, Nishinomiya, Hyogo (JP); Nagano, Eiki, Itami, Hyogo (JP); Sato, Ryo, Toyonaka, Osaka (JP); Sakaki, Masaharu, Toyonaka, Osaka (JP)
(74) Representative: VOSSIUS & PARTNER

(56) References cited:
- EP-A- 0 311 135
- WO-A-90/15057

## Description

The present invention relates to uracil derivatives, and their production and use. More particularly, it relates to uracil derivatives, a process for producing them, and their use as herbicides.

EP-A-311135 discloses some uracil derivatives useful as herbicides. However, these known herbicides are not sufficient in herbicidal potency or have poor selectivity between crop plants and weeds. Their herbicidal activity is thus not necessarily satisfiactory.

WO 90/ 150 57 (priority date: June 9, 1989), published after the priority date of the present invention, relates to herbicidal hydrocyclic compounds represented by the following formula 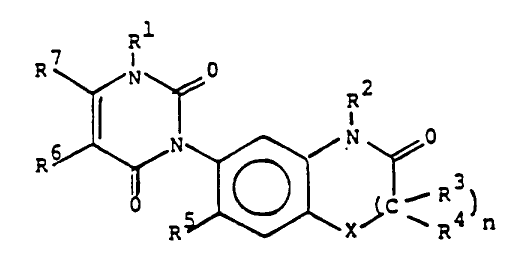 wherein R⁷ is halo-C₁₋₄-alkyl, R¹ is C₁₋₄-alkyl, R⁶ is hydrogen, R⁵ is hydrogen or fluorine, R is C₁₋₄-alkyl, C₃₋₄-alkenyl, C₃₋₄-alkynyl, C₃₋₄-haloalkenyl, R³ or R⁴ are hydrogen or C₁₋₄-alkyl, X is O and n is 0 or 1.

It has now been found that uracil derivatives of the formula: 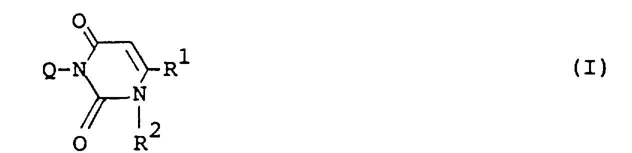 wherein R¹ is a trifluoromethyl group or a pentafluoroethyl group, R is a methyl group or an amino group and Q is either one of the formulas: 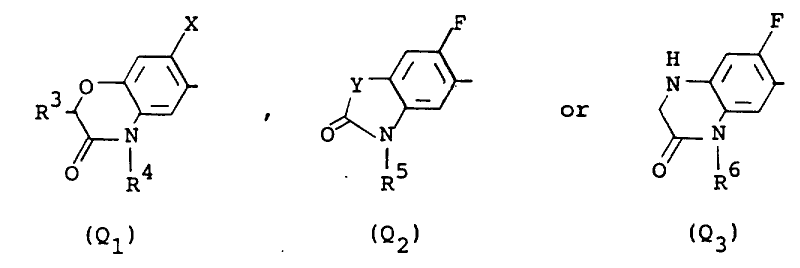 wherein R³ is a hydrogen atom or a methyl group, R⁴, R⁵ and R⁶ are each a C₁-C₇ alkyl group, a C₃-C₇ alkenyl group, a C₃-C₇ alkynyl group, a halo(C₁-C₆)alkyl group, a halo(C₃-C₆)alkenyl group or a C₁-C₄ alkoxy(C₁-C₃)alkyl group, X is a hydrogen atom or a fluorine atom and Y is an oxygen atom or a sulfur atom, with the proviso that when Q is Q₃, R represents a methyl group and when Q is Q₁ or Q₂, R represents an amino group, show a high herbicidal potency against various weeds with a high selectivity between crop plants and weeds. Thus, they produce a strong herbicidal activity against a wide variety of weeds including broad-leaved weeds, Graminaceous weeds, Commelinaceous weeds and Cyperaceous weeds in agricultural plowed fields by foliar or soil treatment without producing any material phytotoxicity on various agricultural crops such as corn, wheat, barley, rice plant, soybean and cotton. Examples of the broad-leaved weeds include wild buckwheat (Polygonum convolvulus), pale smartweed (Polygonum lapathifolium), common purslane (Portulaca oleracea), common chickweed (Stellaria media) , common lambsquarters (Chenopodium album), redroot pigweed (Amaranthus retroflexus), radish (Raphanus sativus), wild mustard (Sinapis arvensis), shepherdspurse (Capsella bursapastoris), hemp sesbania (Sesbania exaltata), sicklepod (Cassia obtusifolia), velvetleaf (Abutilon theophrasti), prickly sida (Sida spinosa), field pansy (Viola arvensis), catchweed bedstraw (Galium aparine), ivyleaf mornigglory (Ipomoea hederacea), tall morningglory (Ipomoea purpurea), field bindweed (Convolvulus arvensis), henbit (Lamium amplexicaure), jimsonweed (Datura stramonium) , black nightshade (Solanum nigrum), persian speedwell (Veronica persica), common cocklebur (Xanthium pensylvanicum), common sunflower (Helianthus annuus) , scentless chamomile (Matricaria perforata), corn marigold (Chrysanthemum segetum), purple deadnettle (Lamium purpureum), sun spurge (Euphorbia helioscopia), spotted spurge (Euphorbia maculata), etc.

Examples of Graminaceous weeds include Japanese millet (Echinochloa frumentacea), barnyardgrass (Echinochloa crus-galli), green foxtail (Setaria viridis) , large crabgrass (Digitaria sanguinalis), annual bluegrass (Poa annua), blackgrass (Alopecurus myosuroides), oats (Avena sativa) , wild oats (Avena fatua), johnsongrass (Sorghum halepense), quackgrass (Agropyron repens), bermudagrass (Cynodon dactylon), downy brome (Bromus tectorum), giant foxtail (Setaria faberi), etc. Example of Commelinaceous weeds include asiatic dayflower (Commelina communis) , etc. Examples of Cyperaceous weeds include rice flatsedge (Cyperus iria), purple nutsedge (Cyperus rotundus), etc.

The uracil derivatives (I) of the invention are also effective in exterminating paddy field weeds including Graminaceous weeds such as barnyardgrass (Echinochloa oryzicola), broad-leaved weeds such as common falsepimpernel (Lindernia procumbens), indian toothcup (Rotala indica) and waterwort (Elatine triandra), Cyperaceous weeds such as water nutgrass (Cyperus serotinus), hardstem bulrush (Scirpus juncoides) , needle spikerush (Eleocharis acicularis) and umbrella sedge (Cyperus difformis), and others such as monochoria (Monochoria vaginalis) and arrowhead (Sagittaria pygmaea) without producing any phytotoxicity to rice plants on flooding treatment.

Among above defined compounds (I), are those compounds preferred wherein R¹ and R are each as defined above and Q is either one of Q₁ and Q₂ in which R³, R⁴, R⁵ and X are each as defined above and Y is a sulfur atom. More preferred are those wherein R¹ and R are each as defined above and Q is either one of Q₁ and Q₂ in which R³ is a hydrogen atom, R⁴ is a C₃-C₇ alkynyl group, R⁵ is a C₁-C₇ alkyl group, X is a fluorine atom and Y is a sulfur atom.

The compound (I) of the invention can be produced by various procedures. For instance, the compound (I: Q = Q₁) of the formlula: 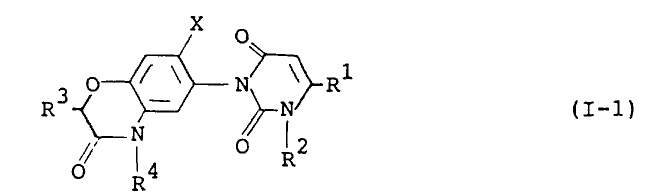 wherein R¹, R, R³, R⁴ and X are each as defined above is obtainable by the procedure as set forth below.

The compound (I-1: R = NH₂) is produced by reacting a compound of the formula: 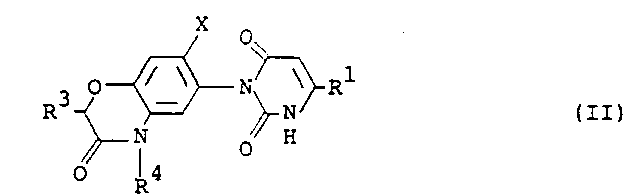 wherein R¹, R³, R⁴ and X are each as defined above, with an aminating agent.

The reaction is usually carried out in an inert solvent at a temperature of about 0 to 100°C for a period of about 0.5 to 10 hours.

The aminating agent is used in an amount of about 1 to 1.2 equivalents to one equivalent of the compound (II). As the aminating agent, there may be used 2,4-dinitrophenoxyamine or the like.

Examples of the inert solvent are aliphatic hydrocarbons (e.g. hexane, heptane, ligroin, petroleum ether), aromatic hydrocarbons (e.g. benzene, toluene, xylene), ethers (e.g. diethyl ether, diisopropyl ether, dioxane, tetrahydrofuran, diethylene glycol dimethyl ether), tertiary amines (e.g. pyridine, triethylamine, N,N-diethylaniline, tributylamine, N-methylmorpholine), acid amides (e.g. formamide, N,N-dimethylformamide, acetamide), sulfur compounds (e.g. dimethylsulfoxide, sulphorane), etc. These may be used solely or in combination.

After completion of the reaction, the reaction mixture is subjected to ordinary post-treatment. For instance, the reaction mixture is poured into water and extracted with an organic solvent, followed by concentration. If desired, any conventional purification procedure such as chromatography, distillation or recrystallization may be applied to the resulting product.

According to the above procedure, the compounds (I-1) as shown in Table 1 are obtained.

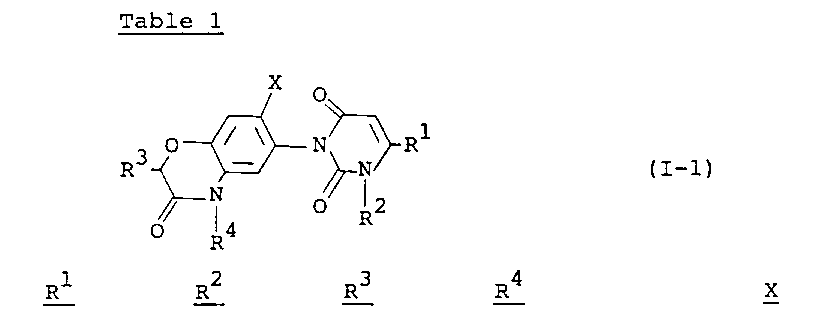

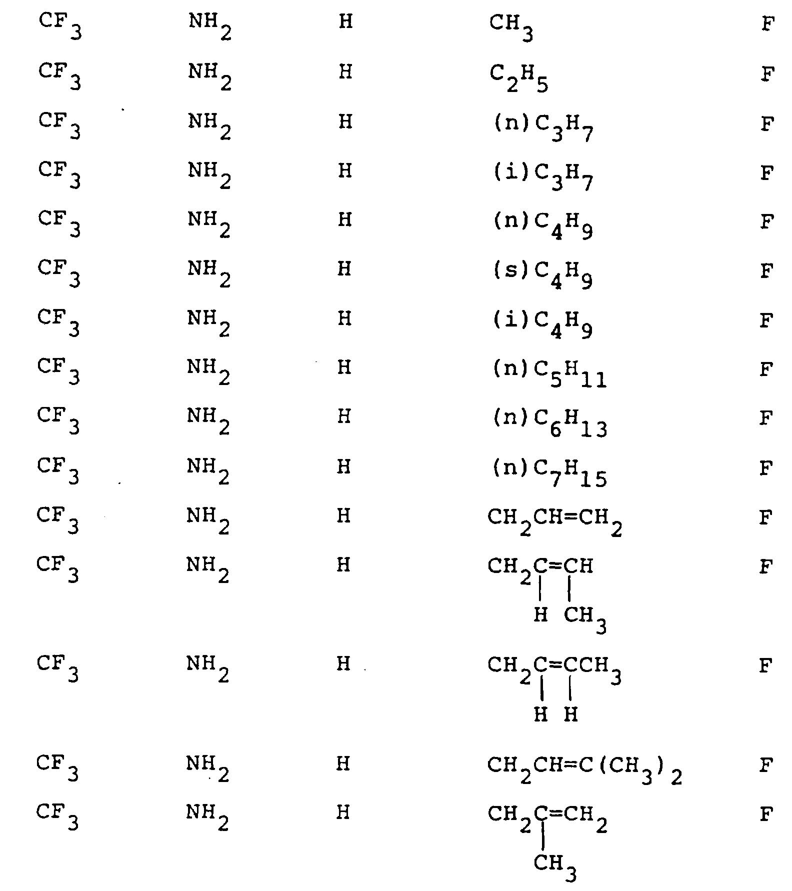

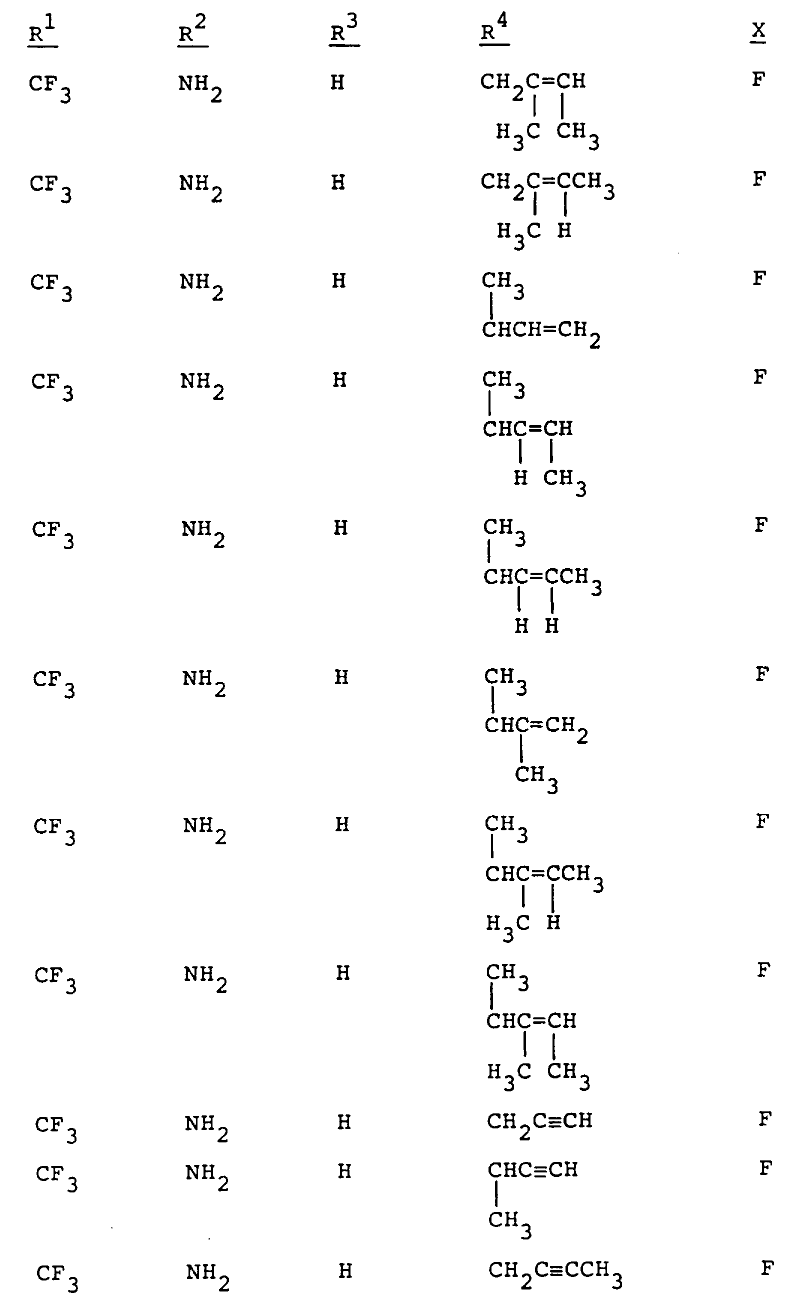

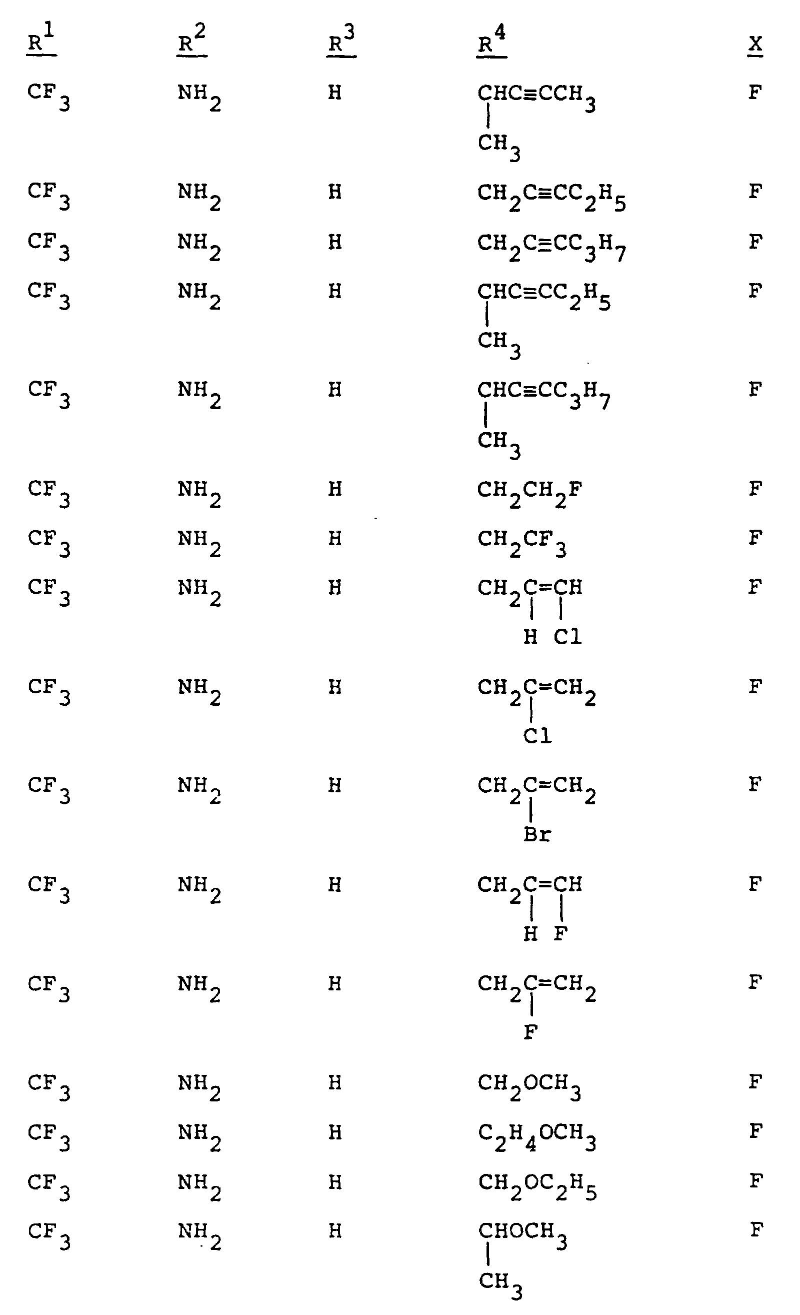

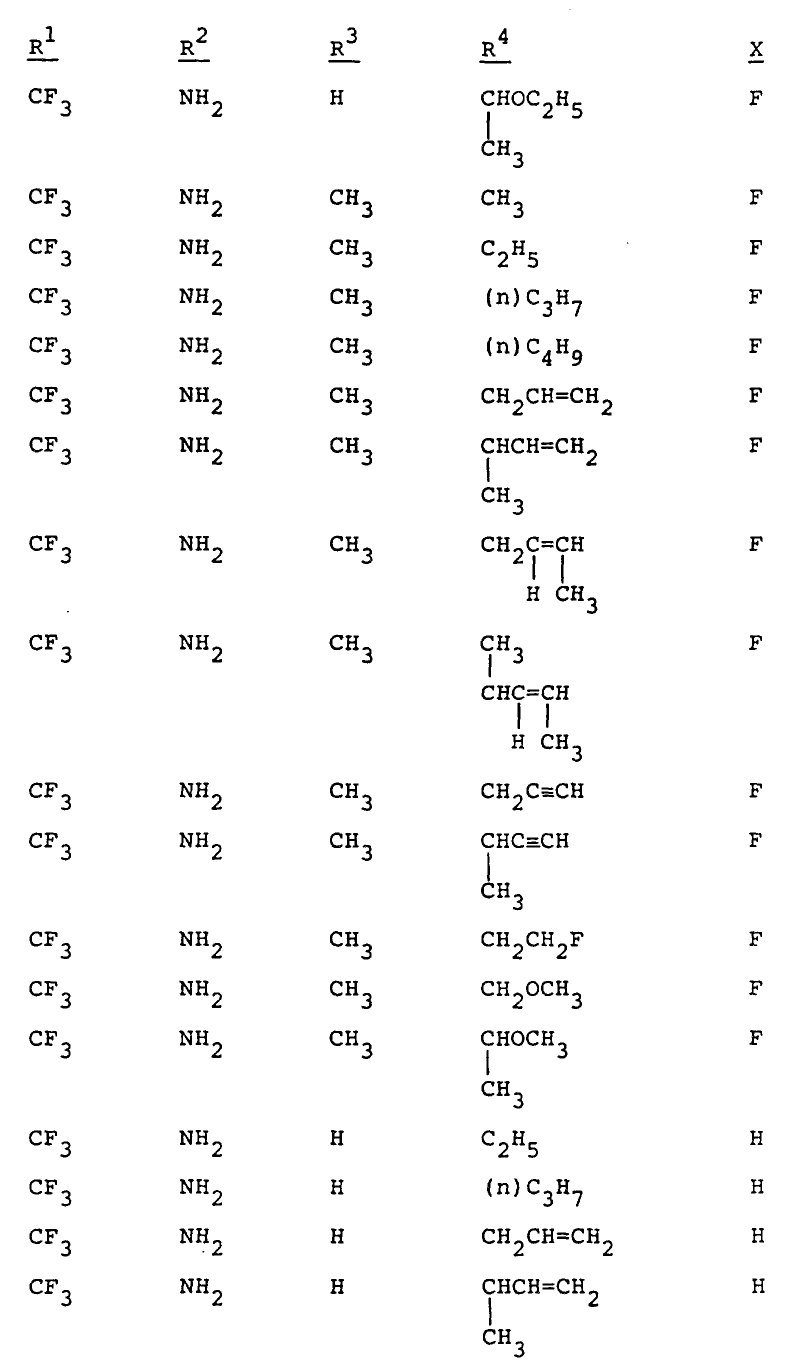

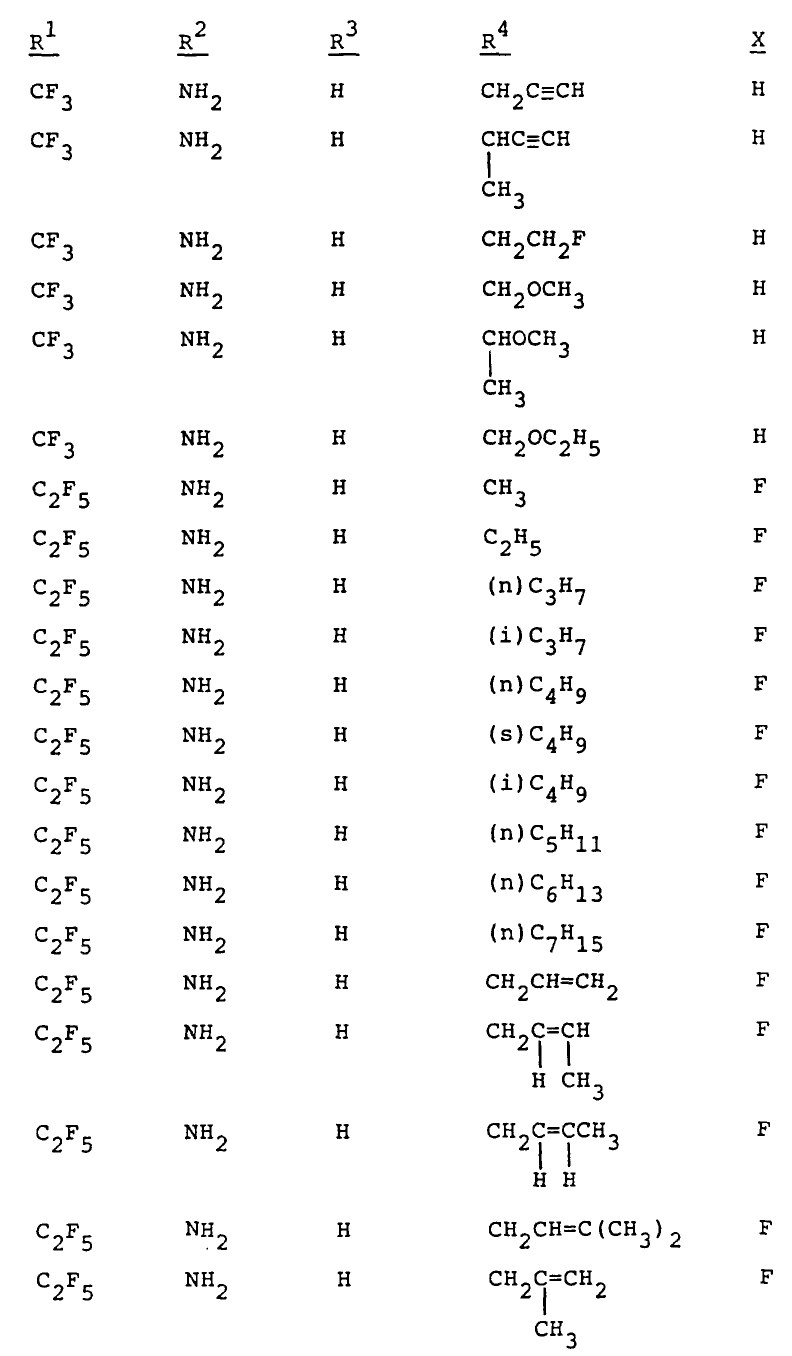

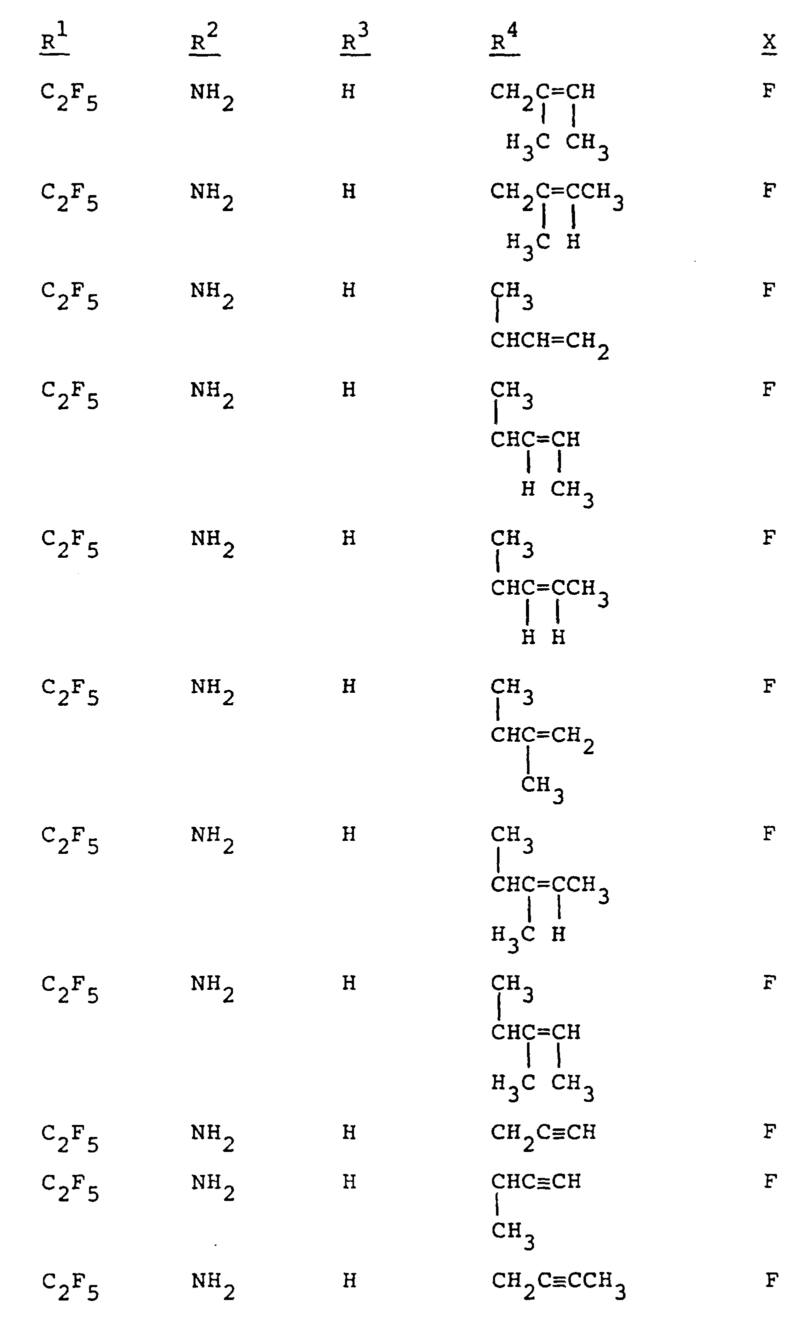

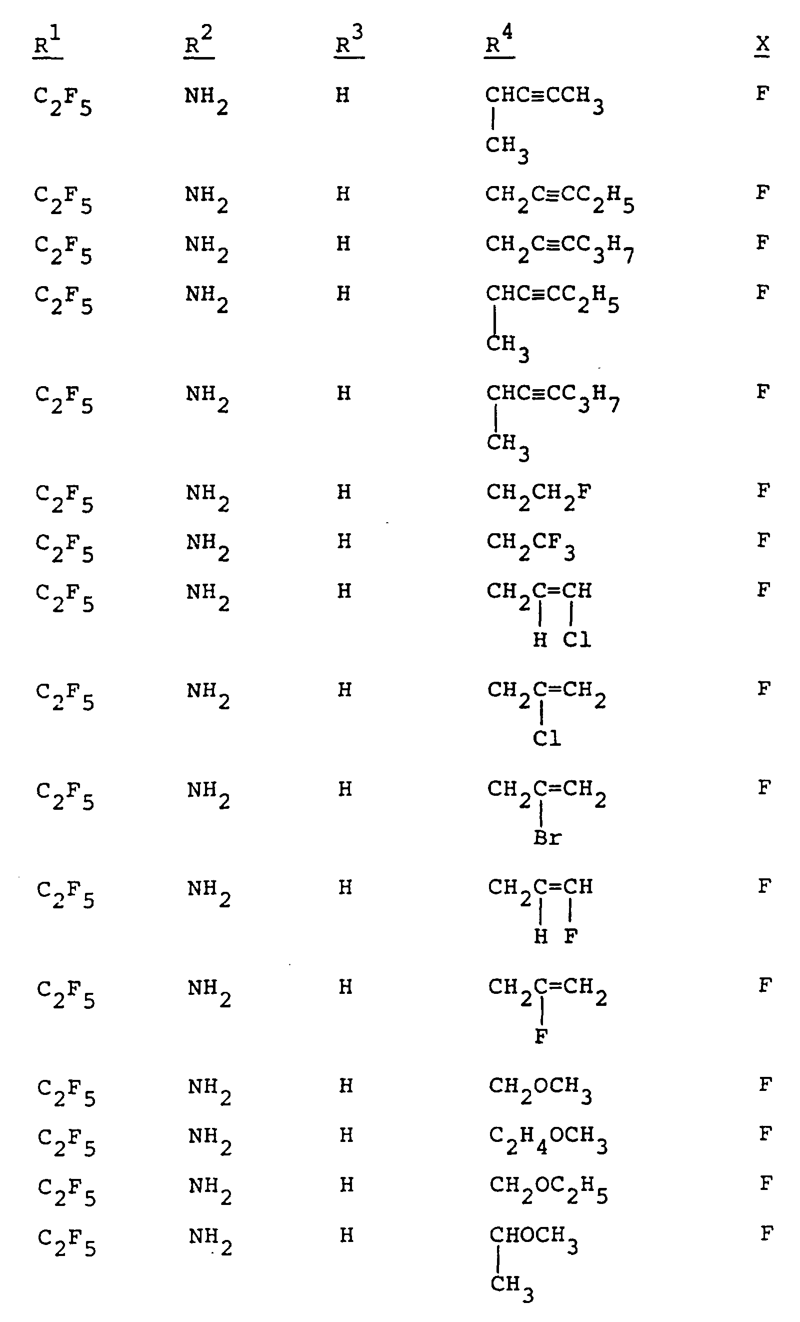

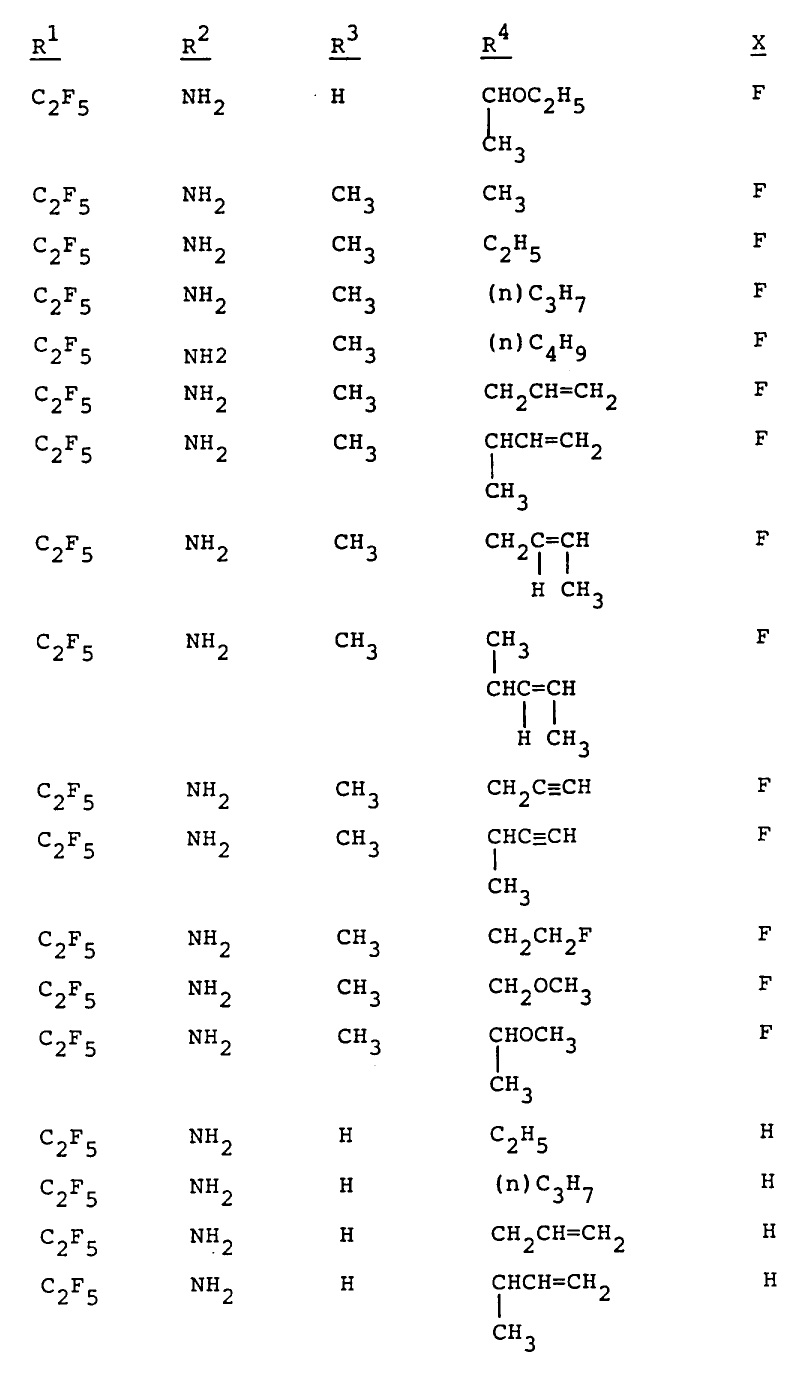

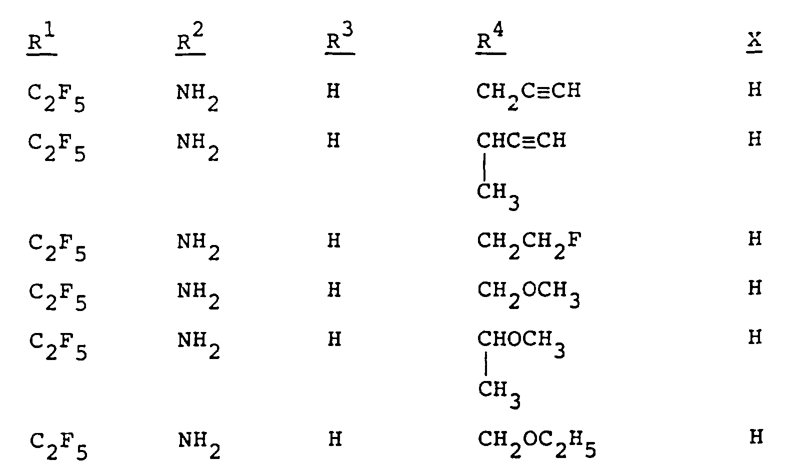

Some of the compounds (I-1) have optical isomers, which are also included within the scope of the invention.

Typical embodiments for production of the compounds (I-1) are illustratively shown in the following Example.

### Example 1

A mixture of 7-fluoro-6-methoxycarbonylamino-4-propargyl-2H-1,4-benzoxazin-3(4H)-one (1.4 g) and ethyl 3-amino-4,4,4-trifluorocrotonate was dissolved in dimethylformamide (5 g) , and sodium hydride (0.2 g) was added thereto. The resultant mixture was stirred for 30 minutes while cooling with ice and heated under reflux for 3 hours. After cooling, 2,4-dinitrophenoxyamine (1.2 g) was added thereto, and the resultant mixture was stirred at 40°C for 3 hours. After completion of the reaction, the reaction mixture was combined with water, extracted with ethyl acetate and concentrated. The residue was purified by silica gel column chromatography to give 1-[7-fluoro-4-propargyl-2H-1,4-benzoxazin-3(4H)-on-6-yl]-3-amino-4-trifluoromethyl-1,2,3,6-tetrahydropyrimidine-2,6-dione (0.2 g).

In the same manner as above, the compounds (I-1) as shown in Table 2 were obtained.

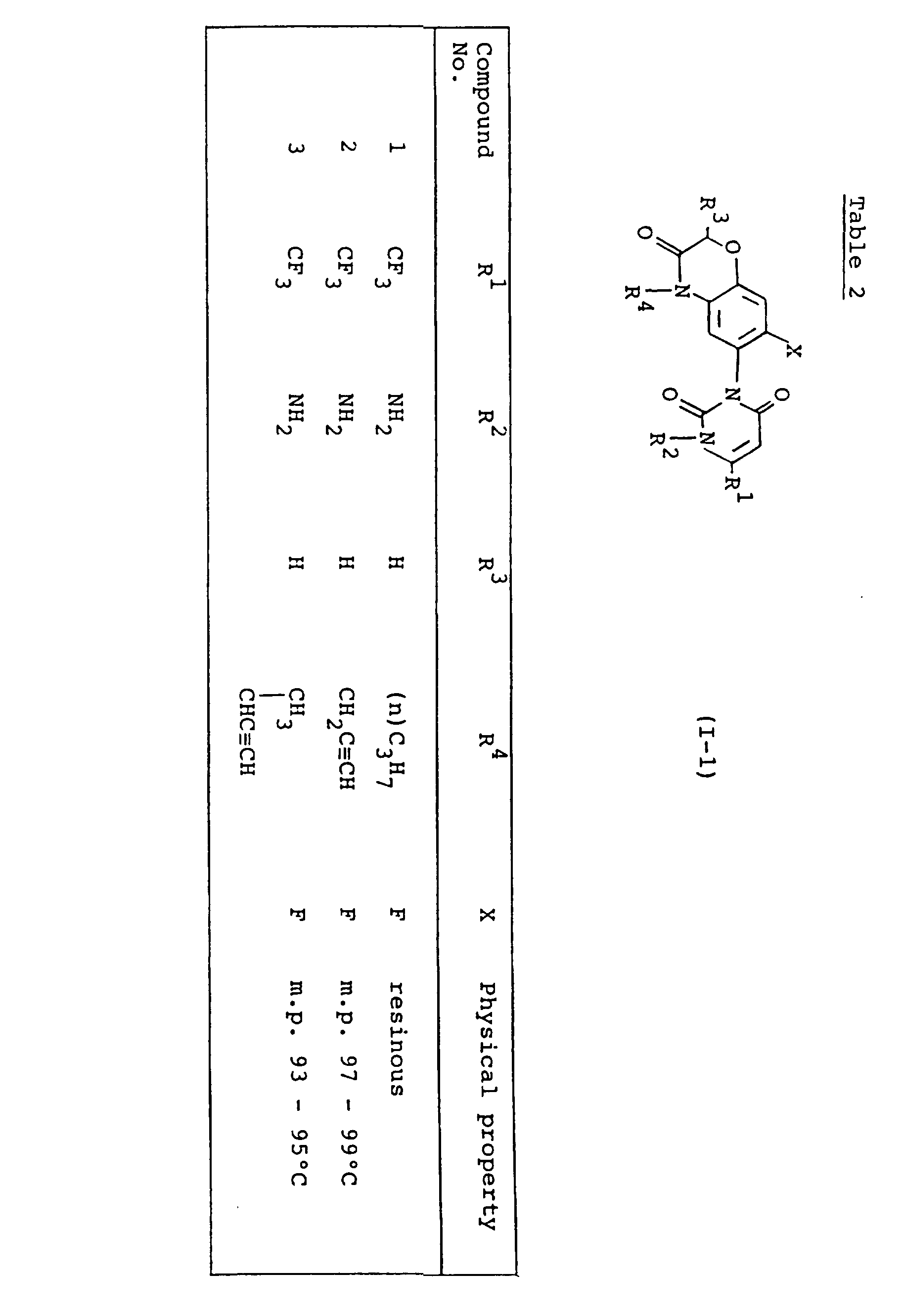

Further, for instance, the compound (I: Q = Q₂) of the formlula: 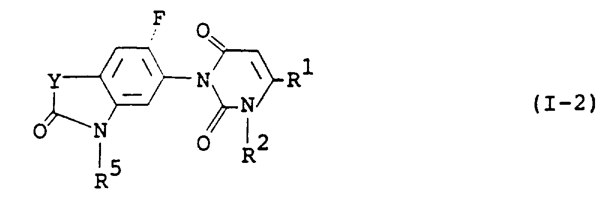 wherein R¹, R, R⁵ and Y are each as defined above is obtainable by the procedure as set forth below.

The compound (I-2: R = NH₂) is obtainable by reacting a compound of the formula: 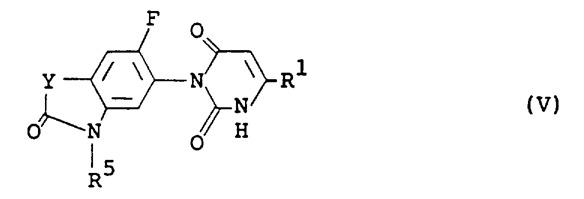 wherein R¹, R⁵ and Y are each as defined above, with an aminating agent.

The reaction is usually carried out in an inert solvent at a temparature of about 20 to 100°C for a period of about 0.5 to 8 hours.

The aminating agent is normally used in an amount of about 1 to 3 equivalents to one equivalent of the compound (V). As the aminating agent, there may be used 2,4-dinitrophenoxyamine, etc.

Examples of the inert solvent are aliphatic hydrocarbons (e.g. hexane, heptane, ligroin, petroleum ether), aromatic hydrocarbons (e.g. benzene, toluene, xylene), halogenated hydrocarbons (e.g. chloroform, carbon tetrachloride, dichloroethane, chlorobenzene, dichlorobenzene), ethers (e.g. diethyl ether, diisopropyl ether, dioxane, tetrahydrofuran, diethylene glycol dimethyl ether), ketones (e.g. acetone, methyl ethyl ketone, methyl isobutyl ketone, isophorone, cyclohexanone) , tertiary amines (e.g. pyridine, triethylamine, N,N-diethylaniline, tributylamine, N-methylmorpholine), acid amides (e.g. formamide, N,N-dimethylformamide, acetamide) , sulfur compounds (e.g. dimethylsulfoxide, sulphorane), water, etc.

After completion of the reaction, the reaction mixture is subjected to ordinary post-treatment. For instance, the reaction mixture is poured into water, and the precipitated crystals are collected by filtration. Alternatively, the reaction mixture is shaken in combination with water and a water-immisicible organic solvent for extraction, and the extract is concentrated. If desired, any conventional purification procedure such as chromatography, distillation or recrystallization may be applied to the resulting product.

According to the above procedure the compounds (I-2) as shown in Table 3 are obtained.

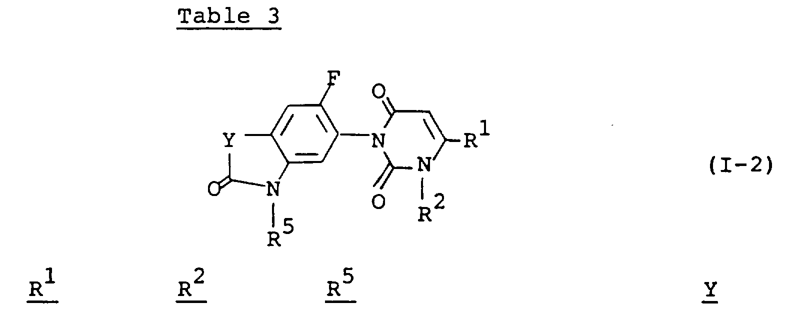

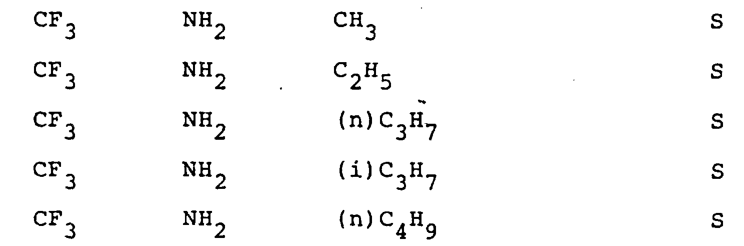

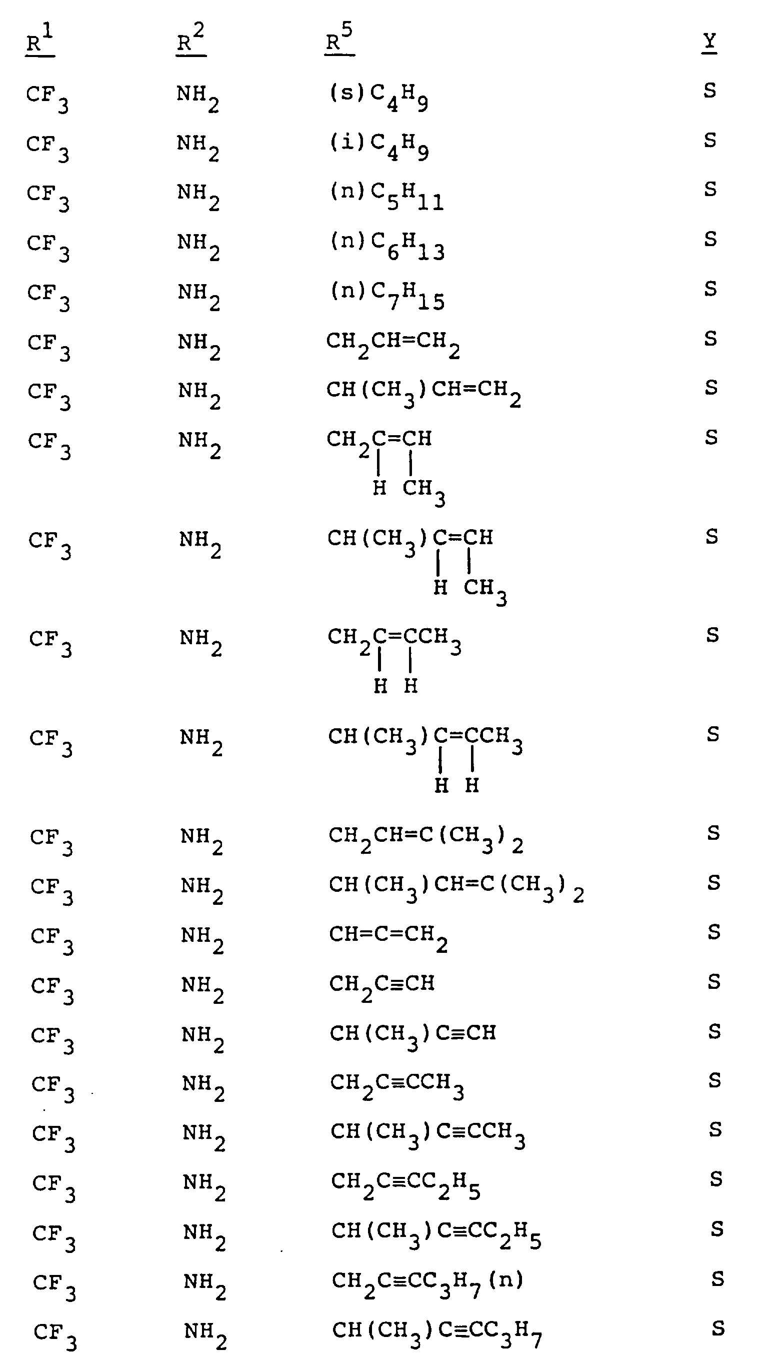

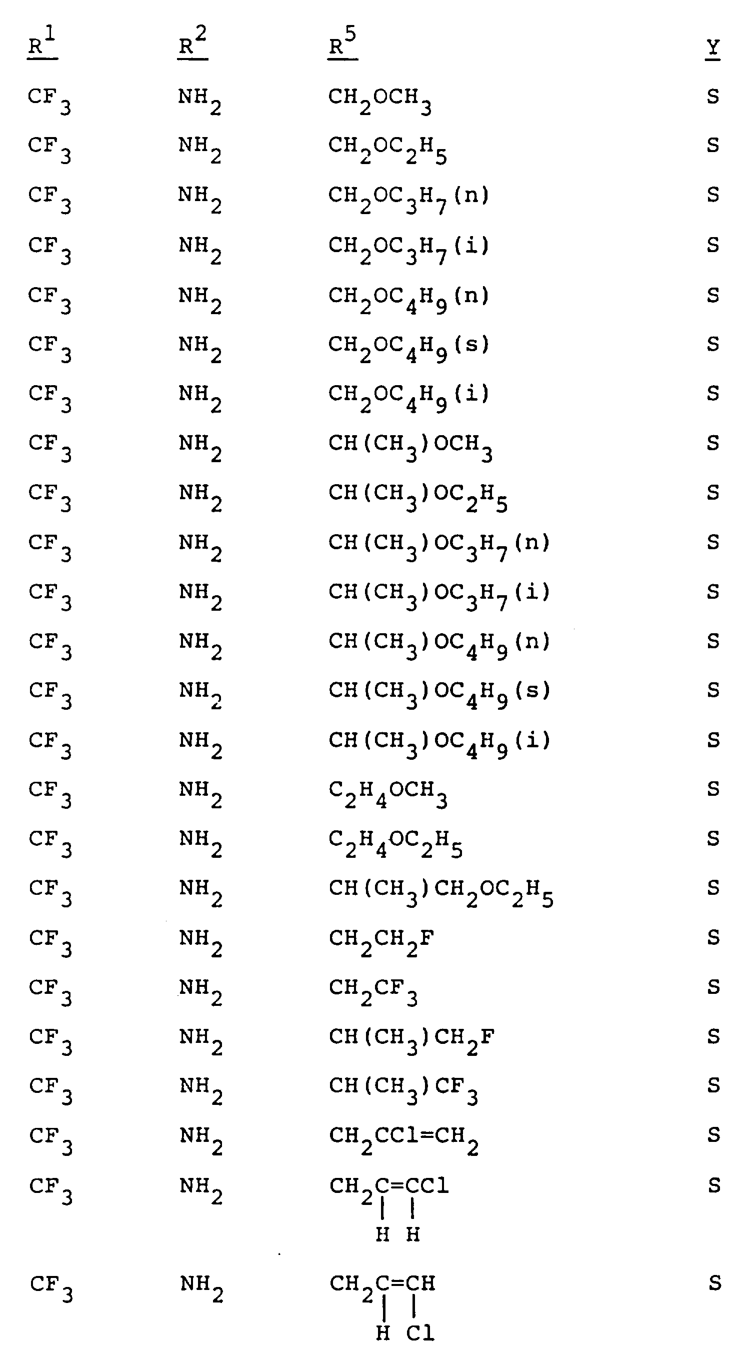

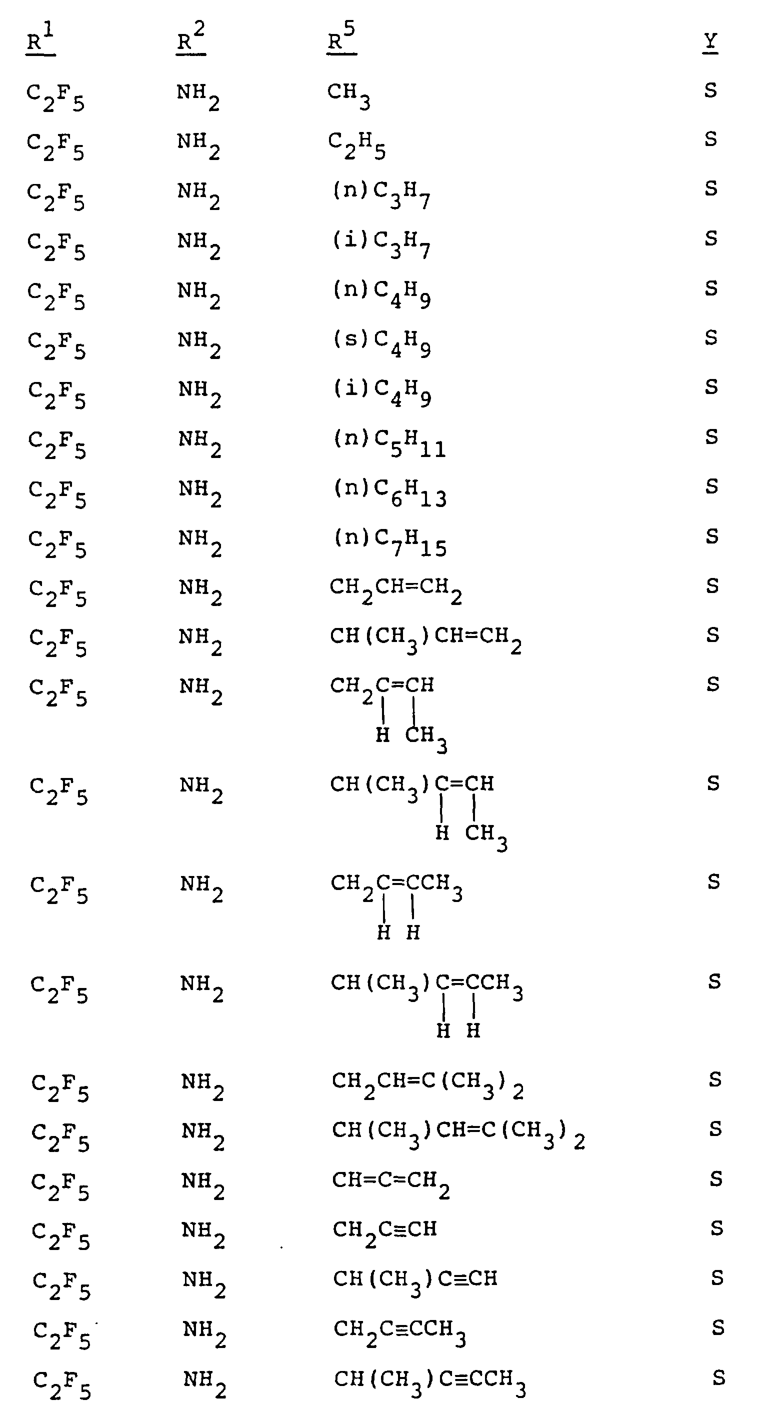

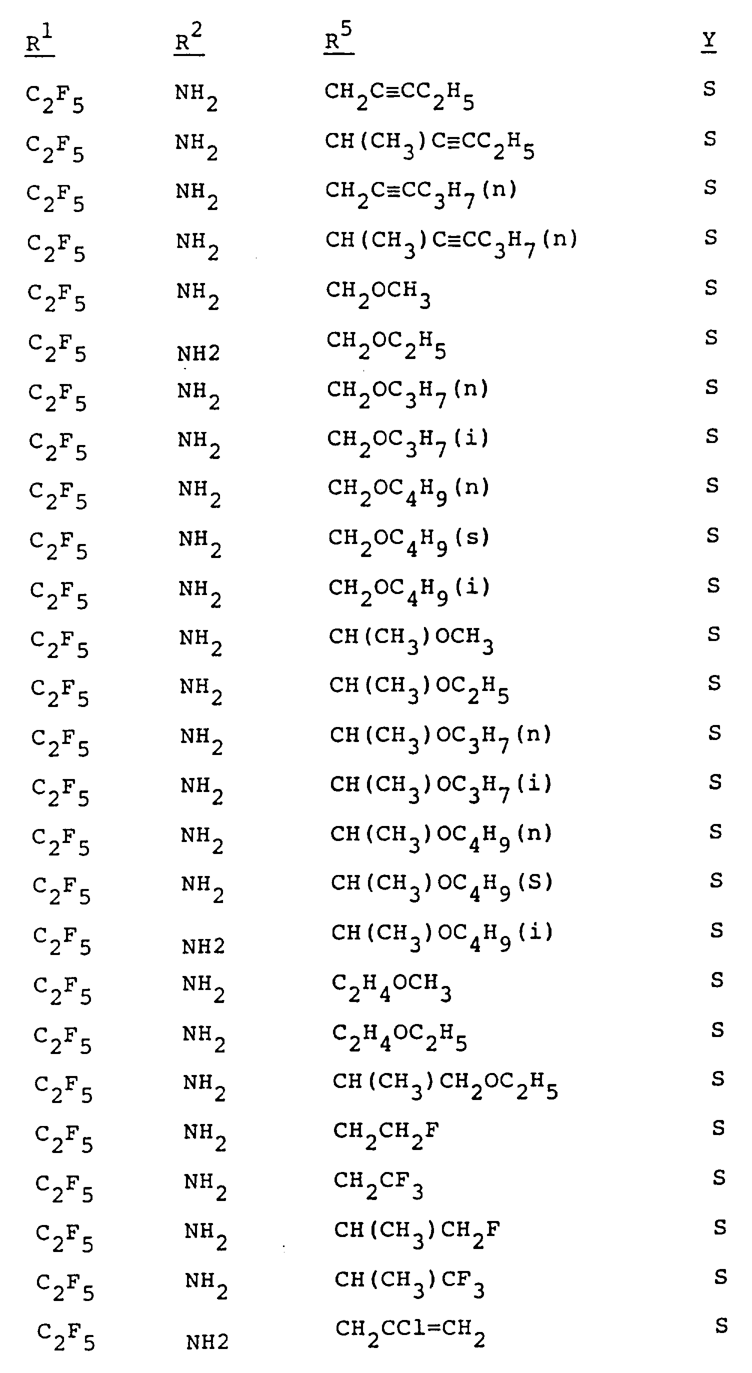

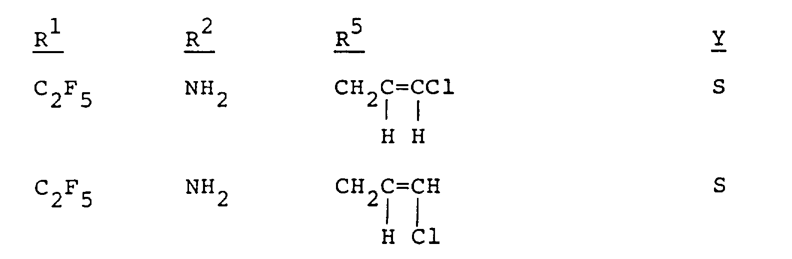

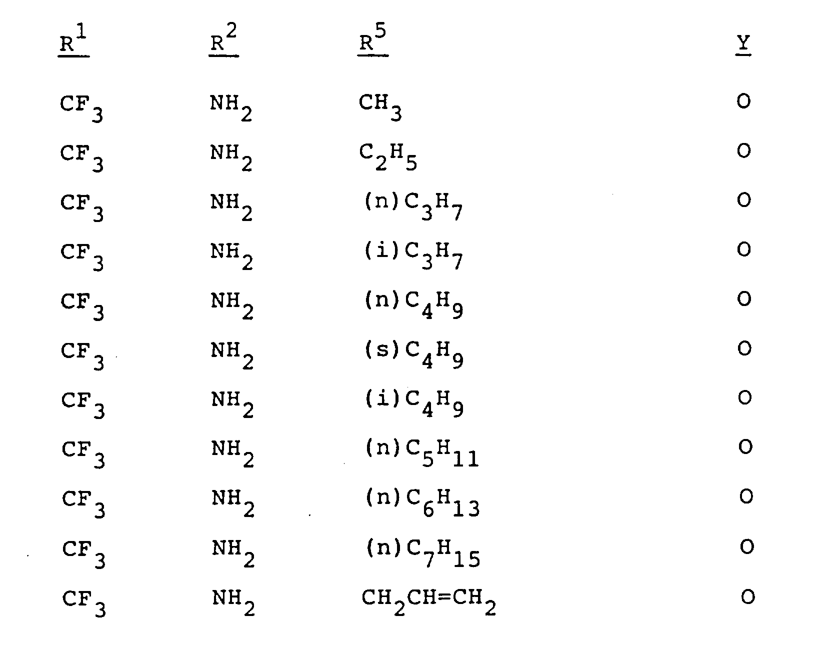

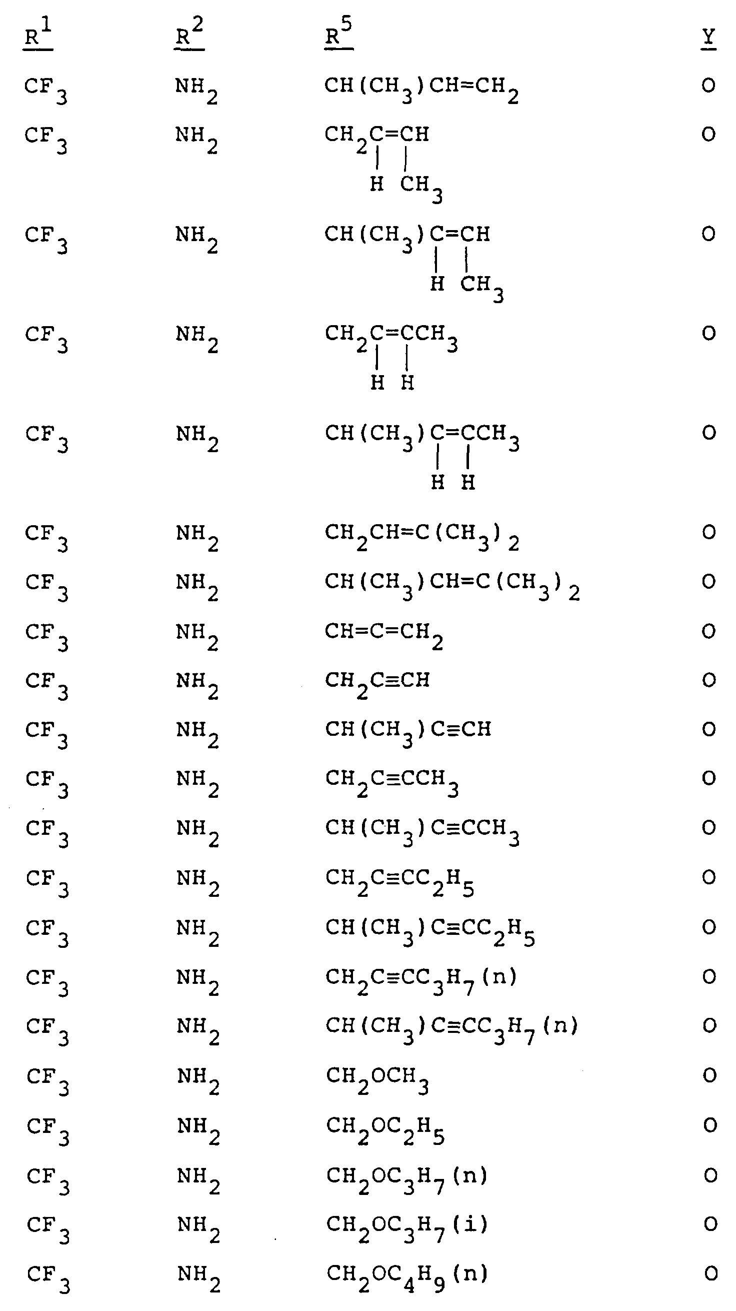

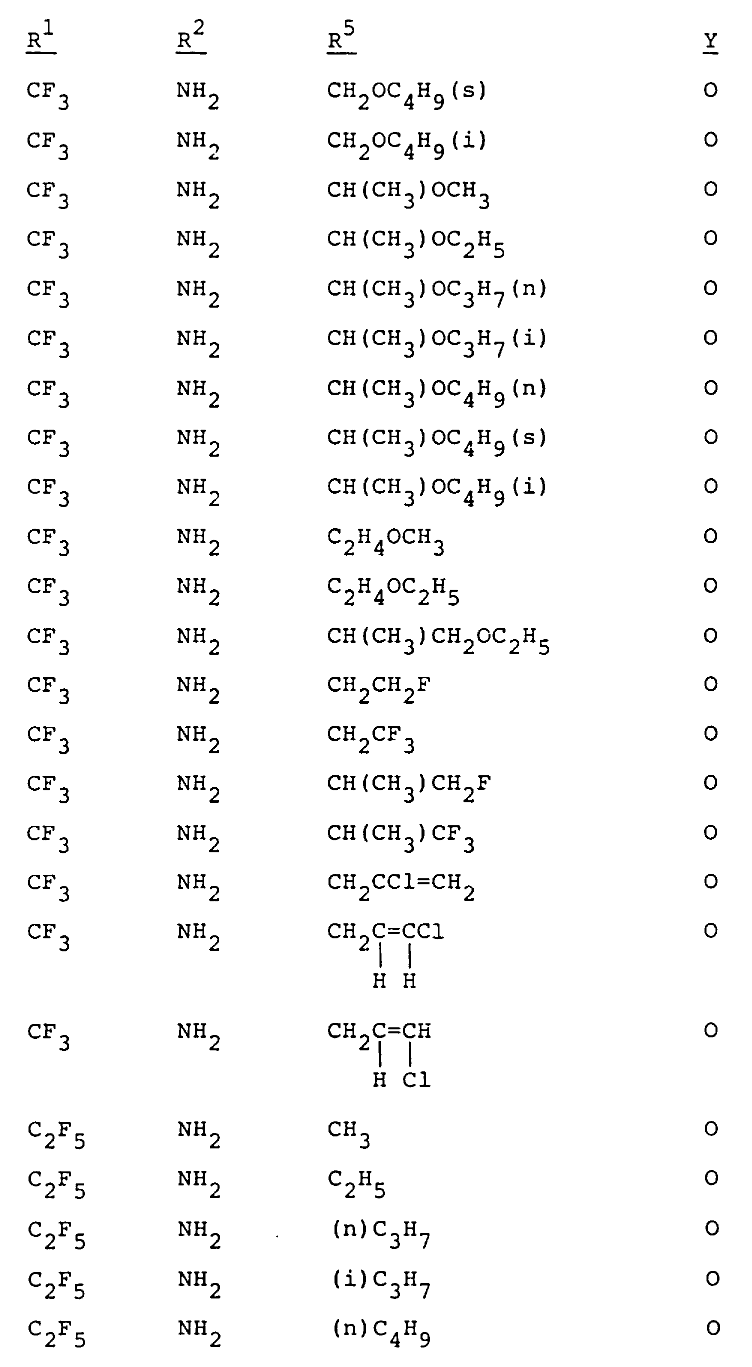

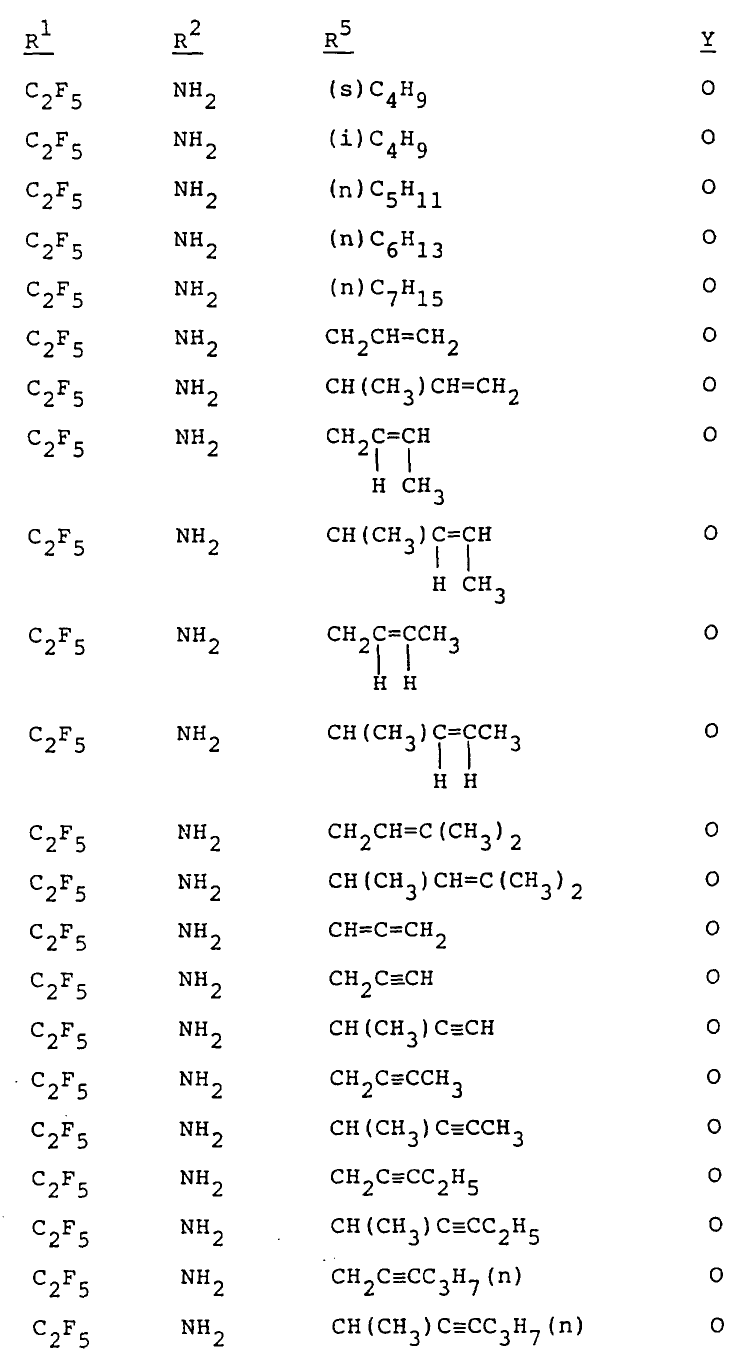

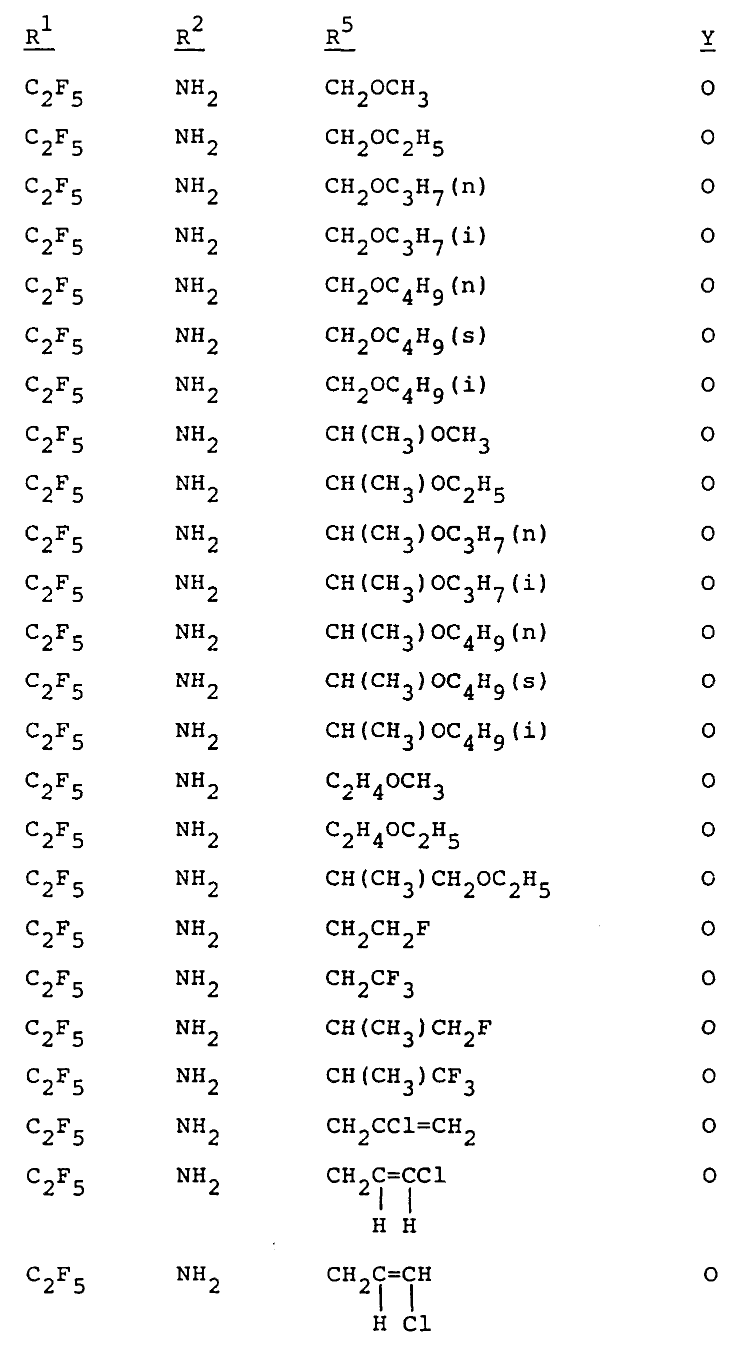

Some of the compounds (I-2) have optical isomers, which are also included within the scope of the invention.

Typical embodiments for production of the compounds (I-2) are illustratively shown in the following Example.

### Example 2

To a solution of 1-[6-fluoro-3-sec-butyl-2(3H)-benzothiazolon-5-yl]-4-trifluoromethyl-1,2,3,6-tetrahydropyrimidine-2,6-dione (2.0 g) in dimethylformamide (10 g), sodium hydride (0.3 g) and 2,4-dinitrophenoxyamine (1.8 g) were added, and the resultant mixture was heated at 40 to 60°C for 3 hours. After completion of the reaction, the reaction mixture was poured into water, extracted with ethyl acetate and concentrated. The residue was purified by silica gel column chromatography to give 1-[6-fluoro-3-sec-butyl-2(3H)-benzothiazolon-5-yl]-3-amino-4-trifluoromethyl-1,2,3,6-tetrahydropyrimidine-2,6-dione (0.4 g).

In the same manner as above, the compounds (I-2) as shown in Table 4 were obtained.

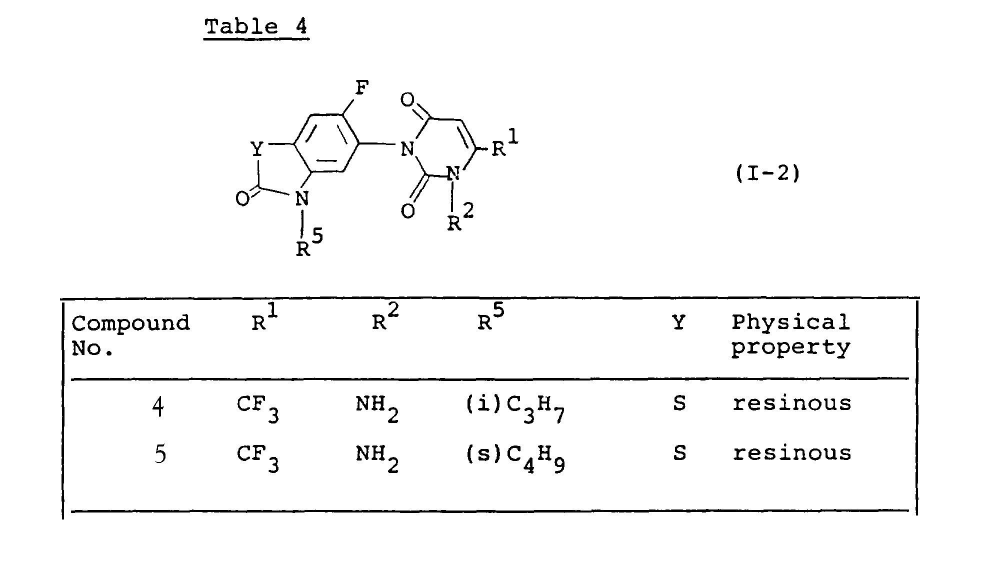

Furthermore, for instance, the compound (I: Q = Q₃) of the formula: 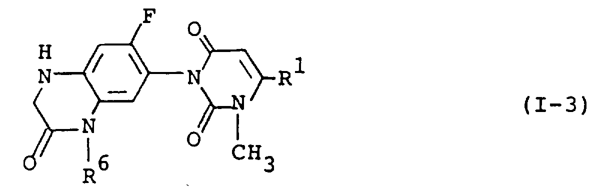 wherein R¹ and R⁶ are each as defined above is obtainable by the procedure as set forth below.

The compound (I-3) is prepared by reacting a compound of the formula: 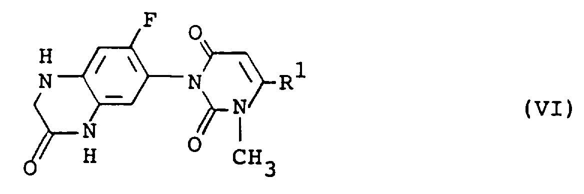 wherein R¹ is as defined above with a compound of the formula:

R⁶-Z (VII)

wherein R⁶ and Z are each as defined above.

The reaction is usually carried out in the presence of a base in an inert solvent at a temparature of about 0 to 100°C for a period of about 0.5 to 12 hours.

The compound (VII) and the base are ordinarily used respectively in amounts of about 1 to 1.2 equivalents and of about 1 to 1.5 equivalents to one equivalent of the compound (VI). As the base, there may be exemplified an inorganic base (e.g. sodium hydroxide, potassium hydroxide, sodium carbonate, potassium carbonate, sodium hydride), an alkali metal alkoxide (e.g. sodium methoxide, sodium ethoxide), etc.

Examples of the inert solvent are aliphatic hydrocarbons (e.g. hexane, heptane, ligroin, petroleum ether) , aromatic hydrocarbons (e.g. benzene, toluene, xylene), halogenated hydrocarbons (e.g. chloroform, carbon tetrachloride, dichloroethane, chlorobenzene, dichlorobenzene), ethers (e.g. diethyl ether, diisopropyl ether, dioxane, tetrahydrofuran, diethylene glycol dimethyl ether), ketones (e.g. acetone, methyl ethyl ketone, methyl isobutyl ketone, isophorone, cyclohexanone), fatty acids (e.g. formic acid, acetic acid, oleic acid), alcohols (e.g. methanol, ethanol, isopropanol, t-butanol, octanol, cyclohexanol, methyl cellosolve, diethylene glycol, glycerine), esters (e.g. ethyl formate, ethyl acetate, butyl acetate, diethyl carbonate) , nitro compounds (e.g. nitroethane, nitrobenzene), nitriles (e.g. acetonitrile, isobutylonitrile), tertiary amines (e.g. pyridine, triethylamine, N,N-diethylaniline, tributylamine, N-methylmorpholine), acid amides (e.g. formamide, N,N-dimethylformamide, acetamide), sulfur compounds (e.g. dimethyl sulfoxide, sulphorane), aqueous ammonia, water, etc. These may be used solely or in combination.

After completion of the reaction, the reaction mixture is subjected to ordinary post-treatment. For instance, the reaction mixture is poured into water, and the precipitated crystals are collected by filtration. Alternatively, the reaction mixture is shaken in combination with water and a water immiscible organic solvent for extraction, and the extract is concentrated. If desired, any conventional purification procedure such as chromatography, distillation or recrystallization may be applied to the resulting product.

According to the above procedure, the compounds (I-3) as shown Table 5 are obtained.

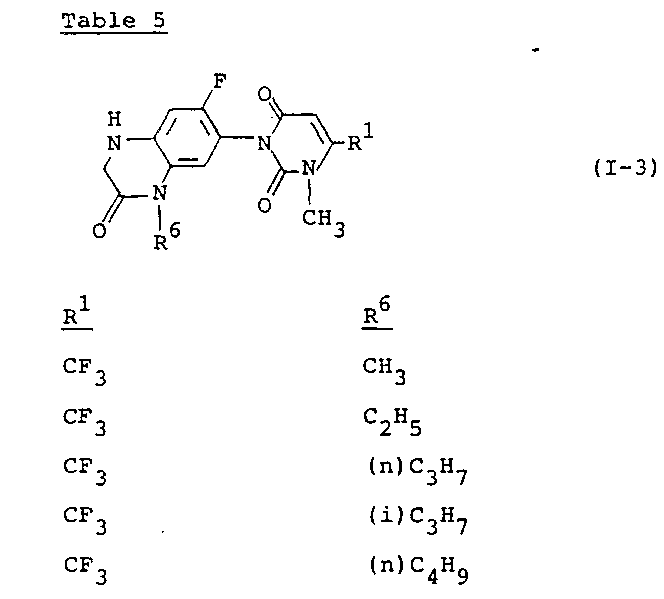

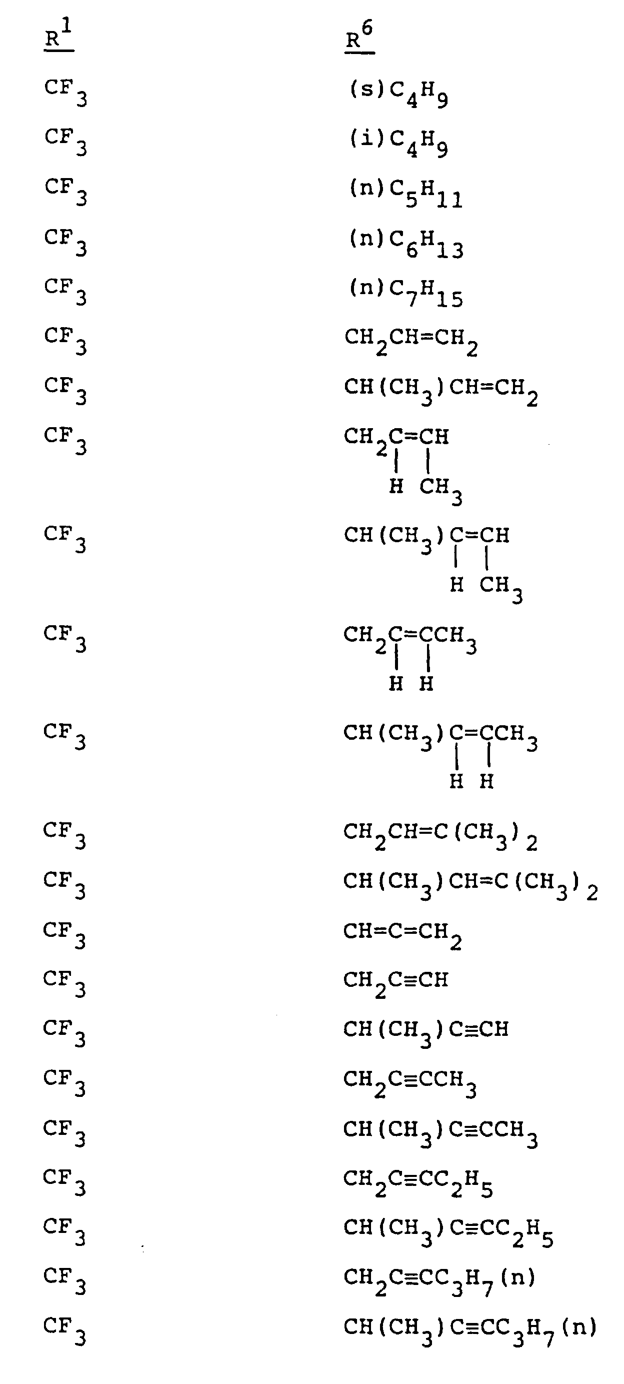

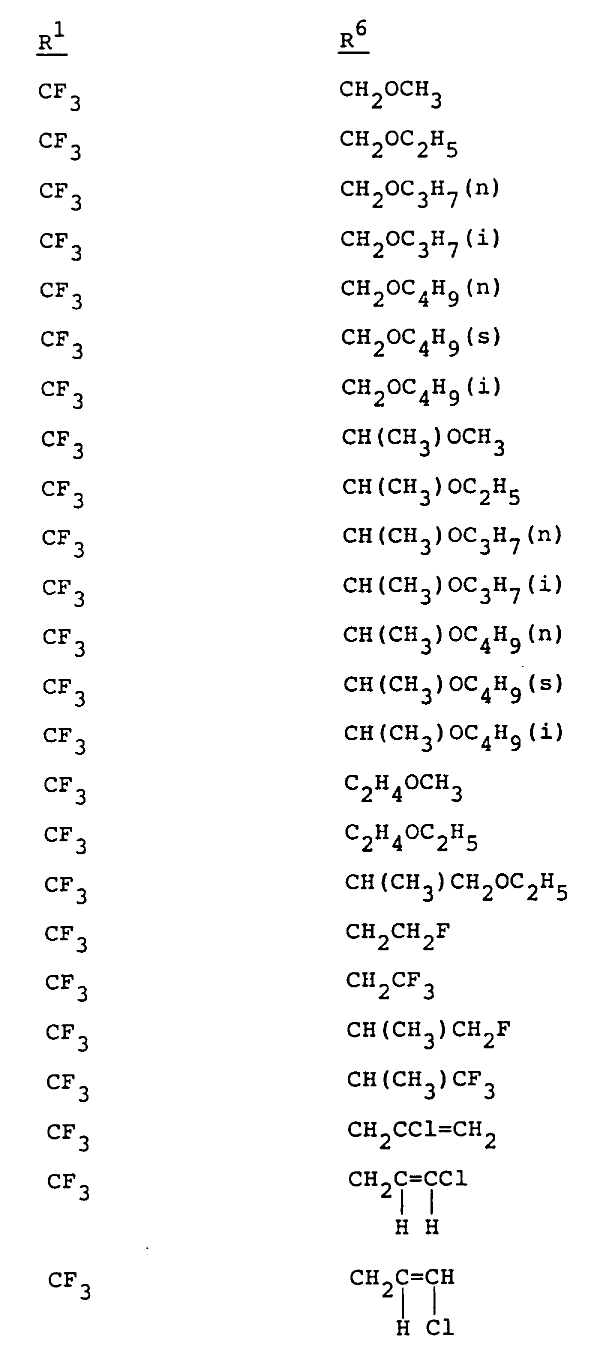

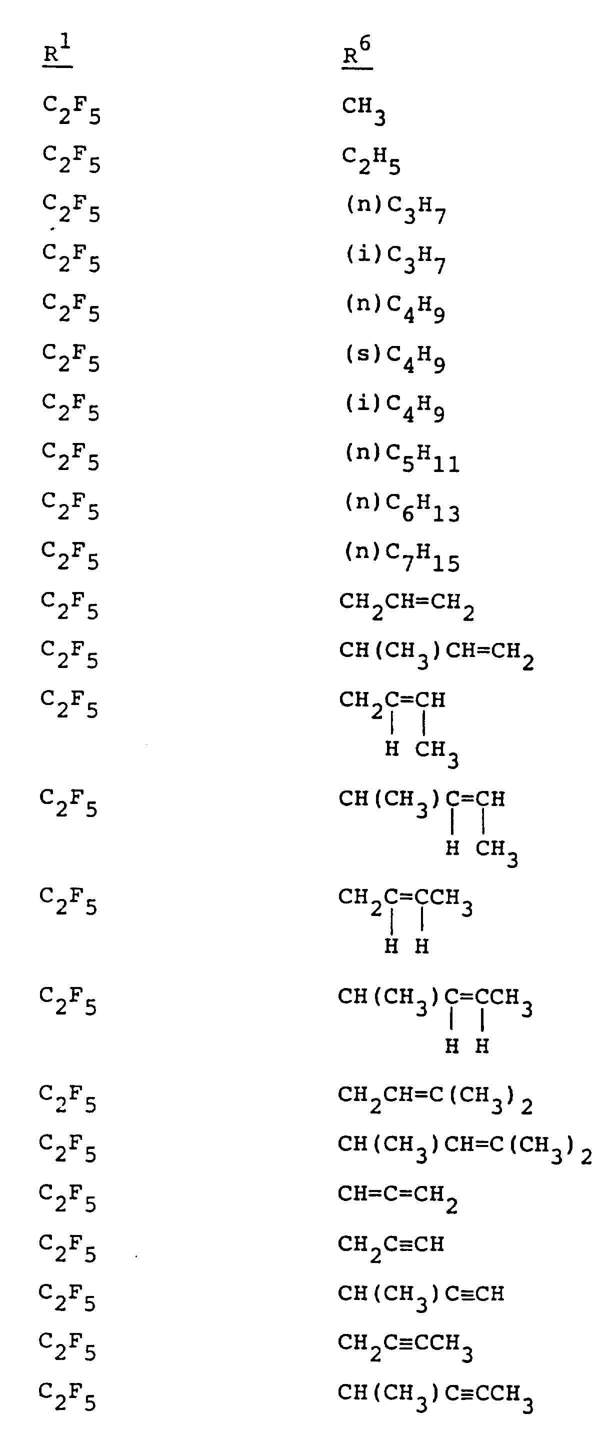

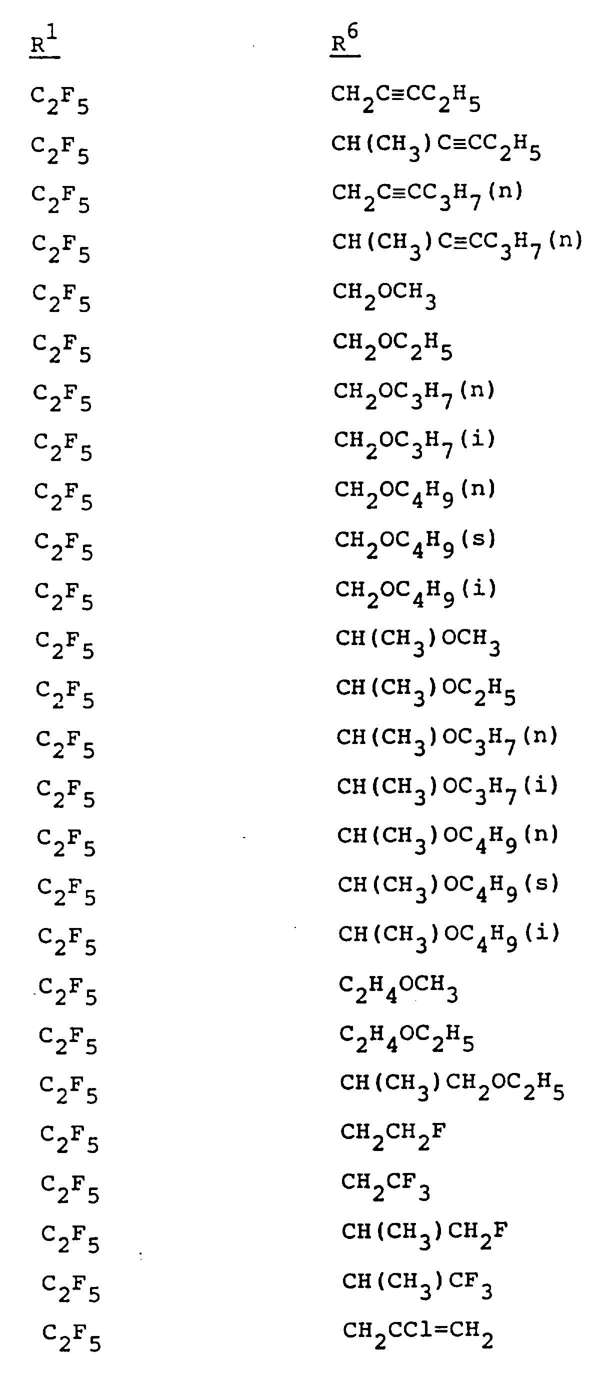

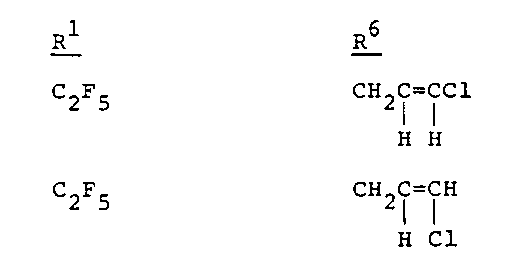

A typical embodiment for production of the compounds (I-3) is illustratively shown in the following Example.

### Example 3

To a solution of 1-[6-fluoro-1,2,3,4-tetrahydroquinoxalin-2-on-7-yl]-3-methyl-4-trifluoromethyl-1,2,3,6-tetrahydropyrimidine-2,6-dione (2.0 g) in dimethylformamide (10 g), sodium hydride (500 mg) and propargyl bromide (1.0 g) were added, and the resultant mixture was heated at 40 to 50°C for 1 hour. After completion of the reaction, the reaction mixture was poured into water, and the precipitated crystals were collected by filtration, washed and dried to give 1-[6-fluoro-1-propargyl-1,2,3,4-tetrahydroquinoxaline-2-on-7-yl]-3-methyl-4-trifluoromethyl-1,2,3,6-tetrahydropyrimidine-2,6-dione (1.0 g).

In the same manner as above, the compounds (I-3) as shown in Table 6 were obtained.

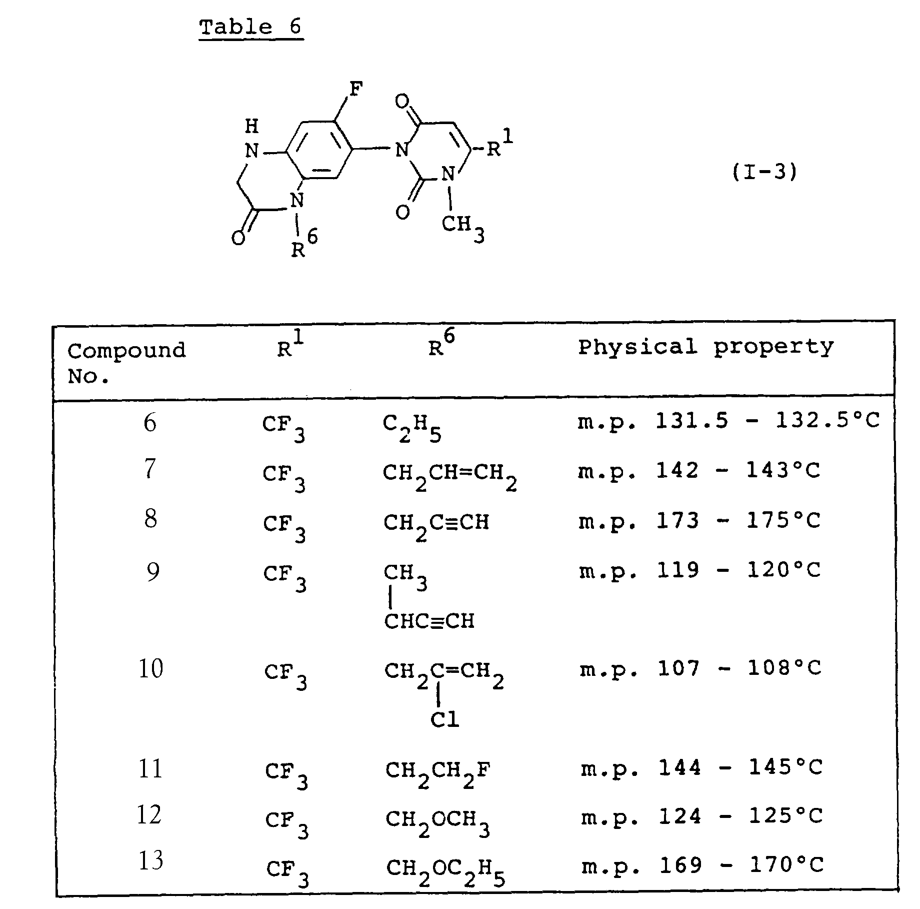

The starting compounds (II) or (V) and (VIII) or (X) may be produced according to the following scheme: 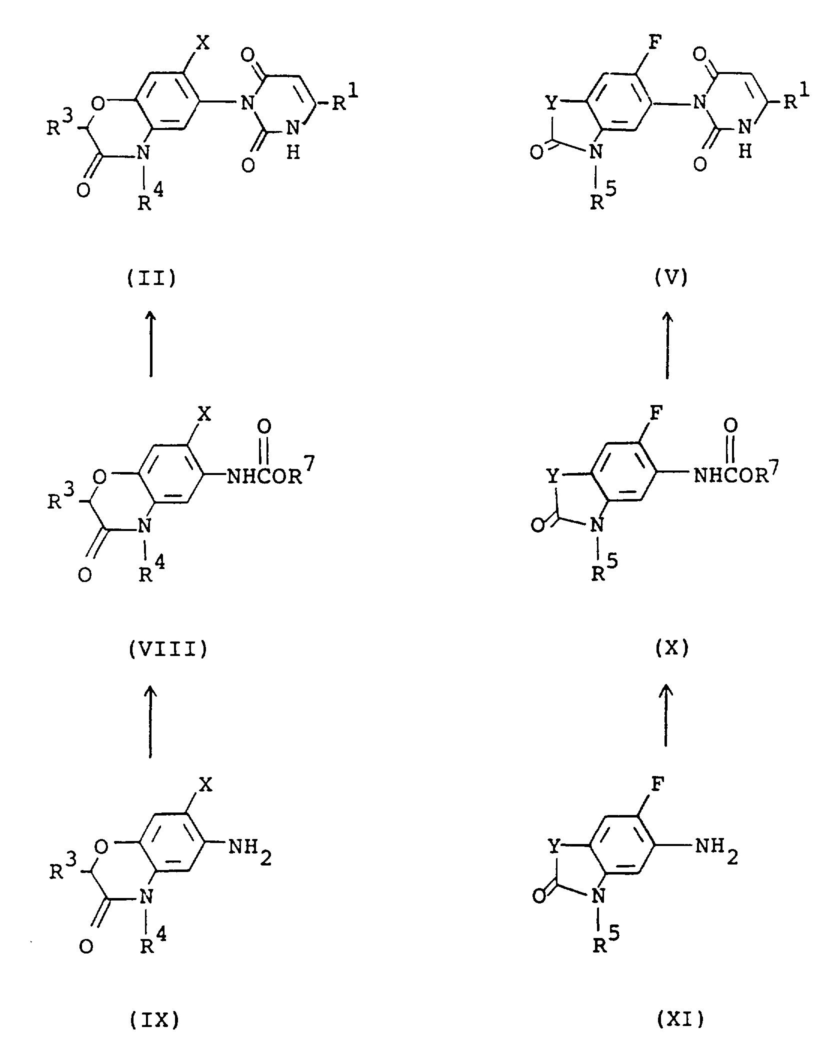 wherein R⁷ is a C₁-C₆ alkyl group,
and R¹, R³, R⁴, R⁵, X and Y are each as defined above.

The reaction at each step in the above scheme will be hereinafter explained in detail.

### (1) Preparation of the compound (II) or the compound (V) from the compound (VIII) or the compound (X):-

The compound (II) or the compound (V) may be produced by reacting the compound (VIII) or the compound (X) with a compound of the formula:

R¹(NH₂)C=CHCOOR⁹ (XII)

wherein R⁹ is a C₁-C₆ alkyl group and R¹ is as defined above usually in the presence of a dehydrogenating agent in an inert solvent at a temparature of about 0 to 200°C for a period of about 0.5 to 10 hours.

In general, the compound (XII) and the dehydrogenating agent are used respectively in amounts of about 1 to 1.2 equivalents and of about 1 to 1.2 equivalents to one equivalent of the compound (VIII) or the compound (X). As the dehydrogenating agent, there may be used an inorganic base (e.g. sodium carbonate, potassium carbonate, sodium hydride), an alkali metal alkoxide (e.g. sodium methoxide, sodium ethoxide), etc.

Examples of the inert solvent are aliphatic hydrocarbons (e.g. hexane, heptane, ligroin, petroleum ether), aromatic hydrocarbons (e.g. benzene, toluene, xylene), ethers (e.g. diethyl ether, diisopropyl ether, dioxane, tetrahydrofuran, diethylene glycol dimethyl ether), tertiary amines (e.g. pyridine, triethylamine, N,N-diethylaniline, tributylamine, N-methylmorpholine), acid amides (e.g. formamide, N,N-dimethylformamide, acetamide), sulfur compounds (e.g. dimethylsulfoxide, sulphorane), etc. These may be used solely or in combination.

After completion of the reaction, the reaction mixture is subjected to ordinary post-treatment. For instance, the reaction mixture is poured into water and extracted with an organic solvent, followed by concentration. If desired, any conventional purification procedure such as chromatography, distillation or recrystallization may be applied to the resulting product.

The compound (II) or the compound (V) may be methylated or aminated without isolation to give the compound (I-1) or the compound (I-2).

A typical embodiment for preparation of the compound (II) or the compound (V) is illustratively shown in the following example.

### Example 4

To a solution of 3-isopropyl-6-fluoro-5-methoxycarbonylamino-2(3H)-benzothiazolone (2.8 g) in N,N-dimethylformamide (10 g), sodium hydride (0.4 g) and ethyl 3-amino-4,4,4-trifluorocrotonate (0.9 g) were added, and the resultant mixture was heated under reflux for 3 hours. After cooling, the reaction mixture was poured into water and extracted with ethyl acetate. The organic layer was concentrated, and the residue was purified by column chromatography to give 1-[3-isopropyl-6-fluoro-2(3H)-benzothiazolon-5-yl]-4-trifluoromethyl-1,2,3,6-tetrahydropyrimidine-2,6-dione (0.8 g).

H-NMR δ (ppm) [CDCl₃, 60 MHz]: 1.55 (6H, d, J = 7 Hz), 4.4-5.5 (1H, m). 6.17 (1H, s), 7.0 (1H, d, J = 6 Hz), 7.25 (1H, d, J = 9 Hz), 9.0-10.1 (1H, m).

In the same manner as above, the compounds (II) and the compounds (V) as shown in Tables 7 and Table 8 were obtained.

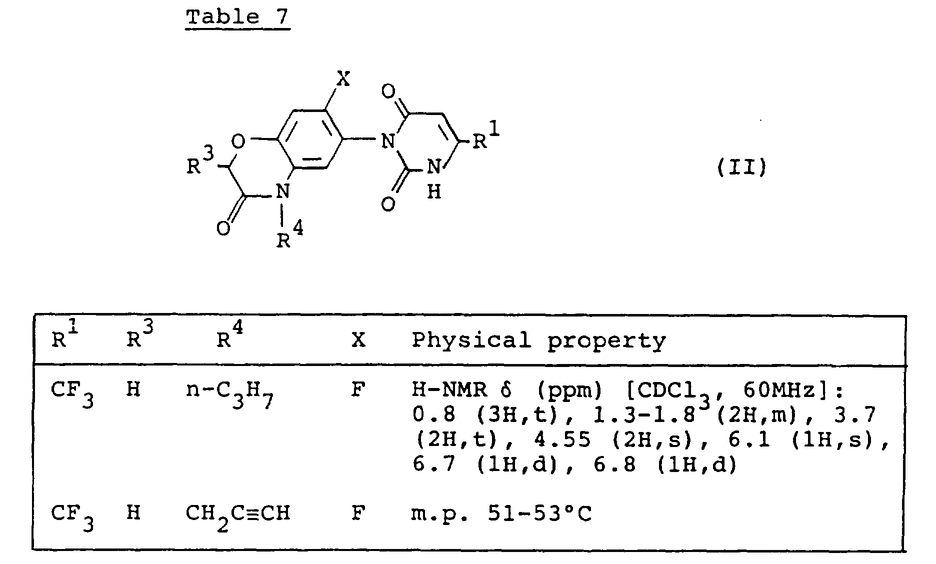

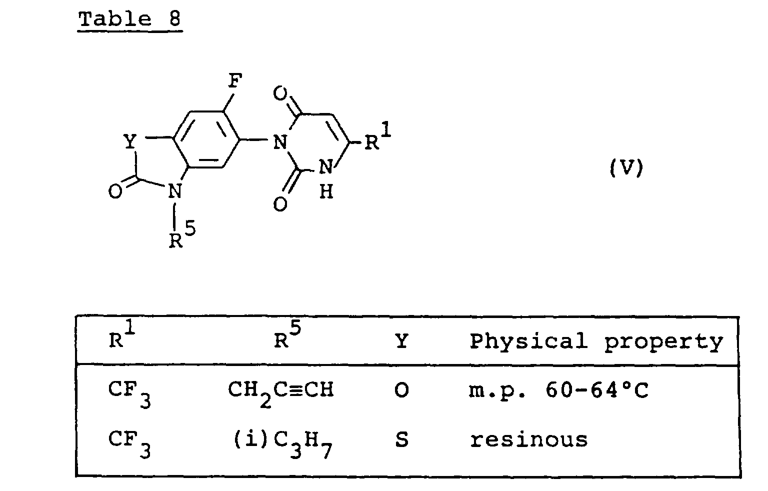

### (2) Preparation of the compound (VIII) or the compound (X) from the compound (IX) or the compound (XI):-

The compound (VIII) or the compound (X) may be produced by reacting the compound (IX) or the compound (XI) with a compound of the formula: 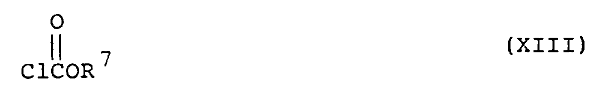 wherein R⁷ is a C₁-C₆ alkyl group in the existence of a dehydrohalogenating agent in the presence or absence of an inert solvent at a temparature of about 0 to 150°C for a period of about 0.5 to 10 hours.

Normally, the compound (XIII) and the dehydrohalogenating agent are used respectively in amounts of about 1 to 1.5 equivalents and of about 1 to 1.5 equivalents to one equivalent of the compound (IX) or the compound (XI). As the dehydrohalogenating agent, there may be used an organic base (e.g. pyridine, trithylamine, N,N-diethylaniline), an inorganic base (e.g. sodium hydroxide, potassium hydroxide, sodium carbonate, potassium carbonate, sodiums hydride), an alkali metal alkoxide (e.g. sodium methoxide, sodium ethoxide), etc.

Examples of the inert solvent are aromatic hydrocarbons (e.g. benzene, toluene, xylene), halogenated hydrocarbons (e.g. chloroform, carbon tetrachloride), ethers (e.g. diethyl ether, dioxane), etc.

After completion of the reaction, the reaction mixture is subjected to ordinary post-treatment. For instance, the reaction mixture is poured into water and extracted with an organic solvent, followed by concentration. If desired, any conventional purification procedure such as chromatography, distillation or recrystallization may be applied to the resulting product.

A typical embodiment for production of the compound (VIII) or the compound (X) is illustratively shown in the following Example.

### Example 5

A mixture of 6-amino-7-fluoro-4-propargyl-2H-1,4-benzoxazin-3(4H)-one (2.1 g), N,N-diethylaniline (1.5 g) and methyl chloroformate (1.0 g) was dissolved in 1,2-dichloroethane (10 g), and the resultant mixture was heated under reflux for 3 hours. After cooling, the reaction mixture was washed with water, and the organic layer was concentrated. The residue was washed with methanol to give 7-fluoro-6-methoxycarbonylamino-4-propargyl-2H-1,4-benzoxazin-3(4H)-one (2.0 g).

In the same manner as above, the compounds (VIII) and the compound (X) as shown in Tables 9 and Table 10 were obtained.

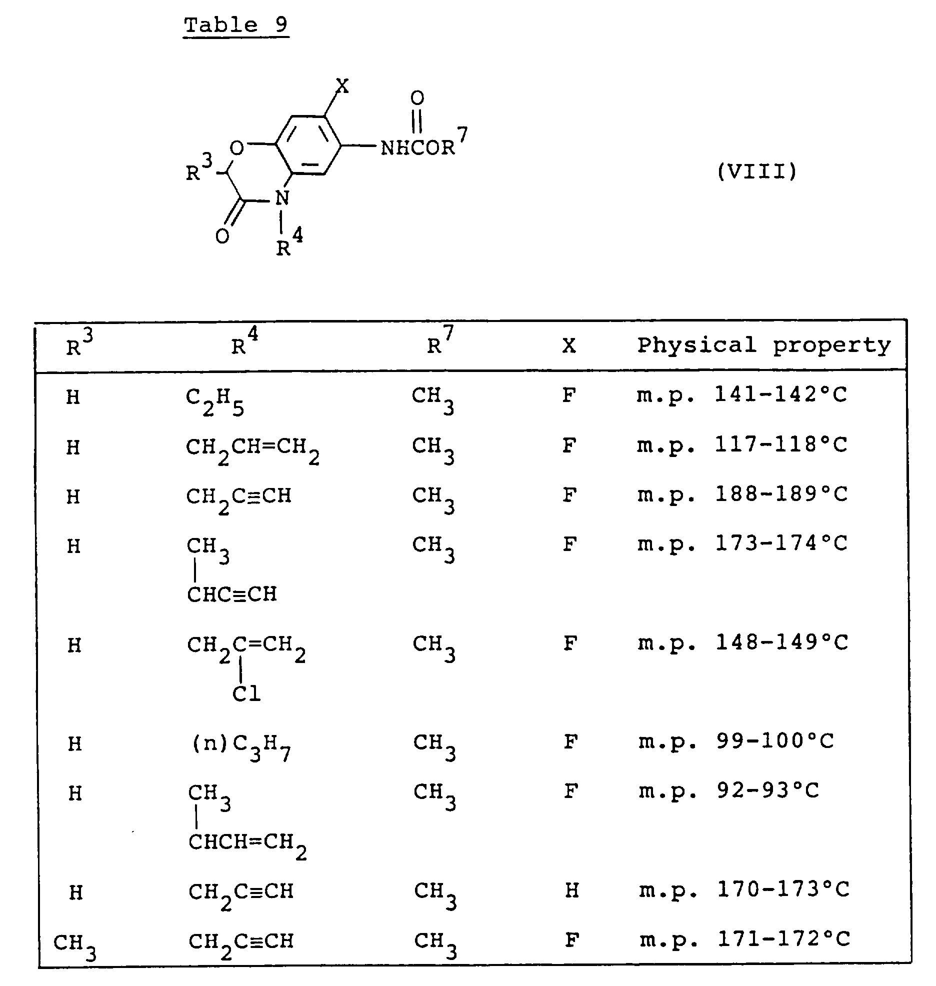

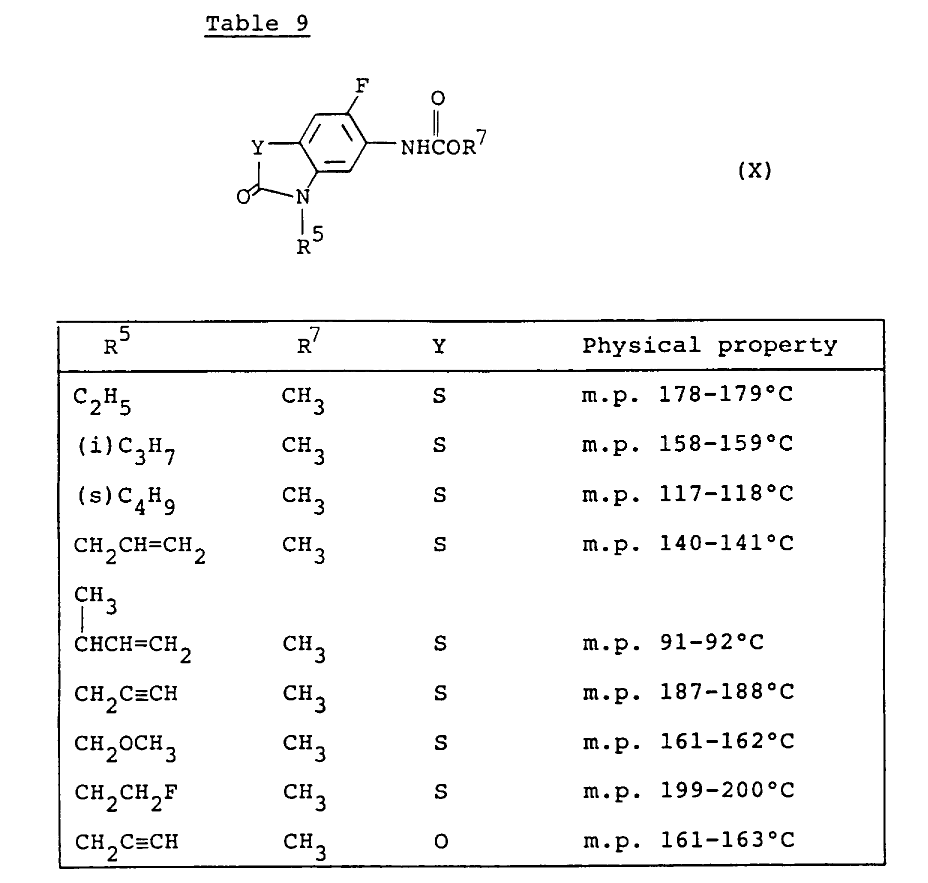

The compound (IX) can be produced by the method as disclosed in U.S. Pat. No. 4,640,707 and the compound (XI) can be produced by the method as disclosed in U.S. Pat. No. 4,640,707 or U.S. Pat. No. 4,720,297.

The starting compound (VI) may be prepared according to the following scheme: 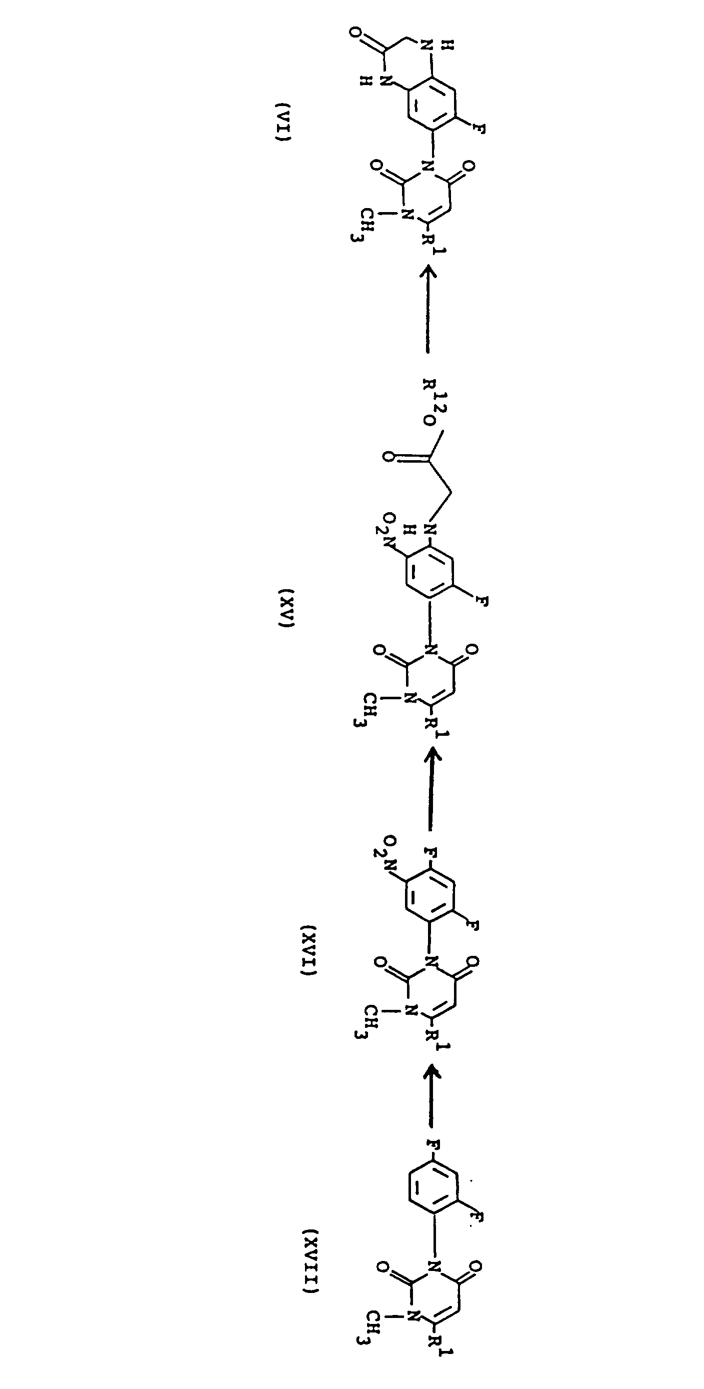 wherein R¹ is a C₁-C₆ alkyl group and R¹ is as defined above.

The reaction at each step in the above scheme will be hereinafter explained in detail.

### (3) Preparation of the compound (VI) from the compound (XV):-

The compound (VI) can be obtained by subjecting the compound (XV) to reductive cyclization. The reductive cyclization may be accomplished, for instance, by treatment of the compound (XV) with a reducing agent such as iron powders or by catalytic reduction of or the compound (XV).

Treatment with iron powders may be carried out using iron powders in an amount of about 2.5 to 10 equivqlents to one equivalent of the compound (XV) in the presence of a small amount of an acid (e.g. acetic acid, chloric acid) in an inert solvent at a temperature of about 50 to 200 °C for a period of about 0.5 to 10 hours. Examples of the solvent are toluene, 1,2-dichloroethane, methyl isobuthyl ketone, acetic acid, water, etc. Thier mixtures are also usable.

Catalytic reduction may be performed in the presence of a catalytic amount of palladium-carbon in an inert solvent (e.g. methanol, ethanol) at room temperature for a period of about 0.5 to 20 hours.

After completion of the reaction, the reaction mixture may be subjected to ordinary post-treatment. For instance, the reaction mixture is filtered, the filtrate is combined with water, and the resultant mixture is extracted with an organic solvent, followed by concentration. When desired, any conventional purification procedure such as recrystallization or column chromatography may be applied to the resulting product.

Typical examples for production of the compound (VI) are illustratively shown in the following Example.

### Example 6

A suspension of l-(2-fluoro-4-ethoxycarbonylmethylamino-5-nitrophenyl)-3-methyl-4-trifluoromethyl-1,2,3,6-tetrahydropyrimidine-2,6-dione (11.2 g), water (3 g) and iron powders (9 g) in acetic acid (30 g) was refluxed for 3 hours. After completion of the reaction, the reaction mixture was filtered by the use of celite, and the filtrate was combined with water and extracted with ethyl acetate. The organic layer was concentrated, and the residue was purified by column chromatography to give 1-[6-fluoro-1,2,3,4-tetrahydroquinoxalin-2-on-7-yl]-3-methyl-4-trifluoromethyl-1,2,3,6-tetrahydropyrimidine-2,6-dione (7.6 g). m.p., 162 - 163°C.

### (5) Preparation of the compound (XV) from the compound (XVI):-

The compound (XV) may be produced by reacting the compound (XVI) with a compound of the formula: 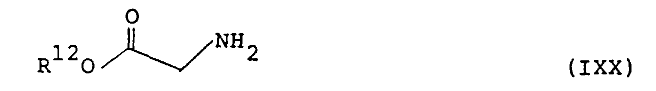 wherein R¹ is as defined above, usually in the presence of a base in an inert solvent at a temperature of about 25 to 200°C for a period of about 0.5 to 10 hours. In this reaction, the compound (IXX) and the base may be respectively used in amounts of about 1.0 to 1.2 equivalents and of about 1.0 to 4.0 equivalents to the compound (XVI). As the base, there may be used an organic base such as triethylamine. Examples of the solvent are aromatic hydrocarbons (e.g. toluene, xylene), halogenated hydrocarbons (e.g. dichloromethane, 1,2-dichloroethane), ethers (e.g. tetrahydrofuran, dioxane), etc.

After completion of the reaction, the reaction mixture is subjected to ordinary post-treatment. For instance, the reaction mixture is poured into water and extracted with an organic solvent, followed by concentration. If desired, any conventional purification procedure such as chromatography and recrystallization may be adopted.

A typical embodiment for production of the compound (XV) is illustratively shown in the following Example.

### Example 7

A suspension of 1-(2,4-difluoro-5-nitrophenyl)-3-methyl-4-trifluoromethyl-1,2,3,6-tetrahydropyrimidine-2,6-dione (10 g), ethyl aminoacetate (5 g) and triethylamine (6.1 g) in dioxane (50 g) was refluxed for 4 hours. After completion of the reaction, the reaction mixture was poured into water and extracted with ethyl acetate. The extract was dried over magnesium sulfate and concentrated. The residue was purified by silica gel column chromatography using a mixture of hexane and ethyl acetate as an eluent to give 1-(2-fluoro-4-ethyloxycarbonylmethylamino-5-nitrophenyl)-3-methyl-4-trifluoromethyl-1,2,3,6-tetrahydropyrimidine-2,6-dione (12 g).

H-NMR δ (ppm) [CDCl₃, 60 MHz]: 1.33 (2H, t), 3.53 (3H, s), 4.0 (2H, d), 4.27 (2H, q), 6.27 (1H, s), 6.43 (1H, d), 8.11 (1H, d), 8.3-8.8 (1H, m).

### (6) Preparation of the compound (XVI) from the compound (XVII):-

The compound (XVI) is obtainable by nitrating the compound (XVII) with nitric acid in conc. sulfuric acid normally at a temperature of about 0 to 30°C for a period of 0.3 to 3 hours. In the nitration, nitric acid is used in an amount of about 1 to 1.5 equivalents to one equivalent of the compound (XVII). Post-treatment of the reaction mixture after completion of the nitration may be carried out in a per se conventional manner.

A typical embodiment for production of the compound (XVI) is illustratively shown in the following Example.

### Example 8

A solution of 1-(2.4-difluorophenyl)-3-methyl-4-trifluoromethyl-1,2,3,6-tetrahydropyrimidine-2,6-dione (30.6 g) in conc. sulfuric acid (100 g) was cooled to 5°C, 61 % nitric acid (10.3 g) was dropwise added thereto at a temperature below 10°C, followed by stirring for 3 hours. After completion of the reaction, the reaction mixture was poured into water, and the precipitated crystals were collected by filtration and dried to give 1-(2,4-difluoro-5-nitrophenyl)-3-methyl-4-trifluoromethyl-1,2,3,6-tetrahydropyrimidine-2,6-dione (33 g). m.p., 156 - 157°C.

For the practical usage of the compound (I), it is usually formulated with conventional solid or liquid carriers or diluents as well as surface active agents or auxiliary agents into conventional preparation forms such as emulsifiable concentrates, wettable powders, suspensions, water dispersible granules and granules. The content of the compound (I) as the active ingredient in such preparation forms is normally within a range of about 0.02 to 80 % by weight, preferably of about 0.05 to 70 % by weight. Examples of the solid carrier or diluent are fine powders or granules of kaolin clay, attapulgite clay, bentonite, terra alba, pyrophyllite, talc, diatomaceous earth, calcite, walnut powders, urea, ammonium sulfate and synthetic hydrous silicate, etc. As the liquid carrier or diluent, there may be exemplified aromatic hydrocarbons (e.g. xylene, methylnaphthalene), alcohols (e.g. isopropanol, ethylene glycol, cellosolve), ketones (e.g. acetone, cyclohexanone, isophorone), soybean oil, cotton seed oil, dimethylsulfoxide, N,N-dimethylformamide, acetonitrile, water, etc.

The surface active agent used for emulsification, dispersion or spreading may be of any type, for instance, either anionic or non-ionic. Example of the surface active agent include alkylsulfates, alkylarylsulfonates, dialkylsulfosuccinates, phosphates of polyoxyethylenealkylaryl ethers, polyoxyethylene alkyl ethers, polyoxyethylene alkylaryl ethers, polyoxyethylene polyoxypropylene block copolymer, sorbitan fatty acid esters, polyoxyethylene sorbitan fatty acid esters, etc. Examples of the auxiliary agents include ligninsulfonates, sodium alginate, polyvinyl alcohol, gum arabic, CMC (carboxymethyl cellulose), PAP (isopropyl acid phosphate), etc.

Practical embodiments of the herbicidal composition according to the present invention are illustratively shown in the following examples wherein parts are by weight. The number of the active ingredient corresponds to the one in Tables 2, 4 and 6.

### Formulation Example 1

Fifty parts of any one of Compound Nos. 2, 3 or 6 to 13, 3 parts of calcium ligninsulfonate, 2 parts of sodium laurylsulfate and 45 parts of synthetic hydrous silicate are well mixed while being powdered to obtain a wettable powder.

### Formulation Example 2

Five parts of any one of Compound Nos. 1 to 13, 14 parts of polyoxyethylenestyrylphenyl ether, 6 parts of calcium dodecylbenzenesulfonate, 25 parts of xylene and 50 parts of cyclohexanone are well mixed to obtain an emulsifiable concentrate.

### Formulation Example 3

Two parts of any one of Compound Nos. 1 to 13, 1 part of synthetic hydrous silicate, 2 parts of calcium ligninsulfonate, 30 parts of bentonite and 65 parts of kaolin clay are well mixed while being powdered. The mixture is then kneaded with water, granulated and dried to obtain granules.

### Formulation Example 4

Twenty-five parts of any one of Compound Nos. 2, 3 or 6 to 13 are mixed with 3 parts of polyoxyethylene sorbitan monooleate, 3 parts of carboxymethyl cellulose and 69 parts of water and pulverized until the particle size of the mixture becomes less than 5 microns to obtain a suspension.

### Formulation Example 5

0.05 Part of any one of Compound Nos. 1 to 13, 1 part of synthetic hydrous silicate, 2 parts of calcium ligninsulfonate, 30 parts of bentonite and 66.95 parts of kaolin clay are well mixed while being powdered. The mixture is then kneaded with water, granulated and dried to obtain granules.

The compound (I) thus formulated in any suitable preparation form is useful for pre-emergence or postemergence control of undesired weeds by soil or foliar treatment as well as flood fallowing treatment. These treatments include application to the soil surface prior to or after planting, incorporation into the soil prior to planting or transplanting, etc. The foliar treatment may be effected by spraying the herbicidal composition containing the compound (I) over the top of plants. It may also be applied directly to the weeds if care is taken to keep the chemical off the crop foliage.

The compound (I) may be used together with any other herbicide to improve its activity as a herbicide, and in some cases, a synergistic effect can be expected. Further, it may be applied in combination with an insecticide, an acaricide, a nematocide, a fungicide, a plant growth regulator, a fertilizer, a soil improver, etc. It is also useful as a herbicide to be employed for orchards, pasture lands, lawns, forests, non-agricultural fields, etc.

The dosage of the compound (I) may vary depending on the prevailing weather conditions, the formulation used, the prevailing season, the mode of application, the soil involved, the crop and weed species, etc. Generally, however, the dosage is from about 0.01 to 80 grams, preferably from about 0.02 to 40 grams, of the active ingredient per are. The herbicidal composition of the invention formulated in the form of an emulsifiable concentrate, a wettable powder, a water-dispersible granule or a suspension may ordinarily be employed by diluting it with water at a volume of about 1 to 10 liters per are, if necessary with addition of an auxiliary agent such as a spreading agent. Examples of the spreading agent include, in addition to the surface active agents as noted above, polyoxyethylene resin acid (ester), ligninsulfonate, abietylenic acid salt, dinaphthylmethanedisulfonate, paraffin, etc. The composition formulated in the form of granules may be normally applied as such without dilution.

The biological data of the compound (I) as herbicides will be illustratively shown in the following Examples wherein the phytotoxicity to crop plants and the herbicidal activity on weeds were observed visually as to the degree of germination as well as the growth inhibition and rated with an index 0, 1, 2, 3, 4, or 5, the numeral "0" indicating no material difference as seen in comparison with the untreated plants and the numeral "5" indicating the complete inhibition or death of the test plants.

The compounds as shown in Table 11 were used for comparison.

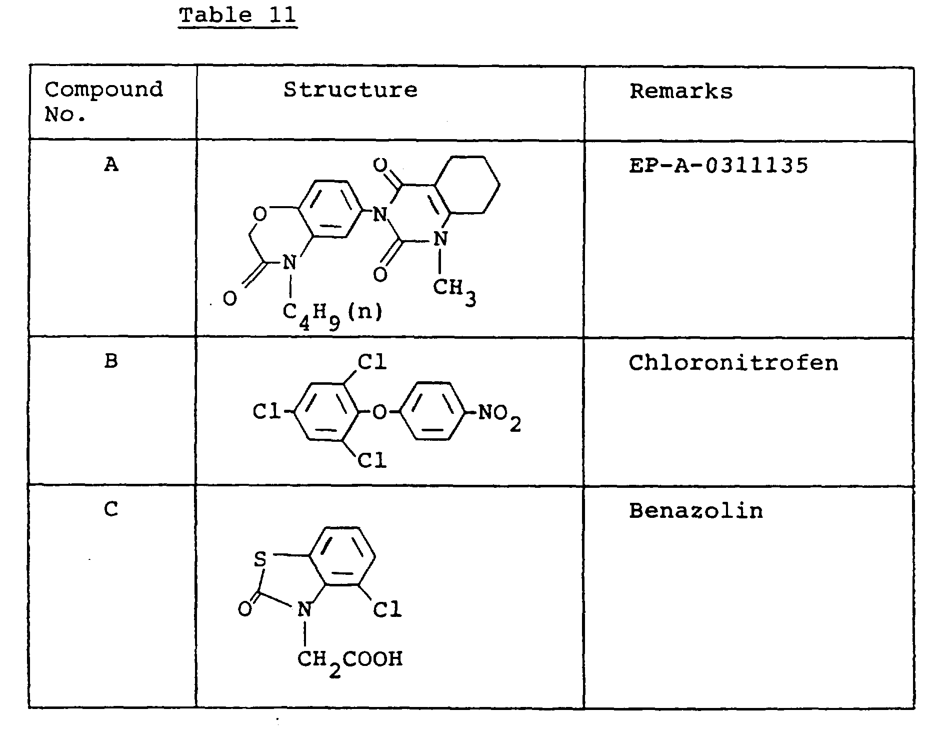

### Test Example 1

Cylindrical plastic pots (diameter, 10 cm; height, 10 cm) were filled with upland field soil, and the seeds of Japanese millet, oats, tall morningglory and velvetleaf were sowed therein and covered with soil. A designated amount of the test compound formulated in an emulsifiable concentrate as in Formulation Example 2 was diluted with water, and the dilution was sprayed onto the soil surface by means of a small hand sprayer at a spray volume of 10 liters per are. The test plants were grown in a greenhouse for 20 days, and the herbicidal activity was examined. The results are shown in Table 12.

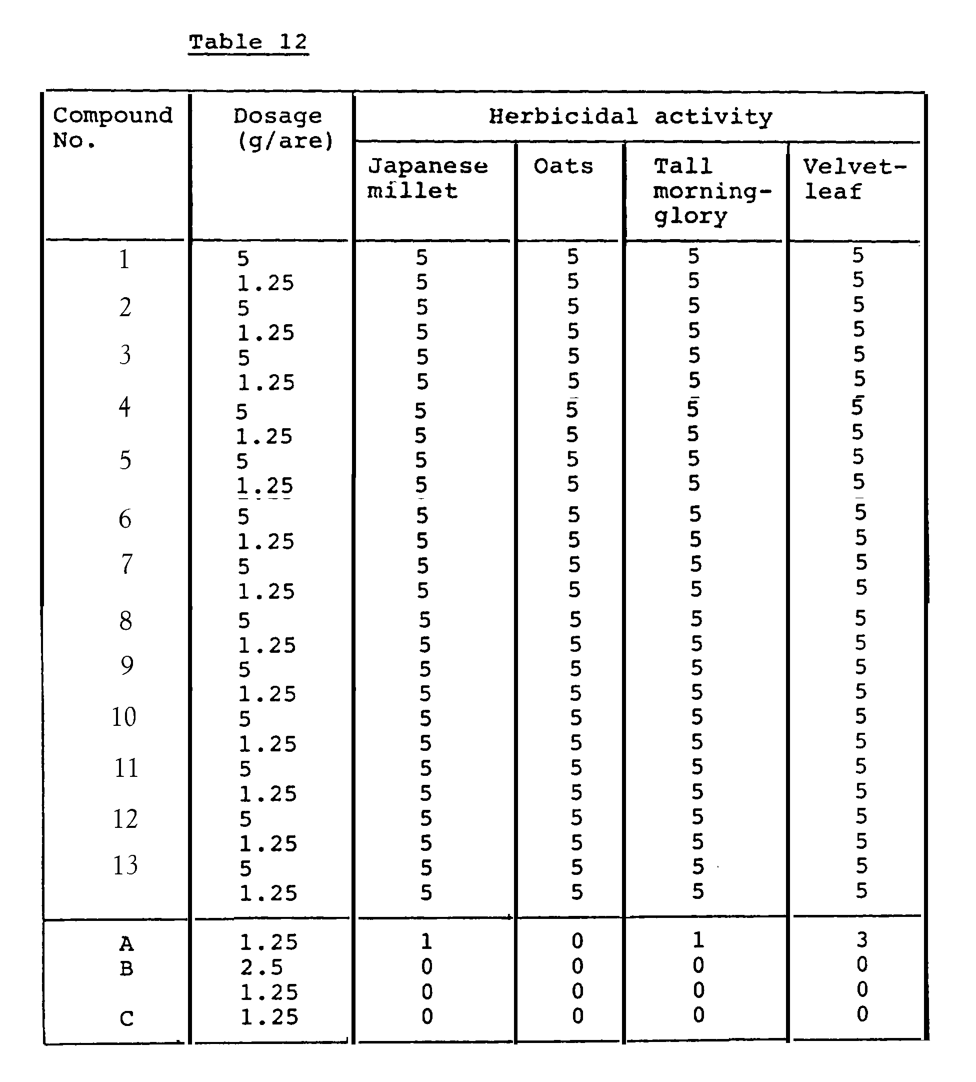

### Test Example 2

Cylindrical plastic pots (diameter, 10 cm; height, 10 cm) were filled with upland field soil, and the seeds of Japanese millet, tall morningglory, radish, velvetleaf and oats were sowed therein and cultivated in a greenhouse for 10 days. A designated amount of the test compound formulated in an emulsifiable concentrate as in Formulation Example 2 was diluted with water containing a spreading agent, and the dilution was sprayed over the foliage of the test plant by means of a small hand sprayer at a spray volume of 10 liters per are. The test plants were further grown in the greenhouse for 20 days, and the herbicidal activity was examined. The results are shown in Table 13.

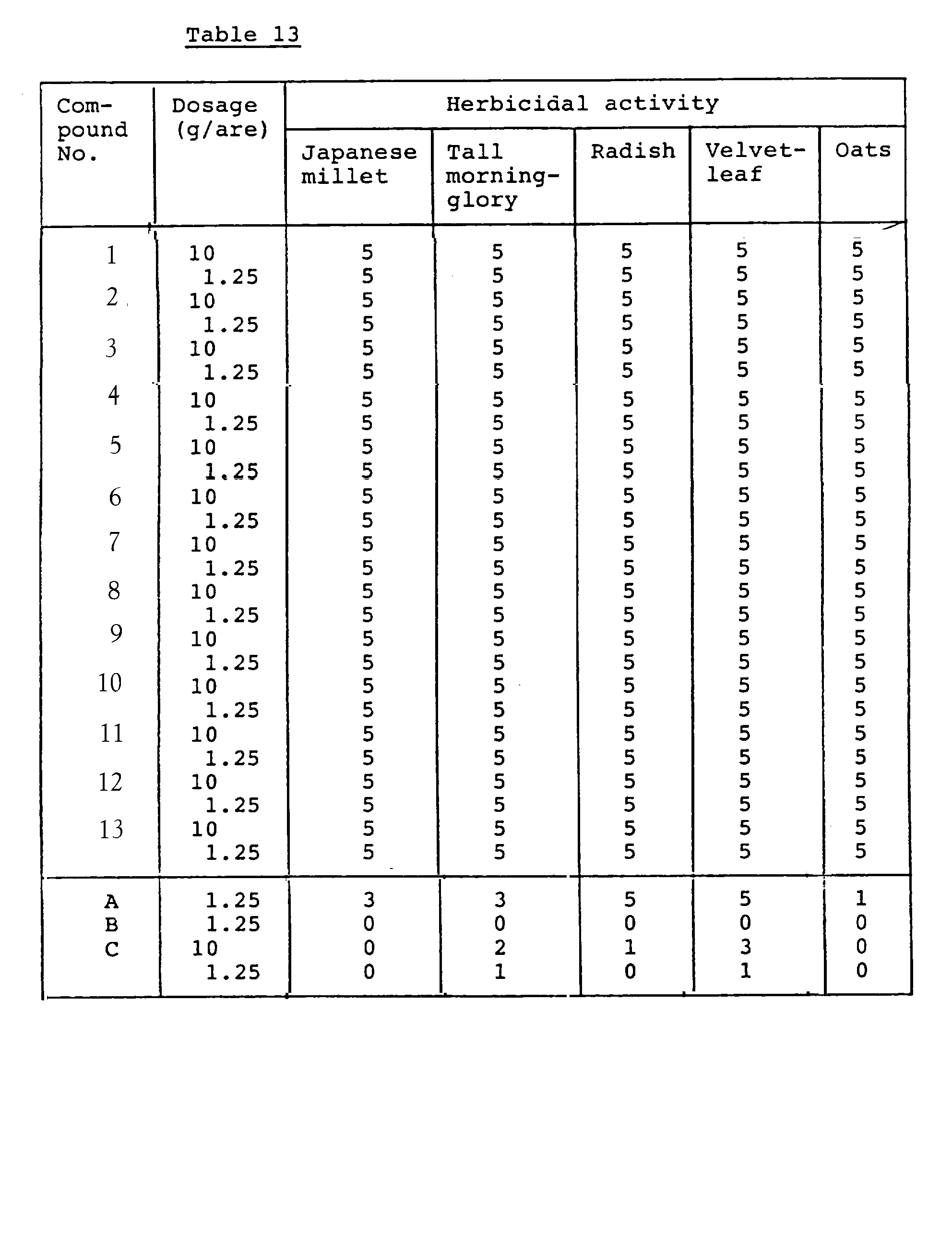

### Test Example 3

Vats (33 cm x 23 cm x 11 cm) were filled with upland field soil, and the seeds of rice plant, soybean, corn, tall morningglory, velvetleaf, black nightshade, johnsongrass and green foxtail were sowed therein in 1 to 2 cm depth. A designated amount of the test compound formulated in an emulsifiable concentrate as in Formulation Example 2 was diluted with water, and the dilution was sprayed onto the soil surface by means of a small hand sprayer at a spray volume of 10 liter per are. The test plants were grown in a greenhouse for 20 days, and the herbicidal activity and phytotoxicity were examined. The results are shown in Table 14.

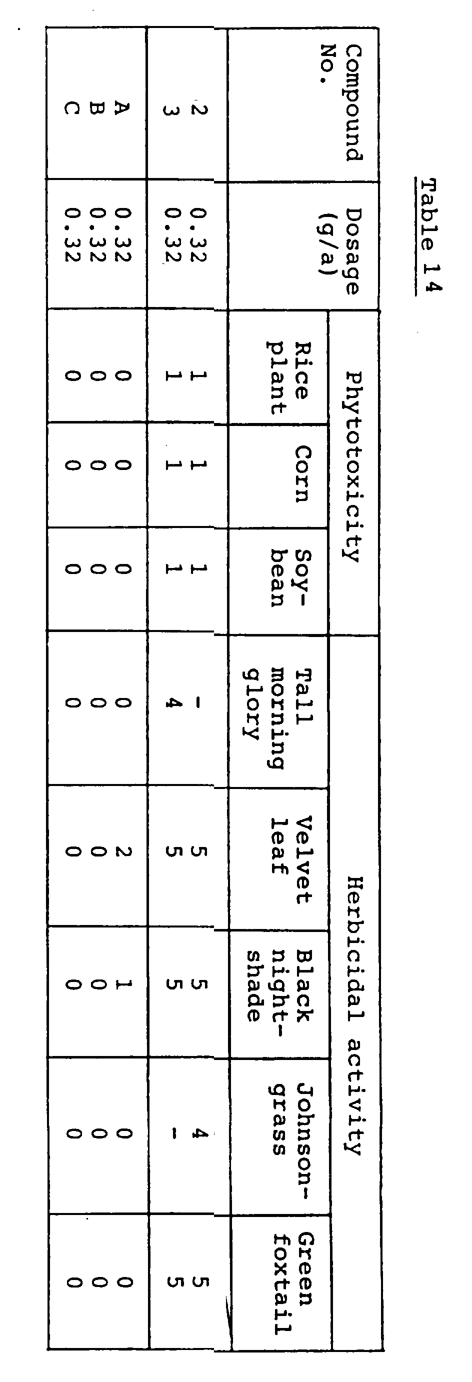

### Test Example 4

Vats (33 cm x 23 cm x 11 cm) were filled with upland field soil, and the seeds of wheat, barley, pale smartweed, persian speedwell, field pansy and catchweed bedstraw were sowed therein in 1 to 2 cm depth. A designated amount of the test compound formulated in an emulsifiable concentrate as in Formulation Example 2 was diluted with water, and the dilution was sprayed onto the soil surface by means of a small hand sprayer at a spray volume of 10 liters per are. The test plants were grown in a greenhouse for 27 days, and the herbicidal activity and phytotoxicity were examined. The results are shown in Table 15.

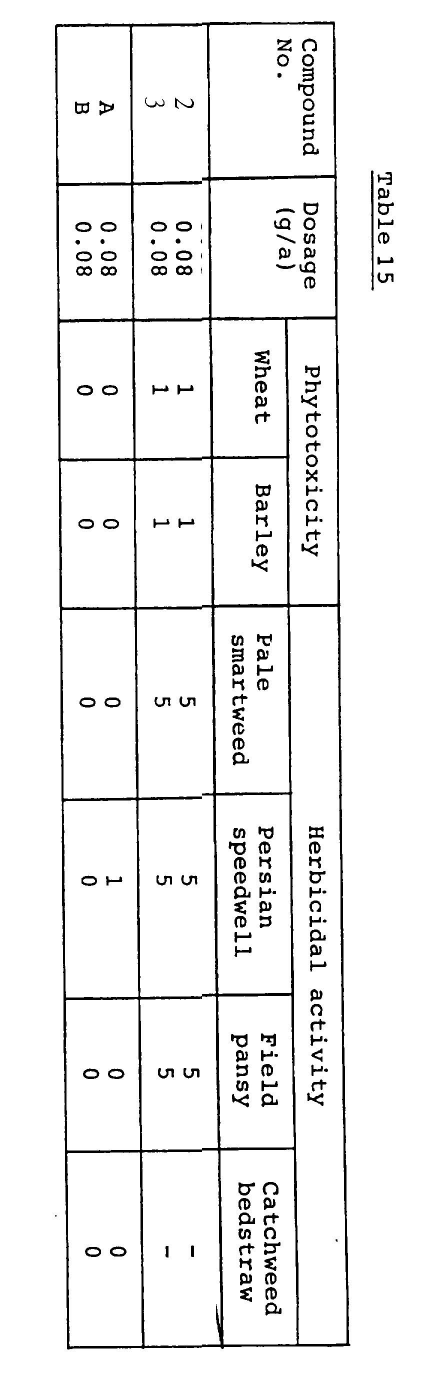

### Test Example 5

Vats (33 cm x 23 cm x 11 cm) were filled with upland soil, and the seeds of wheat, barley, pale smartweed, common chickweed, persian speedwell and field pansy were sowed therein in 1 to 2 cm depth. A designated amount of the test compound formulated in an emulsifiable concentrate as in Formulation Example 2 was diluted with water, and the dilution was sprayed onto the soil surface by means of a small hand sprayer at a spray volume of 10 liters per are. The test plants were grown in a greenhouse for 27 days, and the herbicidal activity and phytotoxicity were examined. The results are shown in Table 16.

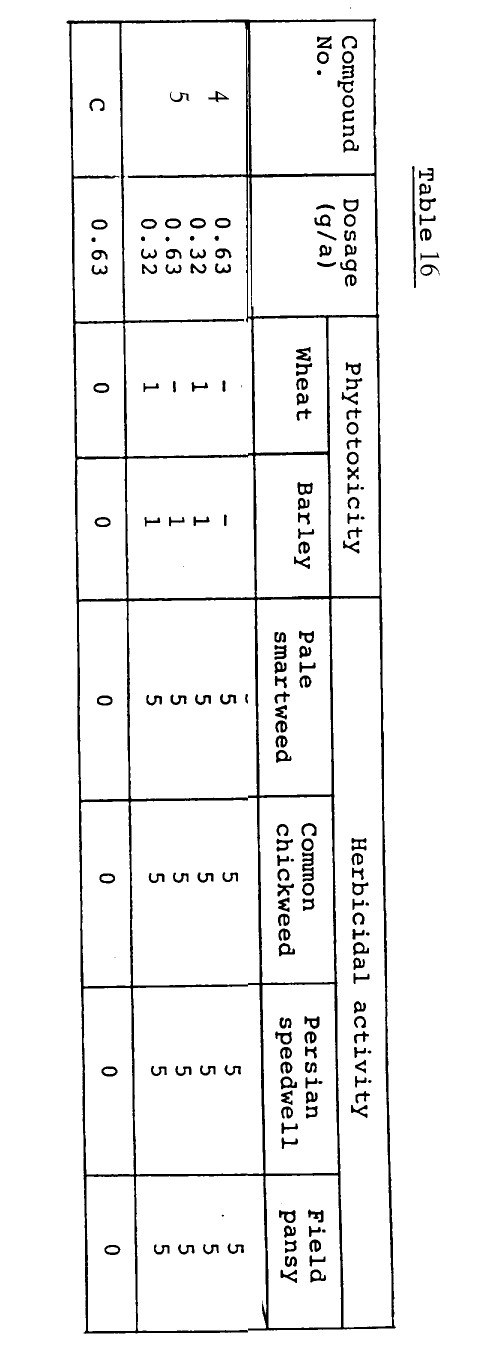

### Test Example 6

Vats (33 cm x 23 cm x 11 cm) were filled with upland field soil, and the seeds of corn, rice plant, tall morningglory, common cocklebur, velvetleaf, sicklepod and black nightshade were sowed therein and cultivated for 18 days in a greenhouse. A designated amount of the test compound formulated in an emulsifiable concentrate as in Formulation Example 2 was diluted with water, and the dilution was sprayed over the foliage of the test plants by means of a small hand sprayer at a spray volume of 10 liters per are. The test plants were further grown in the greenhouse for 20 days, and the herbicidal activity and phytotoxicity were examined. At the time of the application, the test plants were generally at the 1 to 4 leaf stage and in 2 to 12 cm height, although the growing stage of the test plants varied depending on their species. The results are shown in Table 17.

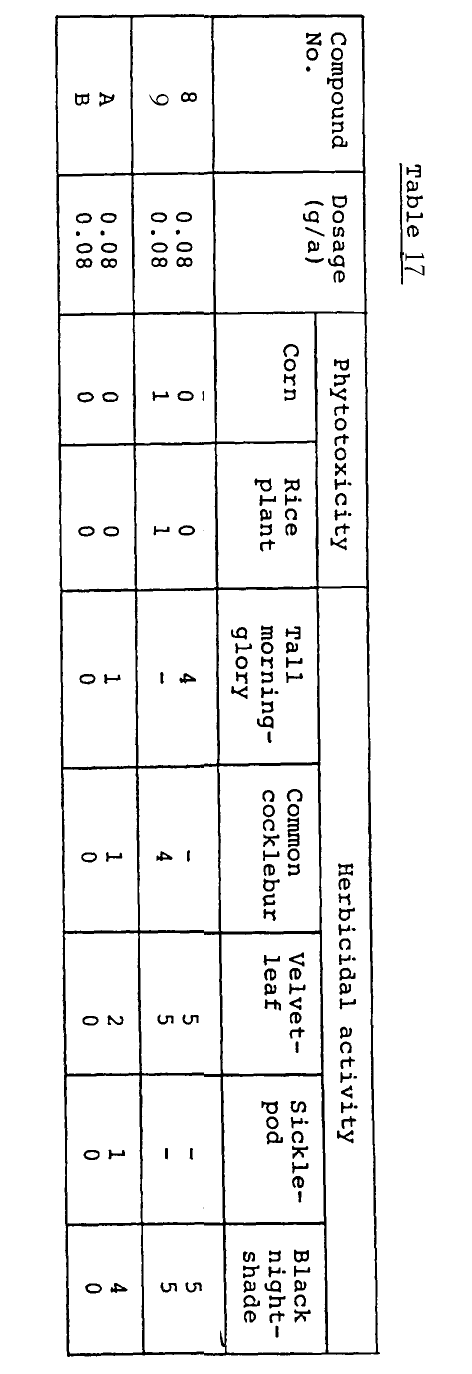

### Test Example 7

Vats (33 cm x 23 cm x 11 cm) were filled with upland field soil, and the seeds of wheat, barley, pale smartweed, catchweed bedstraw, common chickweed, persean speedwell and field pansy were sowed therein and cultivated for 25 days in a greenhouse. A designated amount of the test compound formulated in an emulsifiable concentrate as in Formulation Example 2 was diluted with water, and the dilution was sprayed over the foliage of the test plants by means of a small hand sprayer at a spray volume of 10 liters per are. The test plants were further grown in the greenhouse for 27 days, and the herbicidal activity and phytotoxicity were examined. At the time of the application, the test plants were generally at the 1 to 4 leaf stage and in 2 to 12 cm height, although the growing stage of the test plants varied depending on their species. The results are shown in Table 18.

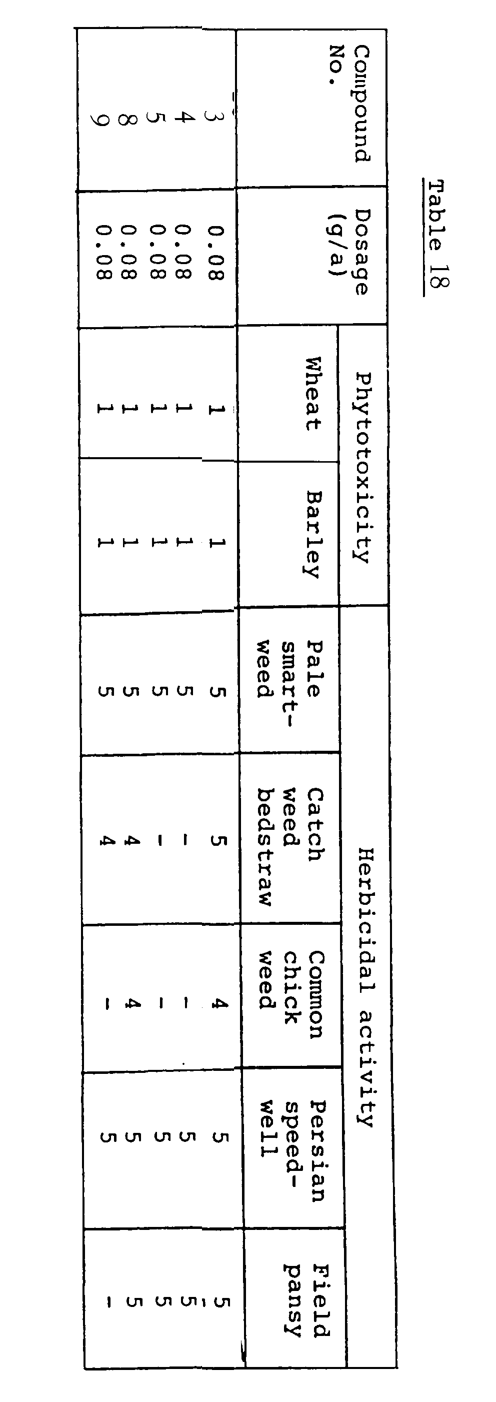

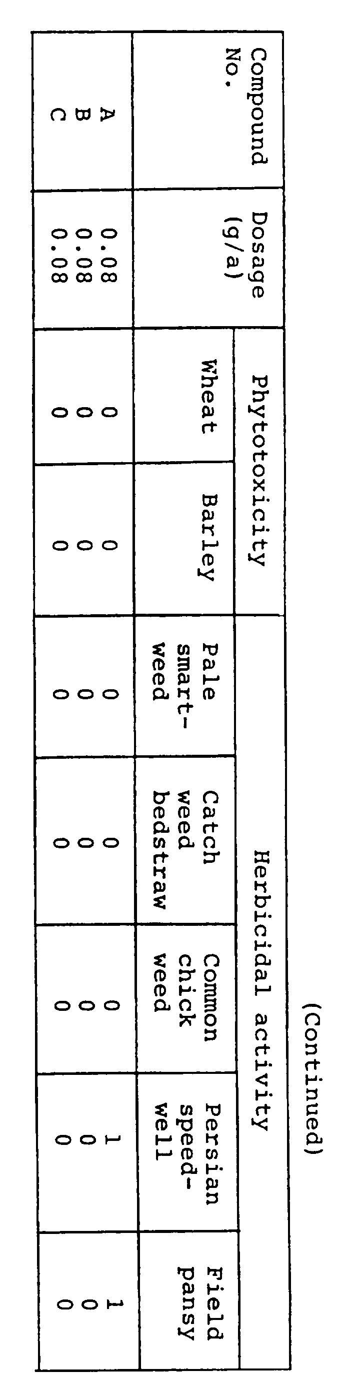

### Test Example 8

Vats (33 cm x 23 cm x 11cm) were filled with upland field soil, and the seeds of cotton, corn, rice plant, velvetleaf, common cocklebur, tall morningglory, black nightshade, redroot pigweed and hemp sesbania were sowed therein in 1 to 2 cm depth. A designated amount of the test compound formulated in an emulsifiable concentrate as in Formulation Example 2 was diluted with water, and the dilution was sprayed onto the soil surface by means of a small hand sprayer at a spray volume of 10 liters per are. The test plants were grown in a greenhouse for 20 days, and the herbicidal activity and phytotoxicity were examined. The results are shown in Table 19.

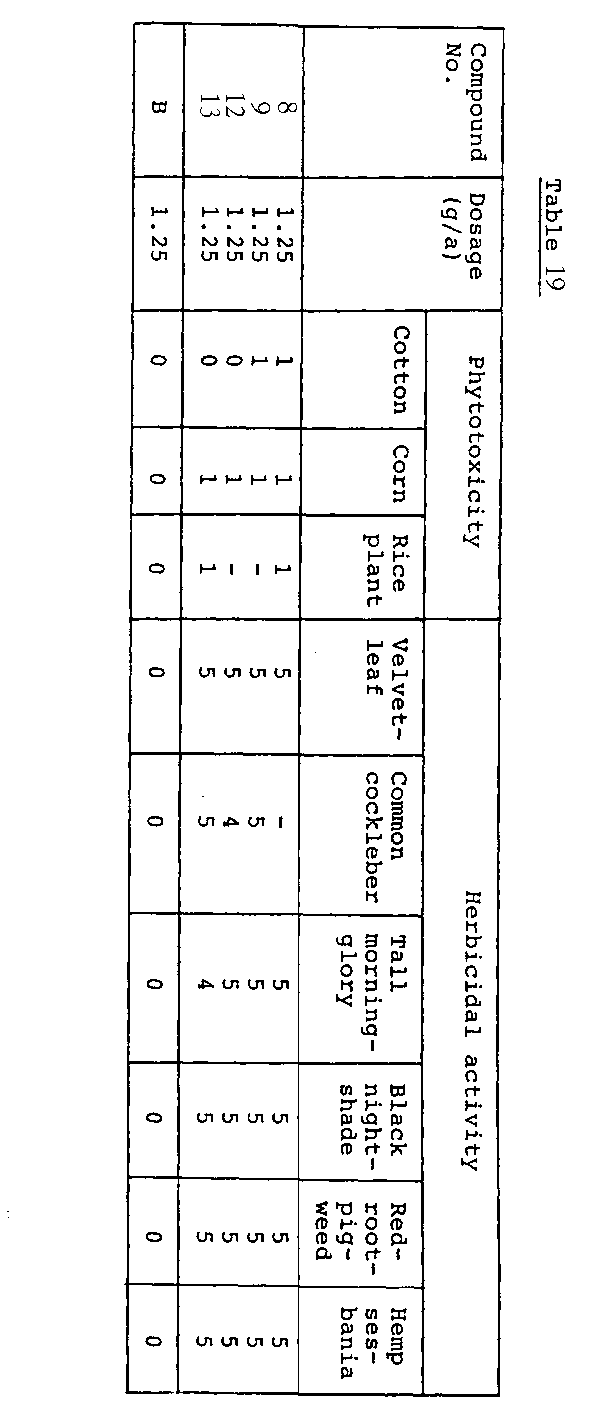

### Test Example 9

Vats (33 cm x 23 cm x 11cm) were filled with upland field soil, and the seeds of corn, rice plant, velvetleaf, tall morningglory, black nightshade, redroot pigweed and green foxtail were sowed therein in 1 to 2 cm depth. A designated amount of the test compound formulated in an emulsifiable concentrate as in Formulation Example 2 was diluted with water, and the dilution was sprayed onto the soil surface by means of a small hand sprayer at a spray volume of 10 liters per are. The test plants were grown in a greenhouse for 20 days, and the herbicidal activity and phytotoxicity were examined. The results are shown in Table 20.

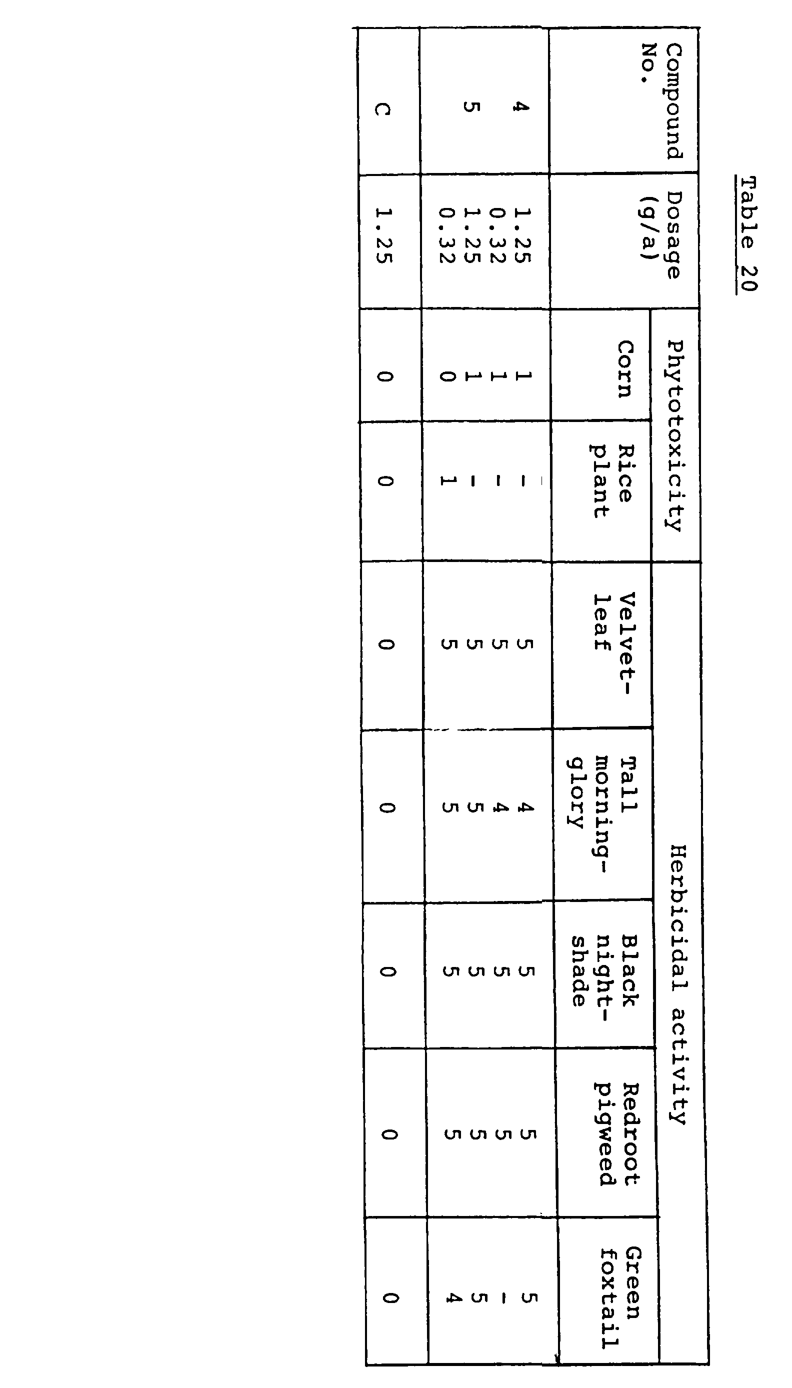

### Test Example 10

Vats (33 cm x 23 cm x 11 cm) were filled with upland field soil, and the seeds of barnyardgrass, johnsongrass, green foxtail, large crabgrass were sowed therein in 1 to 2 cm depth. A designated amount of the test compound formulated in an emulsifiable concentrate as in Formulation Example 2 was diluted with water, and the dilution was sprayed onto the soil surface by means of a small hand sprayer at a spray volume of 10 liters per are. The test plants were grown in a greenhouse for 20 days, and the herbicidal activity was examined. The results are shown in Table 21.

**Table 21**

| Compound No. | Dosage (g/a) | Herbicidal activity | | | |
|---|---|---|---|---|---|
| | | Barnyard-grass | Johnson-grass | Green foxtail | Large crab-grass |
| 1 | 5 | 5 | 5 | 5 | 5 |
| 2 | 5 | 5 | 5 | 5 | 5 |
| 3 | 5 | 5 | 5 | 5 | 5 |
| 8 | 5 | 5 | 5 | 5 | 5 |
| 9 | 5 | 5 | 5 | 5 | 5 |
| 12 | 5 | 5 | 5 | 5 | 5 |
| 13 | 5 | 5 | 5 | 5 | 5 |

### Test Example 11

Vats (33 cm x 23 cm x 11 cm) were filled with upland field soil, and the seeds of corn, common cocklebur, velvetleaf, tall morningglory and black nightshade were sowed therein and cultivated for 18 days in a greenhouse. A designated amount of the test compound formulated in an emulsifiable concentrate as in Formulation Example 2 was diluted with water, and the dilution was sprayed over the foliage of the test plants by means of a small hand sprayer at a spray volume of 10 liters per are. The test plants were further grown in the greenhouse for 20 days, and the herbicidal activity and phytotoxicity were examined. At the time of the application, the test plants were generally at the 1 to 4 leaf stage and in 2 to 12 cm height, although the growing stage of the test plants varied depending on their species. The results are shown in Table 22.

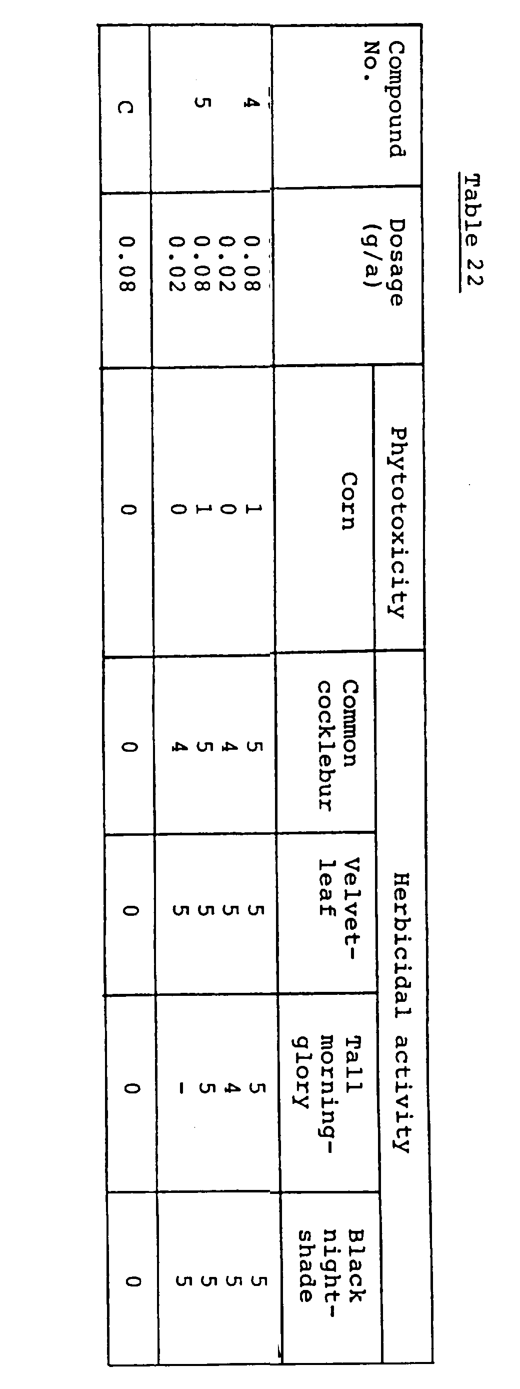

### Test Example 12

Vats (33 cm x 23 cm x 11 cm) were filled with upland field soil, and the seeds of corn, common cocklebur, velvetleaf, tall morningglory, sicklepod, black nightshade, barnyardgrass and johnsongrass were sowed therein and cultivated for 18 days in a greenhouse. A designated amount of the test compound formulated in an emulsifiable concentrate as in Formulation Example 2 was diluted with water, and the dilution was sprayed over the foliage of the test plants by means of a small hand sprayer at a spray volume of 10 liters per are. The test plants were further grown in the greenhouse for 20 days, and the herbicidal activity and phytotoxicity were examined. At the time of the application, the test plants were generally at the 1 to 4 leaf stage and in 2 to 12 cm height, although the growing stage of the test plants varied depending on their species. The results are shown in Table 23.

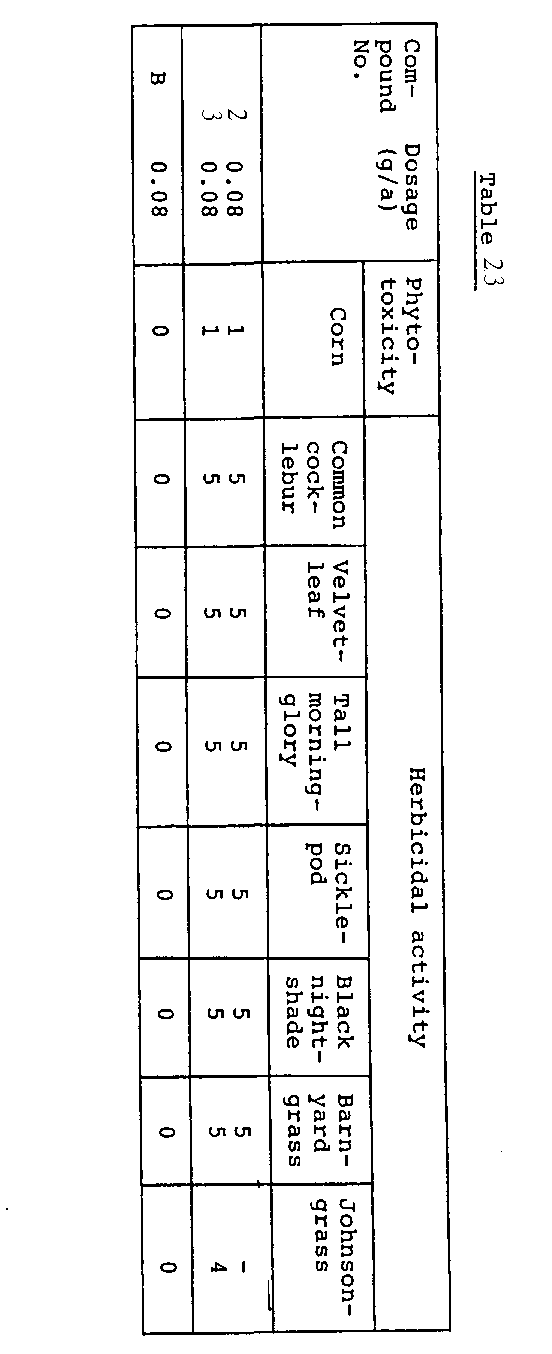

## Claims (Claims for the following Contracting State(s): BE, CH, DE, FR, GB, IT, LI, NL)

1. A compound of the formula I 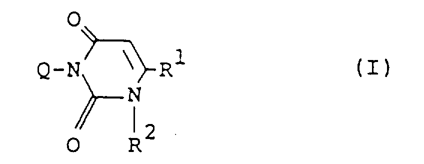 wherein R¹ is a trifluoromethyl group or a pentafluoroethyl group, R is a methyl group or an amino group and Q is either one of the formulas Q₁, Q₂ or Q₃ 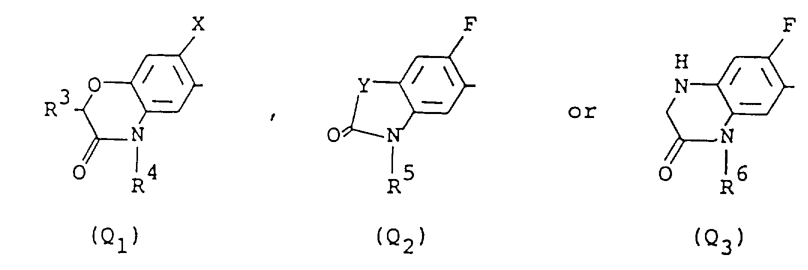 wherein R³ is a hydrogen atom or a methyl group, R⁴, R⁵ and R⁶ are each a C₁-C₇ alkyl group, a C₃-C₇ alkenyl group, a C₃-C₇ alkynyl group, a halo(C₁-C₆)alkyl group, a halo(C₃-C₆)alkenyl group or a C₁-C₄ alkoxy(C₁-C₃)alkyl group, X is a hydrogen atom or a fluorine atom and Y is an oxygen atom or a sulfur atom, with the proviso that when Q is Q₃, R represents a methyl group, and when Q is Q₁ or Q₂, R represents an amino group.

2. The compound according to claim 1, which has the formula: 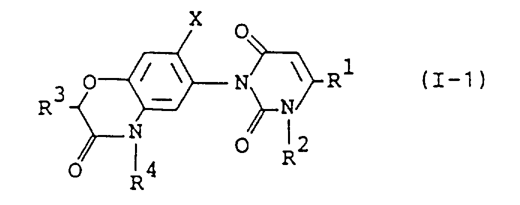 wherein R¹, R, R³, R⁴ and X are each as defined in claim 1.

3. The compound according to claim 1, which has the formula: 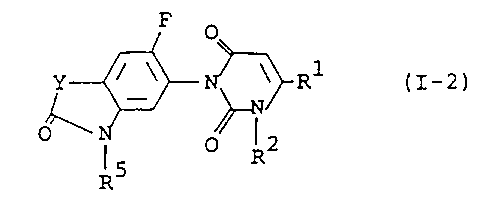 wherein R¹, R, R⁵ and Y are each as defined in claim 1.

4. The compound according to claim 1, which has the formula: 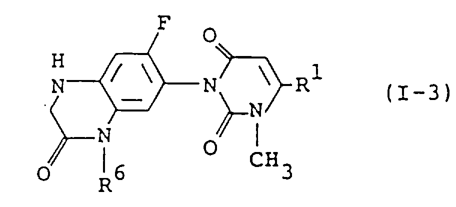 wherein R¹ and R⁶ are each as defined in claim 1.

5. The compound according to claim 2, wherein R³ is a hydrogen atom, R⁴ is a C₃-C₇ alkynyl group and X is a fluorine atom.

6. The compound according to claim 3, wherein Y is a sulfur atom.

7. The compound according to claim 6, wherein R⁵ is a C₁-C₇ alkyl group.

8. A process for preparing a compound of the formula I-1: 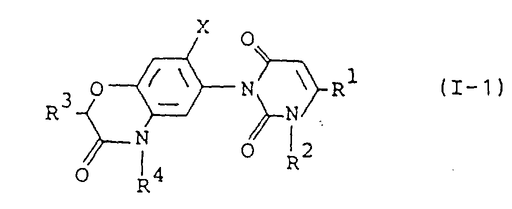 wherein R¹ is a trifluoromethyl group or a pentafluoroethyl group, R is an amino group, R³ is a hydrogen atom or a methyl group, R⁴ is a C₁-C₇ alkyl group, a C₃-C₇ alkenyl group, a C₃-C₇ alkynyl group, a halo(C₁-C₆)alkyl group, a halo(C₃-C₆)alkenyl group or a C₁-C₄ alkoxy(C₁-C₃)alkyl group and X is a hydrogen atom or a fluorine atom, which comprises reacting a compound of the formula II 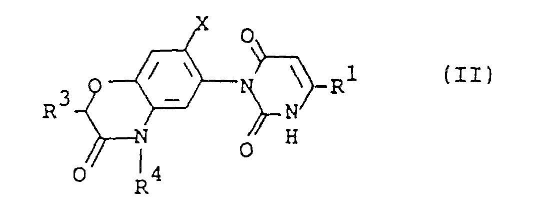 wherein R¹, R³, R⁴ and X are each as defined above with an aminating agent.

9. A process for preparing a compound of the formula I-2: 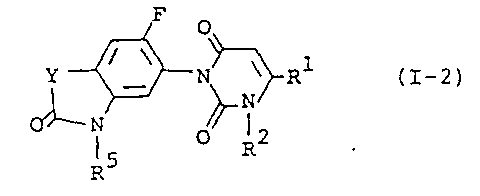 wherein R¹ is a trifluoromethyl group or a pentafluoroethyl group, R is an amino group, R⁵ is a C₁-C₇ alkyl group, a C₃-C₇ alkenyl group, a C₃-C₇ alkynyl group, a halo (C₁-C₆) alkyl group, a halo(C₃-C₆) alkenyl group or a C₁-C₄ alkoxy(C₁-C₃)alkyl group and Y is an oxygen atom or a sulfur atom, which comprises reacting a compound of the formula V: 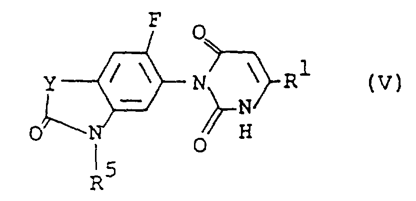 wherein R¹, R⁵ and Y are as defined above with an aminating agent.

10. A process for preparing a compound of the formula I-3: 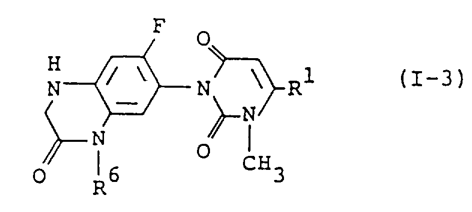 wherein R¹ is a trifluoromethyl group or a pentafluoroethyl group and R⁶ is a C₁-C₇ alkyl group, a C₃-C₇ alkenyl group, a C₃-C₇ alkynyl group, a halo(C₁-C₆)alkyl group, a halo(C₃-C₆)alkenyl group or a C₁-C₄ alkoxy(C₁-C₃)alkyl group, which comprises reacting a compound of the formula VI: 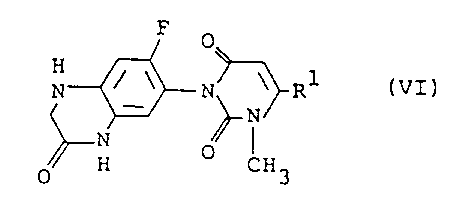 wherein R¹ is as defined above with a compound of the formula VII:
R⁶-Z (VII)
wherein Z is a leaving group and R⁶ is as defined above.

11. A compound of the formula VI: 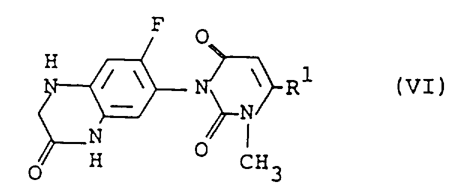 wherein R¹ is a trifluoromethyl group or a pentafluoroethyl group.

12. A compound of the formula VIII: 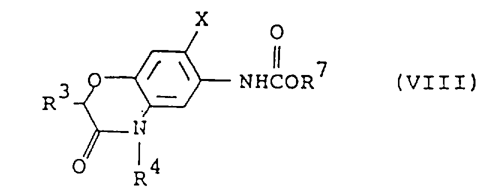 wherein R³ is a hydrogen atom or a methyl group, R⁴ is a C₁-C₇ alkyl group, a C₃-C₇ alkenyl group, a C₃-C₇ alkynyl group, a halo(C₁-C₆)alkyl group, a halo(C₃-C₆)alkenyl group or a C₁-C₄ alkoxy(C₁-C₃)alkyl group, R⁷ is a C₁-C₆ alkyl group and X is a hydrogen atom or a fluorine atom.

13. A compound of the formula X: 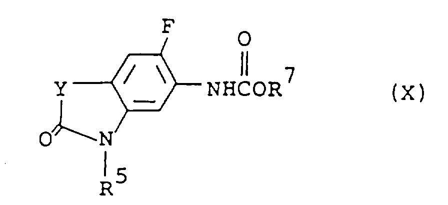 wherein R⁵ is a C₁-C₇ alkyl group, a C₃-C₇ alkenyl group, a C₃-C₇ alkynyl group, a halo(C₁-C₆)alkyl group, a halo(C₃-C₆)alkenyl group or a C₁-C₄ alkoxy(C₁-C₃)alkyl group, R⁷ is a C₁-C₆ alkyl group and Y is an oxygen atom or a sulfur atom.

14. A herbicidal composition which comprises as an active ingredient a herbicidally effective amount of the compound according to any of claims 1 to 7, and an inert carrier or diluent.

15. A method for exterminating harmful weeds, which comprises applying a herbicidally effective amount of the compound according to any of claims 1 to 7 and an inert carrier or diluent to the area where the undesired weeds grow or will grow.

16. Use of the compounds according to any of claims 1 to 7 as herbicides.

## Claims (Claims for the following Contracting State(s): ES)

1. A process for preparing a compound of the formula I 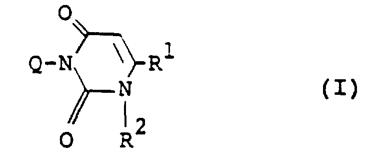 wherein R¹ is a trifluoromethyl group or a pentafluoroethyl group, R is a methyl group or an amino group and Q is either one of the formulas: 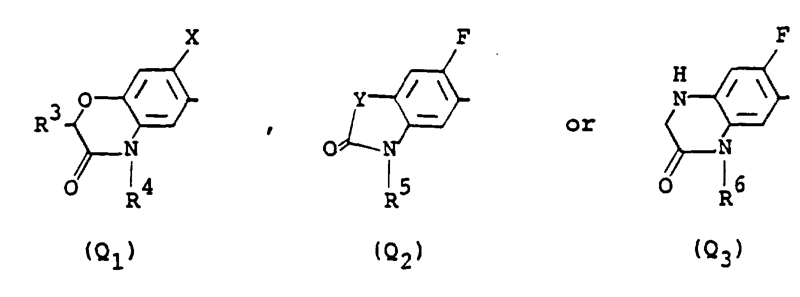 wherein R³ is a hydrogen atom or a methyl group, R⁴, R⁵ and R⁶ are each a C₁-C₇ alkyl group, a C₃-C₇ alkenyl group, a C₃-C₇ alkynyl group, a halo(C₁-C₆)alkyl group, a halo(C₃-C₆)alkenyl group or a C₁-C₄ alkoxy(C₁-C₃)alkyl group, X is a hydrogen atom or a fluorine atome and Y is an oxygen atom or a sulfur atom, with the proviso that when Q is Q₃, R represents a methyl group, and when Q is Q₁ or Q₂, R represents an amino group,
characterized in that
(a) for preparing a compound I, in which Q is Q₁ and all other substituents are as defined above, namely a compound which has the formula I-1 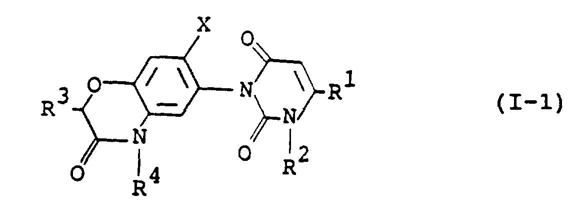 wherein R¹, R, R³, R⁴ and X are each as defined above,
a compound of the formula II 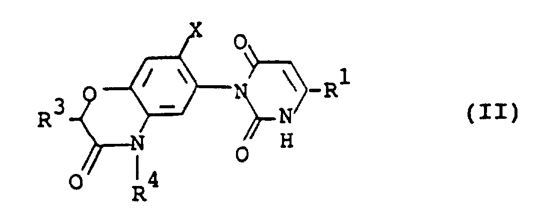 wherein R¹, R³, R⁴ and X are each as defined above,
is reacted with an aminating agent, or
(b) for preparing a compound I, wherein Q is Q₂, and all other substituents are as defined above, namely a compound which has the formula I-2 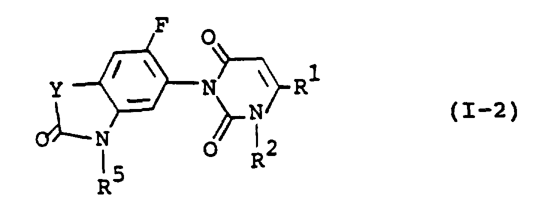 wherein R¹, R, R⁵ and Y are each as defined above,
a compound of the formula V 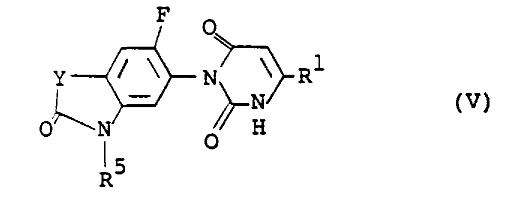 wherein R¹, R⁵ and Y are as defined above,
is reacted with an aminating agent, or
(c) for preparing a compound I, wherein Q is Q₃, and all other substituents are as defined above, namely a compound of the formula I-3 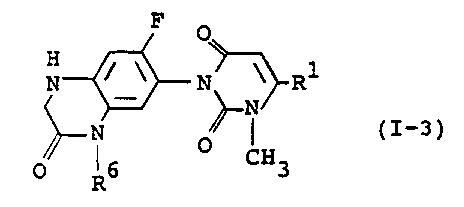 wherein R¹ and R⁶ are as defined above,
a compound of the formula VI 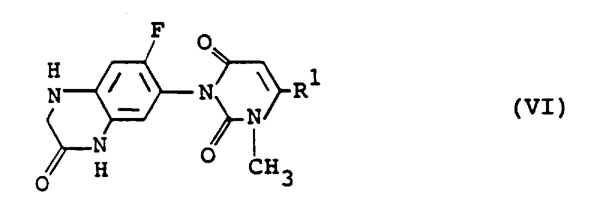 wherein R¹ is as defined above,
is reacted with a compound of the formula VII
R⁶-Z (VII)
wherein Z is a leaving group and R⁶ is as defined above.

2. A process for preparing a compound according to claim 1(a), wherein R³ is a hydrogen atom, R⁴ is a C₃-C₇ alkynyl group and X is a fluorine atom.

3. A process according to claim 1(b) for preparing a compound, wherein Y is a sulfur atom.

4. A process according to claim 3 for preparing a compound, wherein R⁵ is a C₁-C₇ alkyl group.

5. A herbicidal composition which comprises as an active ingredient a herbicidally effective amount of the compound obtainable according to any of claims 1 to 4, and an inert carrier or diluent.

6. A method for exterminating harmful weeds, which comprises applying a herbicidally effective amount of the compound obtainable according to any of claims 1 to 4 and an inert carrier or diluent to the area where the undesired weeds grow or will grow.

7. Use of the compounds obtainable according to any of claims 1 to 4 as herbicides.

8. A process for preparing a compound of the formula II 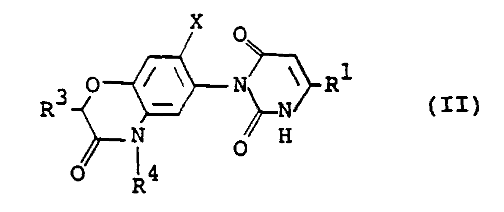 wherein R¹ is a trifluoromethyl group or a pentafluoroethyl group, R³ is a hydrogen atom or a methyl group, R⁴ is a C₁-C₇ alkyl group, a C₃-C₇ alkenyl group, a C₃-C₇ alkynyl group, a halo(C₁-C₆)alkyl group, a halo(C₃-C₆)alkenyl group or a C₁-C₄ alkoxy(C₁-C₃)alkyl group and X is a hydrogen atom or a fluorine atome,
characterized by reacting the compound VIII 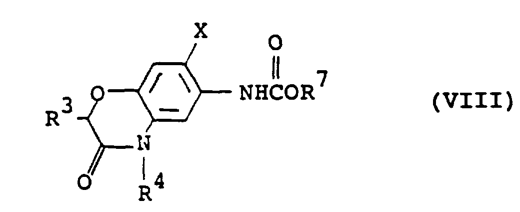 wherein R⁷ is a C₁-C₆ alkyl group, R³, R⁴, and X are each as defined above,
with a compound of the formula XII
R¹(NH₂)C=CHCOOR⁹ (XII)
wherein R⁹ is a C₁-C₆ alkyl group and R¹ is as defined above.

9. A process for preparing a compound of the formula V 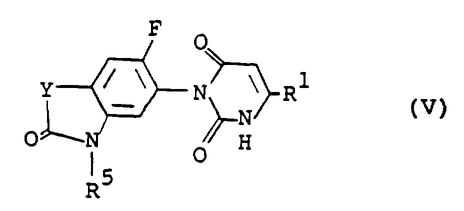 wherein R¹ is a trifluoromethyl group or a pentafluoroethyl group, R⁵ a C₁-C₇ alkyl group, a C₃-C₇ alkenyl group, a C₃-C₇ alkynyl group, a halo(C₁-C₆)alkyl group, a halo(C₃-C₆)alkenyl group or a C₁-C₄ alkoxy(C₁-C₃)alkyl group and Y is an oxygen atom or a sulfur atom,
characterized by reacting the compound X 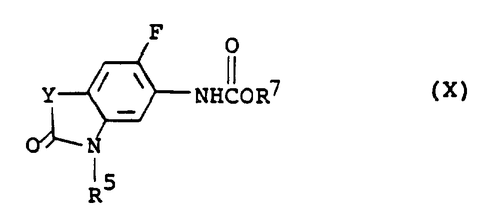 wherein R⁷ is a C₁-C₆ alkyl group and R⁵ and Y are as defined above,
with a compound of the formula XII
R¹(NH₂)C=CHCOOR⁹ (XII)
wherein R⁹ is a C₁-C₆ alkyl group and R¹ is as defined above.

10. A process for preparing a compound of the formula VI 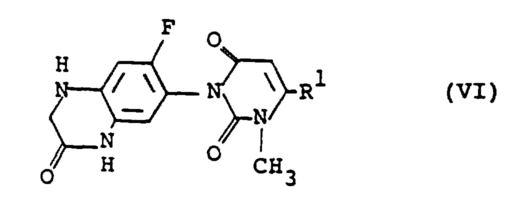 wherein R¹ is a trifluoromethyl group or a pentafluoroethyl group,
characterized by subjecting the compound XV 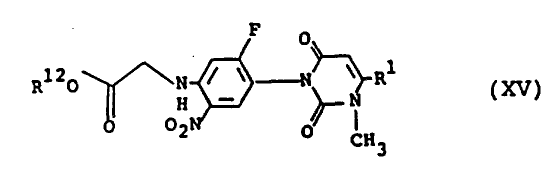 wherein R¹ is a C₁-C₆ alkyl group and R¹ is as defined above,
to reductive cyclization.

11. A process for preparing a compound of the formula VIII 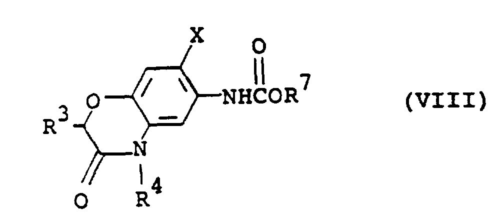 wherein R³ is a hydrogen atom or a methyl group, R⁴ is a C₁-C₇ alkyl group, a C₃-C₇ alkenyl group, a C₃-C₇ alkynyl group, a halo(C₁-C₆)alkyl group, a halo(C₃-C₆)alkenyl group or a C₁-C₄ alkoxy(C₁-C₃)alkyl group, R⁷ is a C₁-C₆ alkyl group and X is a hydrogen atom or a fluorine atom,
characterized by reacting the compound IX 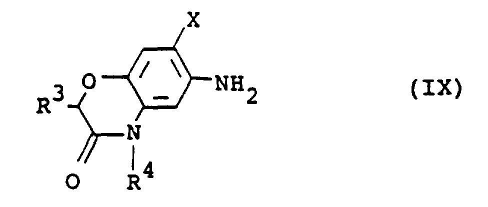 wherein R³, R⁴ and X are each as defined above,
with a compound of the formula XIII 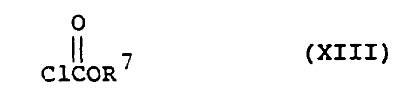 wherein R⁷ is a C₁-C₆ alkyl group.

12. A process for preparing a compound of the formula X 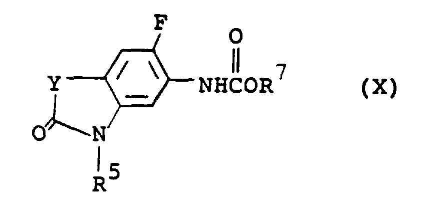 wherein R⁵ is a C_{1-C7} alkyl group, a C₃-C₇ alkenyl group, a C₃-C₇ alkynyl group, a halo(C₁-C₆)alkyl group, a halo(C₃-C₆)alkenyl group or a C₁-C₄ alkoxy(C₁-C₃)alkyl group, R⁷ is a C₁-C₆ alkyl group and Y is an oxygen atom or a sulfur atom,
characterized by reacting the compound XI 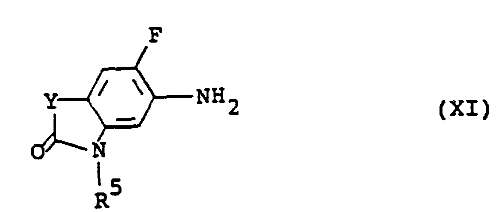 wherein R⁵ and Y are each as defined above,
with a compound of the formula XIII 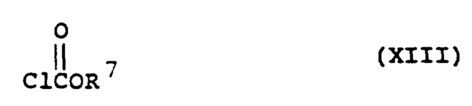 wherein R⁷ is a C₁-C₆ alkyl group.

## Patentansprüche (Patentansprüche für folgende(n) Vertragsstaat(en): BE, CH, DE, FR, GB, IT, LI, NL)

1. Verbindung der Formel I 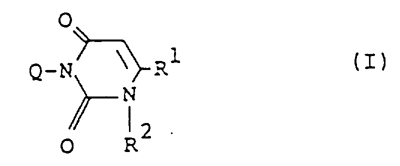 wobei R¹ eine Trifluormethylgruppe oder eine Pentafluorethylgruppe bedeutet, R eine Methylgruppe oder eine Aminogruppe darstellt und Q eine der Formeln Q₁, Q₂ oder Q₃ 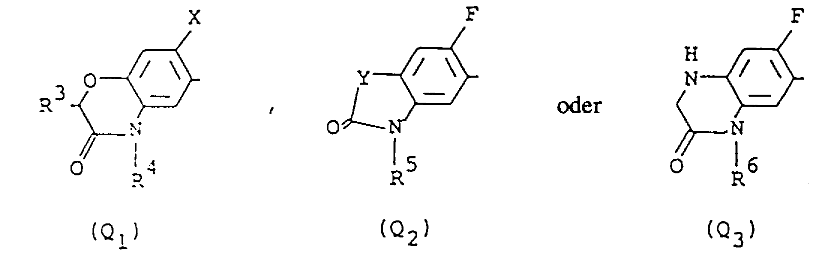 bedeutet, wobei R³ ein Wasserstoffatom oder eine Methylgruppe darstellt, R⁴, R⁵ und R⁶ jeweils einen C₁-C₇-Alkylrest, einen C₃-C₇-Alkenylrest, einen C₃-C₇-Alkinylrest, einen Halogen(C₁-C₆)alkylrest, einen Halogen(C₃-C₆)alkenylrest oder einen C₁-C₄-Alkoxy(C₁-C₃)alkylrest bedeuten, X ein Wasserstoffatom oder ein Fluoratom darstellt, und Y ein Sauerstoffatom oder ein Schwefelatom bedeutet, mit der Maßgabe, daß R eine Methylgruppe wiedergibt, wenn Q Q₃ ist, und R eine Aminogruppe wiedergibt, wenn Q Q₁ oder Q₂ ist.

2. Verbindung nach Anspruch 1 der Formel: 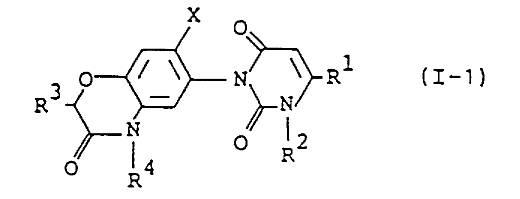 wobei R¹, R, R³, R⁴ und X jeweils wie in Anspruch 1 definiert sind.

3. Verbindung nach Anspruch 1 der Formel: 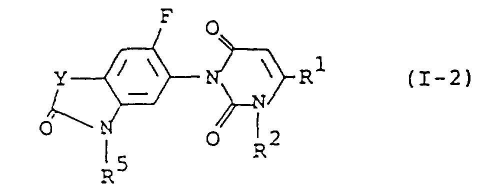 wobei R¹, R, R⁵ und Y jeweils wie in Anspruch 1 definiert sind.

4. Verbindung nach Anspruch 1 der Formel: 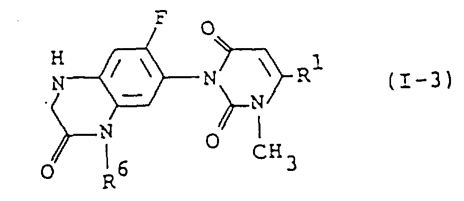 wobei R¹ und R⁶ jeweils wie in Anspruch 1 definiert sind.

5. Verbindung nach Anspruch 2, wobei R³ ein Wasserstoffatom darstellt, R⁴ einen C₃-C₇-Alkinylrest bedeutet und X ein Fluoratom darstellt.

6. Verbindung nach Anspruch 3, wobei Y ein Schwefelatom bedeutet.

7. Verbindung nach Anspruch 6, wobei R⁵ einen C₁-C₇-Alkylrest darstellt.

8. Verfahren zur Herstellung einer Verbindung der Formel I-1: 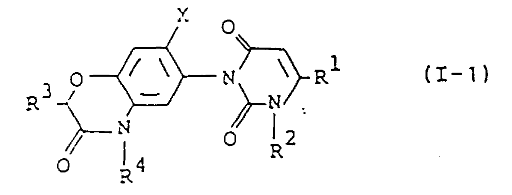 wobei R¹ eine Trifluormethylgruppe oder eine Pentafluorethylgruppe bedeutet, R eine Aminogruppe darstellt, R³ ein Wasserstoffatom oder eine Methylgruppe bedeutet, R⁴ einen C₁-C₇-Alkylrest, einen C₃-C₇-Alkenylrest, einen C₃-C₇-Alkinylrest, einen Halogen(C₁-C₆)alkylrest, einen Halogen(C₃-C₆)alkenylrest oder einen C₁-C₄-Alkoxy(C₁-C₃)alkylrest darstellt und X ein Wasserstoffatom oder ein Fluoratom bedeutet, das das Umsetzen einer Verbindung der Formel II 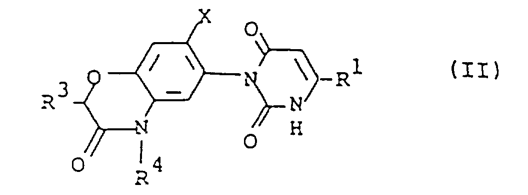 wobei R¹, R³, R⁴ und X jeweils wie vorstehend definiert sind, mit einem Aminierungsmittel umfaßt.

9. Verfahren zur Herstellung einer Verbindung der Formel I-2: 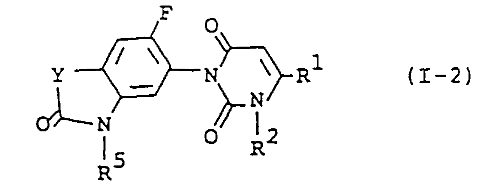 wobei R¹ eine Trifluormethylgruppe oder eine Pentafluorethylgruppe bedeutet, R eine Aminogruppe darstellt, R⁵ einen C₁-C₇-Alkylrest, einen C₃-C₇-Alkenylrest, einen C₃-C₇-Alkinylrest, einen Halogen(C₁-C₆)alkylrest, einen Halogen(C₃-C₆)alkenylrest oder einen C₁-C₄-Alkoxy(C₁-C₃)alkylrest bedeutet und Y ein Sauerstoffatom oder ein Schwefelatom darstellt, das das Umsetzen einer Verbindung der Formel V: 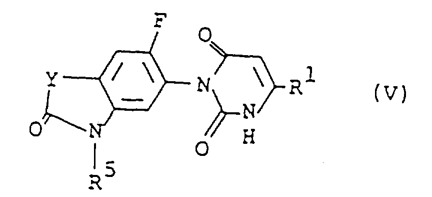 wobei R¹, R⁵ und Y jeweils wie vorstehend definiert sind, mit einem Aminierungsmittel umfaßt.

10. Verfahren zur Herstellung einer Verbindung der Formel I-3: 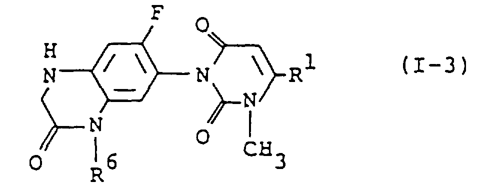 wobei R¹ eine Trifluormethylgruppe oder eine Pentafluorethylgruppe bedeutet, und R⁶ einen C₁-C₇-Alkylrest, einen C₃-C₇-Alkenylrest, einen C₃-C₇-Alkinylrest, einen Halogen(C₁-C₆)alkylrest, einen Halogen(C₃-C₆)alkenylrest oder einen C₁-C₄-Alkoxy(C₁-C₃)alkylrest darstellt, das das Umsetzen einer Verbindung der Formel VI: 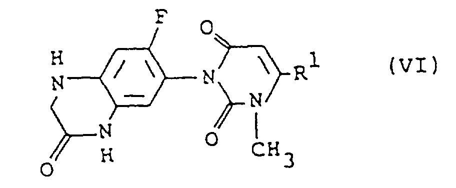 wobei R¹ wie vorstehend definiert ist, mit einer Verbindung der Formel VII:
R⁶-Z (VII)
wobei Z eine Abgangsgruppe bedeutet und R⁶ wie vorstehend definiert ist, umfaßt.

11. Verbindung der Formel VI: 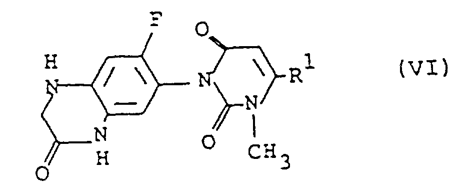 wobei R¹ eine Trifluormethylgruppe oder eine Pentafluorethylgruppe bedeutet.

12. Verbindung der Formel VIII: 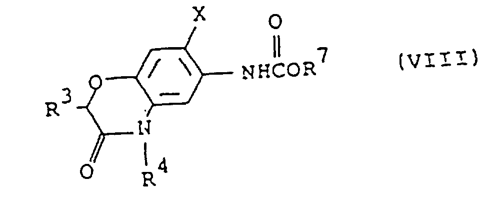 wobei R³ ein Wasserstoffatom oder eine Methylgruppe darstellt, R⁴ einen C₁-C₇-Alkylrest, einen C₃-C₇-Alkenylrest, einen C₃-C₇-Alkinylrest, einen Halogen(C₁-C₆)alkylrest, einen Halogen(C₃-C₆)alkenylrest oder einen C₁-C₄-Alkoxy(C₁-C₃)alkylrest bedeutet, R⁷ einen C₁-C₆-Alkylrest darstellt und X ein Wasserstoffatom oder ein Fluoratom bedeutet.

13. Verbindung der Formel X: 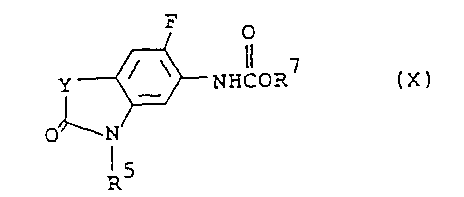 wobei R⁵ einen C₁-C₇-Alkylrest, einen C₃-C₇-Alkenylrest, einen C₃-C₇-Alkinylrest, einen Halogen(C₁-C₆)alkylrest, einen Halogen(C₃-C₆)alkenylrest oder einen C₁-C₄-Alkoxy(C₁-C₃)alkylrest darstellt, R⁷ einen C₁-C₆-Alkylrest bedeutet und Y ein Sauerstoffatom oder ein Schwefelatom darstellt.

14. Herbizides Mittel, das als Wirkstoff eine herbizid wirksame Menge der Verbindung nach einem der Ansprüche 1 bis 7 und ein(en) inerten/s Träger oder Verdünnungsmittel umfaßt.

15. Verfahren zur Vernichtung schädlicher Unkräuter, das das Aufbringen einer herbizid wirksamen Menge der Verbindung nach einem der Ansprüche 1 bis 7 und eines inerten Trägers oder Verdünnungsmittels auf die Fläche, wo unerwünschte Unkräuter wachsen oder wachsen werden, umfaßt.

16. Verwendung der Verbindung nach einem der Ansprüche 1 bis 7 als Herbizid.

## Patentansprüche (Patentansprüche für folgende(n) Vertragsstaat(en): ES)

1. Verfahren zur Herstellung einer Verbindung der Formel I 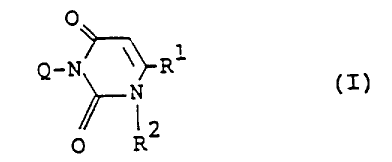 wobei R¹ eine Trifluormethylgruppe oder eine Pentafluorethylgruppe bedeutet, R eine Methylgruppe oder eine Aminogruppe darstellt und Q eine der Formeln bedeutet: 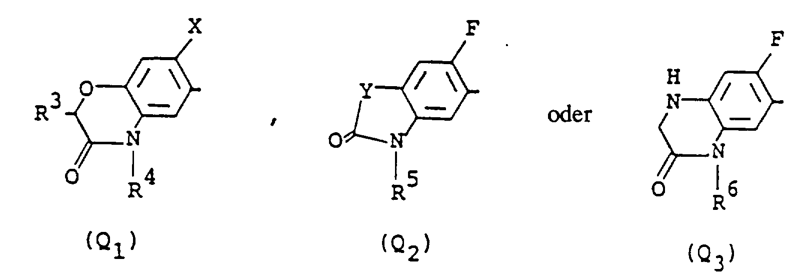 wobei R³ ein Wasserstoffatom oder eine Methylgruppe darstellt, R⁴, R⁵ und R⁶ jeweils einen C₁-C₇-Alkylrest, einen C₃-C₇-Alkenylrest, einen C₃-C₇-Alkinylrest, einen Halogen(C₁-C₆)-Alkylrest, einen Halogen(C₃-C₆)-Alkenylrest oder einen C₁-C₄-Alkoxy(C₁-C₃)alkylrest bedeuten, X ein Wasserstoffatom oder ein Fluoratom darstellt und Y ein Sauerstoffatom oder ein Schwefelatom bedeutet, mit der Maßgabe, daß R eine Methylgruppe wiedergibt, wenn Q Q₃ ist, und R eine Aminogruppe wiedergibt, wenn Q Q₁ oder Q₂ ist, dadurch gekennzeichnet, daß
(a) zur Herstellung einer Verbindung I, in der Q Q₁ ist und alle anderen Substituenten wie vorstehend definiert sind, nämlich einer Verbindung der Formel I-1 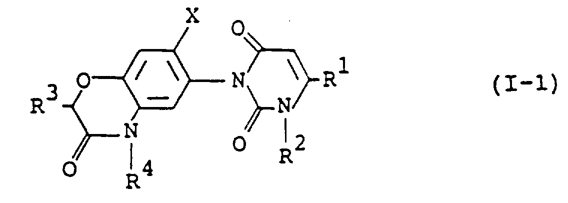 wobei R¹, R, R³, R⁴ und X jeweils wie vorstehend definiert sind,
eine Verbindung der Formel II 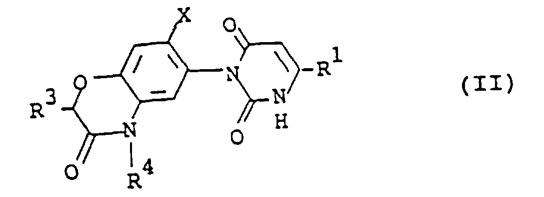 wobei R¹, R³, R⁴ und X jeweils wie vorstehend definiert sind,
mit einem Aminierungsmittel umgesetzt wird oder
(b) zur Herstellung einer Verbindung I, wobei Q Q₂ ist und alle anderen Substituenten wie vorstehend definiert sind, nämlich einer Verbindung der Formel I-2 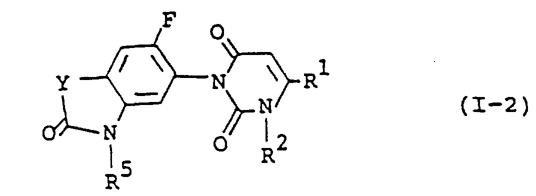 wobei R¹, R, R⁵ und Y jeweils wie vorstehend definiert sind,
eine Verbindung der Formel V 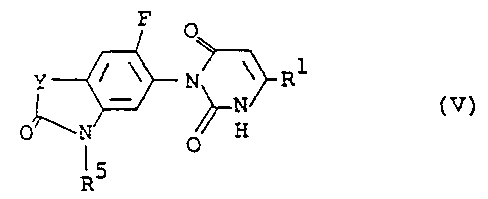 wobei R¹, R⁵ und Y wie vorstehend definiert sind,
mit einem Aminierungsmittel umgesetzt wird oder
(c) zur Herstellung einer Verbindung I, wobei Q Q₃ ist und alle anderen Substituenten wie vorstehend definiert sind, nämlich einer Verbindung der Formel I-3 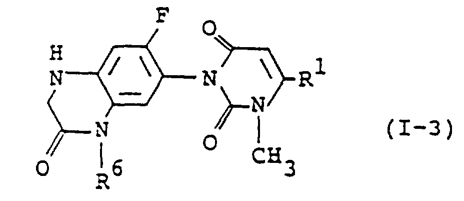 wobei R¹ und R⁶ wie vorstehend definiert sind,
eine Verbindung der Formel VI 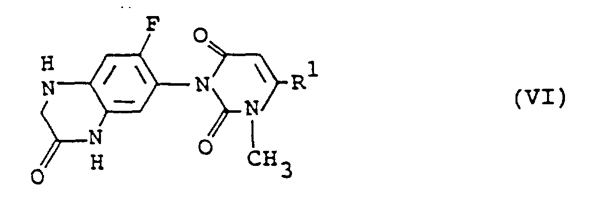 wobei R¹ wie vorstehend definiert ist,
mit einer Verbindung der Formel VII,
R⁶-Z (VII)
umgesetzt wird, wobei Z eine Abgangsgruppe darstellt und R⁶ wie vorstehend definiert ist.

2. Verfahren zur Herstellung einer Verbindung nach Anspruch 1(a), wobei R³ ein Wasserstoffatom darstellt, R⁴ einen C₃-C₇-Alkinylrest bedeutet und X ein Fluoratom darstellt.

3. Verfahren nach Anspruch 1(b) zur Herstellung einer Verbindung, wobei Y ein Schwefelatom darstellt.

4. Verfahren nach Anspruch 3 zur Herstellung einer Verbindung, wobei R⁵ einen C₁-C₇-Alkylrest darstellt.

5. Herbizides Mittel, das als Wirkstoff eine herbizid wirksame Menge der nach einem der Ansprüche 1 bis 4 erhältlichen Verbindung und ein(en) inerten/s Träger oder Verdünnungsmittel umfaßt.

6. Verfahren zur Vernichtung schädlicher Unkräuter, das das Aufbringen einer herbizid wirksamen Menge der nach einem der Ansprüche 1 bis 4 erhältlichen Verbindung und eines inerten Trägers oder Verdünnungsmittels auf die Fläche, wo unerwünschte Unkräuter wachsen oder wachsen werden, umfaßt.

7. Verwendung der nach einem der Ansprüche 1 bis 4 erhältlichen Verbindungen als Herbizide.

8. Verfahren zur Herstellung einer Verbindung der Formel II 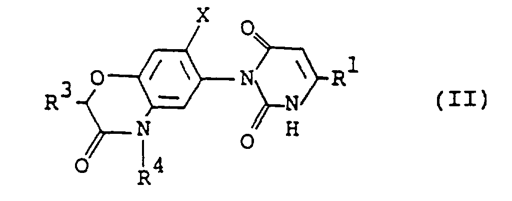 wobei R¹ eine Trifluormethylgruppe oder eine Pentafluorethylgruppe bedeutet, R³ ein Wasserstoffatom oder eine Methylgruppe darstellt, R⁴ einen C₁-C₇-Alkylrest, einen C₃-C₇-Alkenylrest, einen C₃-C₇-Alkinylrest, einen Halogen(C₁-C₆)alkylrest, einen Halogen(C₃-C₆)alkenylrest oder einen C₁-C₄-Alkoxy(C₁-C₃)alkylrest bedeutet und X ein Wasserstoffatom oder ein Fluoratom darstellt,
gekennzeichnet durch Umsetzen einer Verbindung VIII 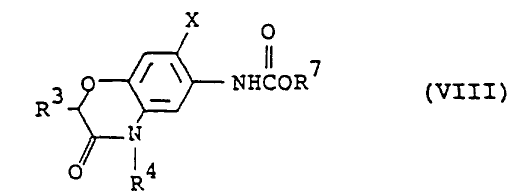 wobei R⁷ einen C₁-C₆-Alkylrest darstellt, R³, R⁴ und X jeweils wie vorstehend definiert sind,
mit einer Verbindung der Formel XII
R¹(NH₂)C=CHCOOR⁹ (XII)
wobei R⁹ einen C₁-C₆-Alkylrest darstellt und R¹ wie vorstehend definiert ist.

9. Verfahren zur Herstellung einer Verbindung der Formel V 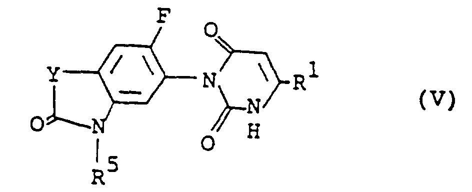 wobei R¹ eine Trifluormethylgruppe oder eine Pentafluorethylgruppe bedeutet, R⁵ einen C₁-C₇-Alkylrest, einen C₃-C₇-Alkenylrest, einen C₃-C₇-Alkinylrest, einen Halogen(C₁-C₆)alkylrest, einen Halogen(C₃-C₆)alkenylrest oder einen C₁-C4-Alkoxy(C₁-C₃)alkylrest darstellt und Y ein Sauerstoffatom oder ein Schwefelatom bedeutet,
gekennzeichnet durch Umsetzen einer Verbindung X 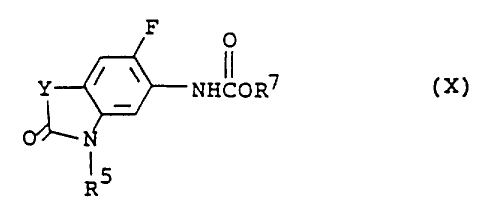 wobei R⁷ einen C₁-C₆-Alkylrest darstellt und R⁵ und Y wie vorstehend definiert sind, mit einer Verbindung der Formel XII
R¹(NH₂)C=CHCOOR⁹ (XII)
wobei R⁹ einen C₁-C₆-Alkylrest darstellt und R¹ wie vorstehend definiert ist.

10. Verfahren zur Herstellung einer Verbindung der Formel VI 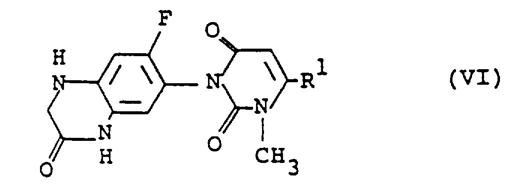 wobei R¹ eine Trifluormethylgruppe oder eine Pentafluorethylgruppe bedeutet,
dadurch gekennzeichnet, daß eine Verbindung XV 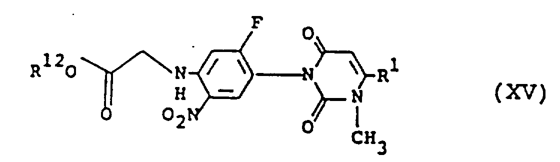 wobei R¹ einen C₁-C₆-Alkylrest darstellt und R¹ wie vorstehend definiert ist,
einer reduktiven Cyclisierung unterworfen wird.

11. Verfahren zur Herstellung einer Verbindung der Formel VIII 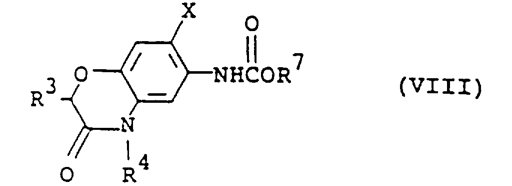 wobei R³ ein Wasserstoffatom oder eine Methylgruppe darstellt, R⁴ einen C₁-C₇-Alkylrest, einen C₃-C₇-Alkenylrest, einen C₃-C₇-Alkinylrest, einen Halogen(C₁-C₆)alkylrest, einen Halogen(C₃-C₆)alkenylrest oder einen C₁-C₄-Alkoxy(C₁-C₃)alkylrest bedeutet, R⁷ einen C₁-C₆-Alkylrest darstellt und X ein Wasserstoffatom oder ein Fluoratom bedeutet, gekennzeichnet durch Umsetzen einer Verbindung IX 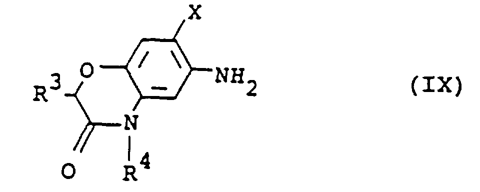 wobei R³, R⁴ und X jeweils wie vorstehend definiert sind,
mit einer Verbindung der Formel XIII 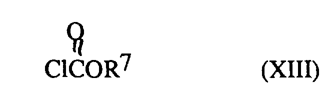 wobei R⁷ einen C₁-C₆-Alkylrest darstellt.

12. Verfahren zur Herstellung einer Verbindung der Formel X 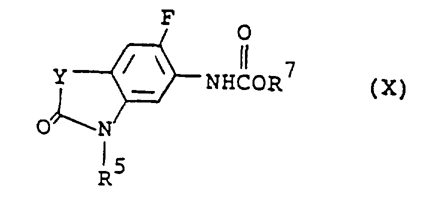 wobei R⁵ einen C₁-C₇-Alkylrest, einen C₃-C₇-Alkenylrest, einen C₃-C₇-Alkinylrest, einen Halogen(C₁-C₆)alkylrest, einen Halogen(C₃-C₆)alkenylrest oder einen C₁-C₄-Alkoxy(C₁-C₃)alkylrest darstellt, R⁷ einen C₁-C₆-Alkylrest bedeutet und Y ein Sauerstoffatom oder ein Schwefelatom darstellt,
gekennzeichnet durch Umsetzen einer Verbindung XI 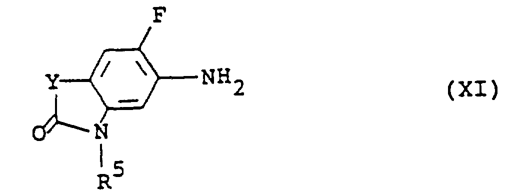 wobei R⁵ und Y jeweils wie vorstehend definiert sind,
mit einer Verbindung der Formel XIII 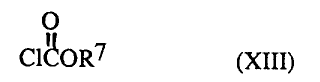 wobei R⁷ einen C₁-C₆-Alkylrest darstellt.

## Revendications (Revendications pour l'(les) Etat(s) contractant(s) suivant(s): BE, CH, DE, FR, GB, IT, LI, NL)

1. Composé de la formule I 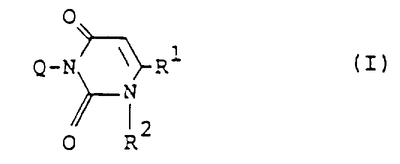 dans laquelle R¹ représente un radical trifluorométhyle ou un radical pentafluoréthyle, R représente un radical méthyle ou un radical amino et Q représente l'une des formules Q₁, Q₂ ou Q₃ 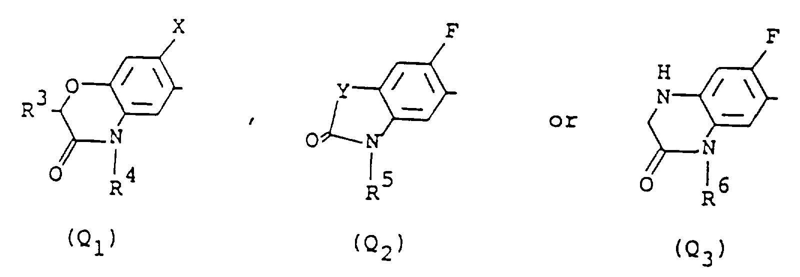 dans lesquelles R³ représente un atome d'hydrogène ou un radical méthyle, R⁴, R⁵ et R⁶ représentent chacun un radical alkyle en C₁ à C₇, un radical alcényle en C₃ à C₇, un radical alcynyle en C₃ à C₇, un radical haloalkyle en C₁ à C₆, un radical haloalcényle en C₃ à C₆, ou un radical alcoxy(C₁-C₄) alkyle(C₁-C₃), X représente un atome d'hydrogène ou un atome de fluor et Y représente un atome d'oxygène ou un atome de soufre, avec la condition que lorsque Q est Q₃, R représente un radical méthyle et que lorsque Q est Q₁ ou Q₂, R représente un radical amino.

2. Composé suivant la revendication 1, caractérisé en ce qu'il répond à la formule : 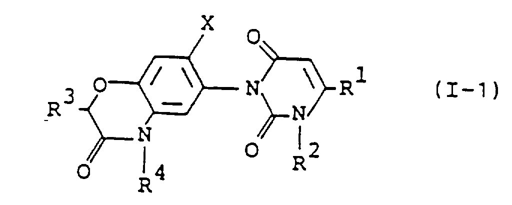 dans laquelle R¹, R, R³, R⁴ et X possèdent chacun les significations qui leur ont été attribuées dans la revendication 1.

3. Composé suivant la revendication 1, caractérisé en ce qu'il répond à la formule : 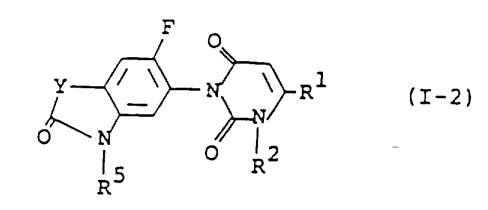 dans laquelle R¹, R, R⁵ et Y possèdent chacun les significations qui leur ont été attribuées dans la revendication 1.

4. Composé suivant la revendication 1, caractérisé en ce qu'il répond à la formule : 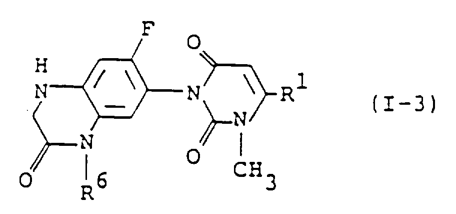 dans laquelle R¹ et R⁶ possèdent chacun les significations qui leur ont été attribuées dans la revendication 1.

5. Composé suivant la revendication 2, caractérisé en ce que R³ représente un atome d'hydrogène, R⁴ représente un radical alcynyle en C₃ à C₇ et X représente un atome de fluor.

6. Composé suivant la revendication 3, caractérisé en ce que Y représente un atome de soufre.

7. Composé suivant la revendication 6, caractérisé en ce que R⁵ représente un radical alkyle en C₁ à C₇.

8. Procédé de préparation d'un composé de la formule I-1 : 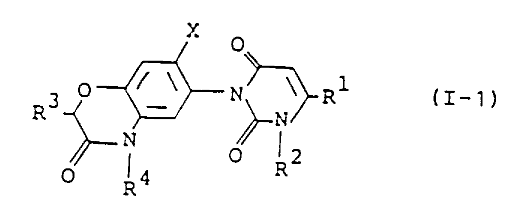 dans laquelle R¹ représente un radical trifluorométhyle ou un radical pentafluoréthyle, R représente un radical amino, R³ représente un atome d'hydrogène ou un radical méthyle, R⁴ représente un radical alkyle en C₁ à C₇, un radical alcényle en C₃ à C₇, un radical alcynyle en C₃ à C₇, un radical haloalkyle en C₁ à C₆, un radical haloalcényle en C₃ à C₆ ou un radical alcoxy(C₁-C₄)alkyle(C₁-C₃) et X représente un atome d'hydrogène ou un atome de fluor, caractérisé en ce que l'on fait réagir un composé de la formule II 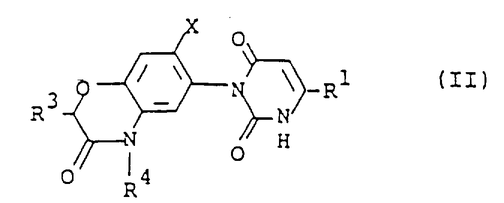 dans laquelle R¹, R³, R⁴ et X possèdent chacun les significations qui leur ont été précédemment attribuées, avec un agent d'amination.

9. Procédé de préparation d'un composé de la formule I-2 : 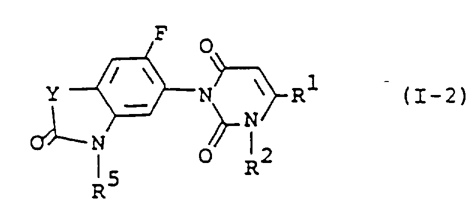 dans laquelle R¹ représente un radical trifluorométhyle ou un radical pentafluoréthyle, R représente un radical amino, R⁵ représente un radical alkyle en C₁ à C₇, un radical alcényle en C₃ à C₇, un radical alcynyle en C₃ à C₇, un radical haloalkyle en C₁ à C₆, un radical haloalcényle en C₃ à C₆ ou un radical alcoxy(C₁-C₄)alkyle(C₁-C₃) et Y représente un atome d'oxygène ou un atome de soufre, caractérisé en ce que l'on fait réagir un composé de la formule V 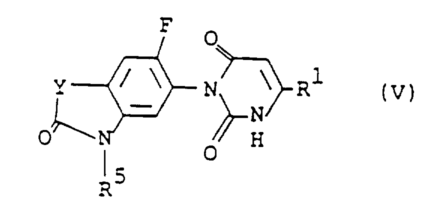 dans laquelle R¹, R⁵ et Y possèdent les significations qui leur ont été précédemment attribuées, avec un agent d'amination.

10. Procédé de préparation d'un composé de la formule I-3 : 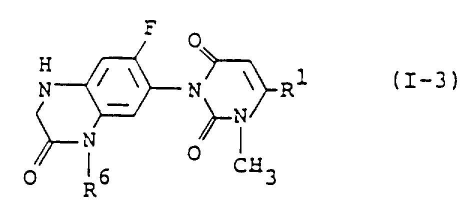 dans laquelle R¹ représente un radical trifluorométhyle ou un radical pentafluoréthyle et R⁶ représente un radical alkyle en C₁ à C₇, un radical alcényle en C₃ à C₇, un radical alcynyle en C₃ à C₇, un radical haloalkyle en C₁ à C₆, un radical haloalcényle en C₃ à C₆ ou un radical alcoxy(C₁-C₄)alkyle(C₁-C₃), caractérisé en ce que l'on fait réagir un composé de la formule VI 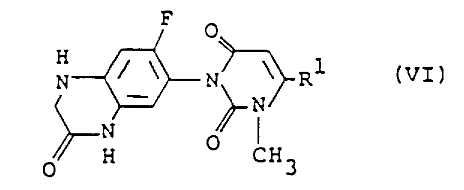 dans laquelle R¹ possède les significations qui lui ont précédemment attribuées, avec un composé de la formule VII :
R⁶-Z (VII)
dans laquelle Z représente un groupe sortant et R⁶ possède les significations qui lui ont été précédemment attribuées.

11. Composé de la formule VI : 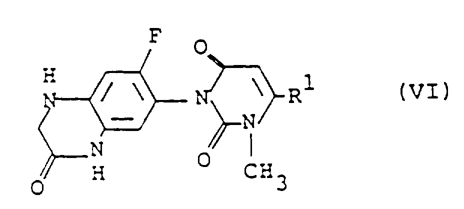 dans laquelle R¹ représente un radical trifluorométhyle ou un radical pentafluoréthyle.

12. Composé de la formule VIII : 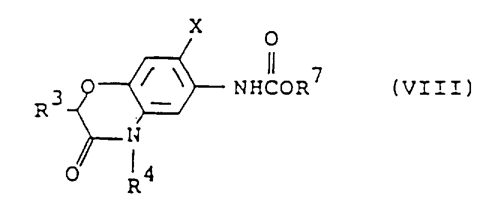 dans laquelle R³ représente un atome d'hydrogène ou un radical méthyle, R⁴ représente un radical alkyle en C₁ à C₇, un radical alcényle en C₃ à C₇, un radical alcynyle en C₃ à C₇, un radical haloalkyle en C₁ à C₆, un radical haloalcényle en C₃ à C₆ ou un radical alcoxy(C₁-C₄)alkyle(C₁-C₃), R⁷ représente un radical alkyle en C₁ à C₆ et X représente un atome d'hydrogène ou un atome de fluor.

13. Composé de la formule X : 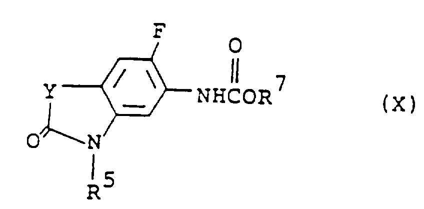 dans laquelle R⁵ représente un radical alkyle en C₁ à C₇, un radical alcényle en C₃ à C₇, un radical alcynyle en C₃ à C₇, un radical haloalkyle en C₁ à C₆, un radical haloalcényle en C₃ à C₆ ou un radical alcoxy(C₁-C₄)alkyle(C₁-C₃), R⁷ représente un radical alkyle en C₁ à C₆ et Y représente un atome d'oxygène ou un atome de soufre.

14. Composition herbicide qui comprend, à titre d'ingrédient actif, une quantité efficace du point de vue herbicide d'un composé suivant l'une quelconque des revendications 1 à 7 ainsi qu'un véhicule ou diluant inerte.

15. Procédé d'extermination de mauvaises herbes, caractérisé en ce que l'on applique une quantité efficace du point de vue herbicide d'un composé suivant l'une quelconque des revendications 1 à 7 et un véhicule ou diluant inerte à la zone où les herbes indésirables poussent ou vont pousser.

16. Utilisation des composés suivant l'une quelconque des revendications 1 à 7 à titre d'herbicides.

## Revendications (Revendications pour l'(les) Etat(s) contractant(s) suivant(s): ES)

1. Procédé de préparation d'un composé répondant de la formule I 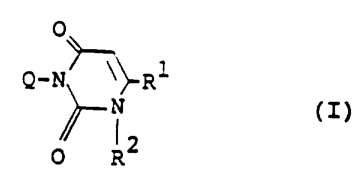 dans laquelle R¹ représente un radical trifluorométhyle ou un radical pentafluoréthyle, R représente un radical méthyle ou radical amino et Q représente l'une des formules 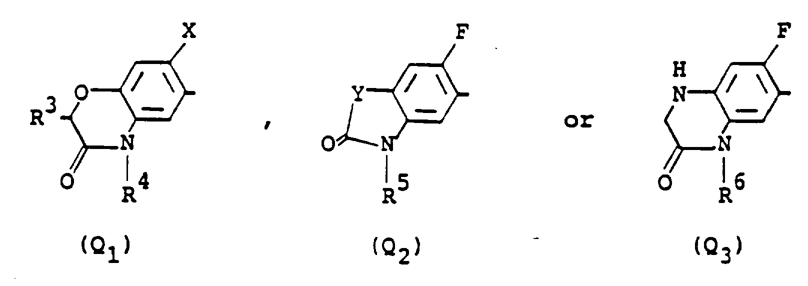 dans lesquelles R³ représente un atome d'hydrogène ou un radical méthyle, R⁴, R⁵ et R⁶ représentent chacun un radical alkyle en C₁ à C₇, un radical alcényle en C₃ à C₇, un radical alcynyle en C₃ à C₇, un radical haloalkyle en C₁ à C₆, un radical haloalcényle en C₃ à C₆, ou un radical alcoxy(C₁-C₄)alkyle(C₁-C₃), X représente un atome d'hydrogène ou un atome de fluor et Y représente un atome d'oxygène ou un atome de soufre, avec la condition que lorsque Q est Q₃, R représente un radical méthyle et que lorsque Q est Q₁ ou Q₂, R représente un radical amino,
caractérisé en ce que
(a) pour préparer un composé I, dans lequel Q est Q₁ et tous les autres substituants possèdent les significations qui leur ont été précédemment attribuées, à savoir un composé qui répond à la formule I-1 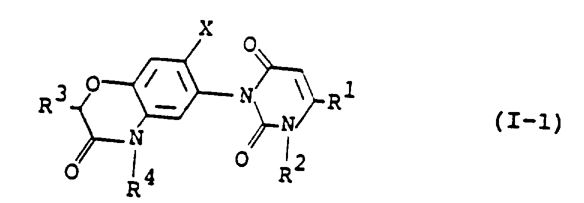 dans laquelle R¹, R, R³, R⁴ et X possèdent chacun les significations qui leur ont été précédemment attribuées, on fait réagir un composé qui répond à la formule II 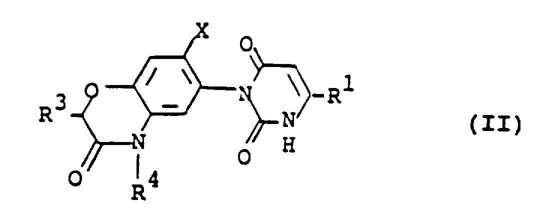 dans laquelle R¹, R³, R⁴ et X possèdent chacun les significations qui leur ont été précédemment attribuées, avec un agent d'amination, ou bien
(b) pour préparer un composé I dans lequel Q est Q₂ et tous les autres substituants possèdent les significations qui leur ont été précédemment attribuées, à savoir un composé qui répond à la formule I-2 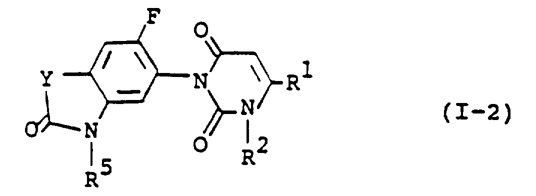 dans laquelle R¹, R, R⁵ et Y possèdent chacun les significations qui leur ont été précédemment attribuées, on fait réagir un composé de la formule V 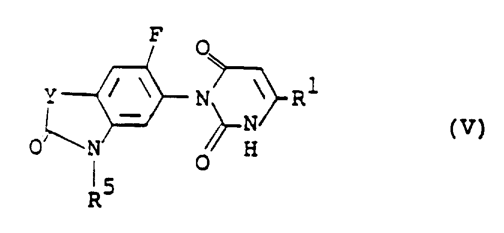 dans laquelle R¹, R⁵ et Y possèdent les significations qui leur ont été précédemment attribuées, avec un agent d'amination, ou bien
(c) pour préparer un composé I dans lequel Q est Q₃ et tous les autres substituants possèdent les significations qui leur ont été précédemment attribuées, à savoir un composé de la formule I-3 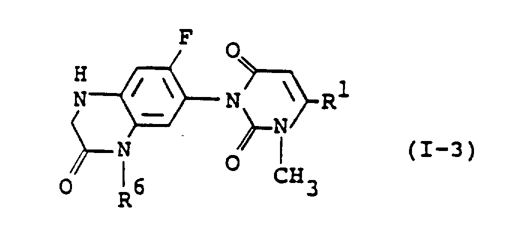 dans laquelle R¹ et R⁶ possèdent les significations qui leur ont été précédemment attribuées,
on fait réagir un composé de la formule VI 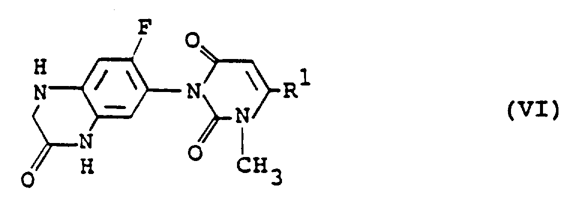 dans laquelle R¹ possède les significations qui lui ont été précédemment attribuées,
avec un composé de la formule VII
R⁶-Z (VII)
dans laquelle Z représente un groupe sortant et R⁶ possède les significations qui lui ont été précédemment attribuées.

2. Procédé de préparation d'un composé suivant la revendication 1(a), dans lequel R³ représente un atome d'hydrogène, R⁴ représente un radical alcynyle en C₃ à C₇ et X représente un atome de fluor.

3. Procédé suivant la revendication I(b) pour la préparation d'un composé dans lequel Y représente un atome de soufre.

4. Procédé suivant la revendication 3 pour la préparation d'un composé dans lequel R⁵ représente un groupe alkyle en C₁ à C₇.

5. Composition herbicide qui comprend, à titre d'ingrédient actif, une quantité efficace du point de vue herbicide du composé pouvant être obtenu suivant l'une quelconque des revendications 1 à 4 et un véhicule ou diluant inerte.

6. Procédé d'extermination de mauvaises herbes, caractérisé en ce que l'on applique une quantité efficace du point de vue herbicide du composé pouvant être obtenu suivant l'une quelconque des revendications 1 à 4 et un véhicule ou diluant inerte à la zone où les herbes indésirables poussent ou vont pousser.

7. Utilisation des composés pouvant être obtenus suivant l'une quelconque des revendications 1 à 4 à titre d'herbicides.

8. Procédé de préparation d'un composé de la formule II 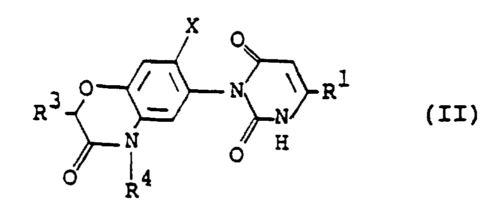 dans laquelle R¹ représente un radical trifluorométhyle ou un radical pentafluoréthyle, R³ représente un atome d'hydrogène ou un radical méthyle, R⁴ représente un radical alkyle en C₁ à C₇, un radical alcényle en C₃ à C₇, un radical alcynyle en C₃ à C₇, un radical haloalkyle en C₁ à C₆, un radical haloalcényle en C₃ à C₆ ou un radical alcoxy(C₁-C₄)alkyle(C₁-C₃) et X représente un atome d'hydrogène ou un atome de fluor,
caractérisé en ce que l'on fait réagir le composé de la formule VIII 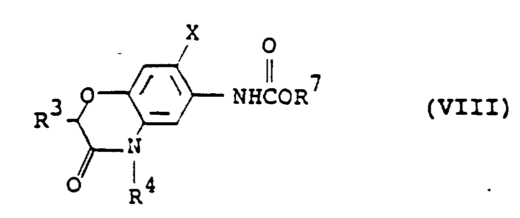 dans laquelle R⁷ représente un radical alkyle en C₁ à C₆, R³, R⁴ et X possèdent chacun les significations qui leur ont été précédemment attribuées,
avec un composé de la formule XII
R¹(NH₂)C=CHCOOR⁹ (XII)
dans laquelle R⁹ représente un radical alkyle en C₁ à C₆ et R¹ possède les significations qui lui ont été précédemment attribuées.

9. Procédé de préparation d'un composé de la formule V 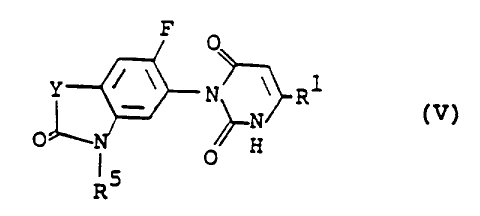 dans laquelle R¹ représente un radical trifluorométhyle ou un radical pentafluoréthyle, R⁵ représente un radical alkyle en C₁ à C₇, un radical alcényle en C₃ à C₇, un radical alcynyle en C₃ à C₇, un radical haloalkyle en C₁ à C₆, un radical haloalcényle en C₃ à C₆ ou un radical alcoxy(C₁-C₄)alkyle(C₁-C₃) et Y représente un atome d'oxygène ou un atome de soufre,
caractérisé en ce que l'on fait réagir le composé de la formule X 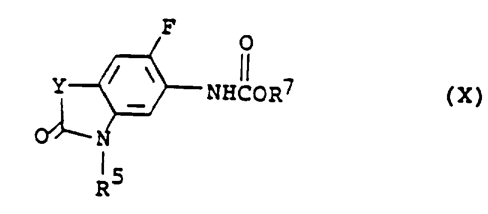 dans laquelle R⁷ représente un radical alkyle en C₁ à C₆ et R⁵ et Y possèdent chacun les significations qui leur ont été précédemment attribuées,
avec un composé de la formule XII
R¹(NH₂)C=CHCOOR⁹ (XII)
dans laquelle R⁹ représente un radical alkyle en C₁ à C₆ et R¹ possède les significations qui lui ont été précédemment attribuées.

10. Procédé de préparation d'un composé de la formule VI 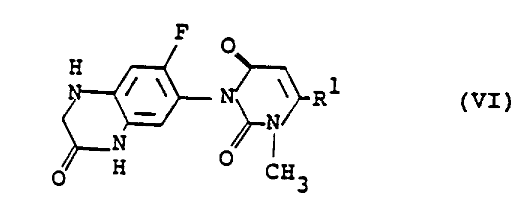 dans laquelle R¹ représente un radical trifluorométhyle ou un radical pentafluoréthyle,
caractérisé en ce que l'on soumet le composé de la formule XV 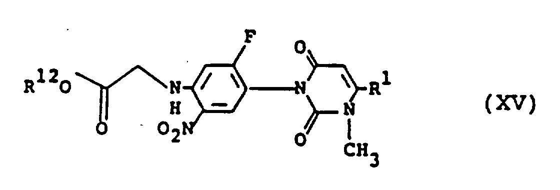 dans laquelle R¹ représente un radical alkyle en C₁ à C₆ et R¹ possède les significations qui lui ont été précédemment attribuées,
à une cyclisation réductrice.

11. Procédé de préparation d'un composé de la formule VIII 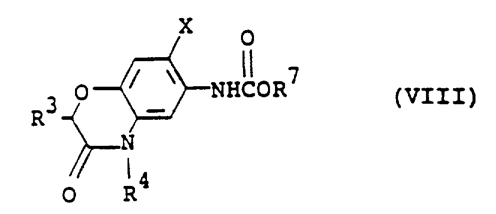 dans laquelle R³ représente un atome d'hydrogène ou un radical méthyle, R⁴ représente un radical alkyle en C₁ à C₇, un radical alcényle en C₃ à C₇, un radical alcynyle en C₃ à C₇, un radical haloalkyle en C₁ à C₆, un radical haloalcényle en C₃ à C₆ ou un radical alcoxy(C₁-C₄)alkyle(C₁-C₃), R⁷ représente un radical alkyle en C₁ à C₆ et X représente un atome d'hydrogène ou un atome de fluor, caractérisé en ce que l'on fait réagir le composé de la formule IX 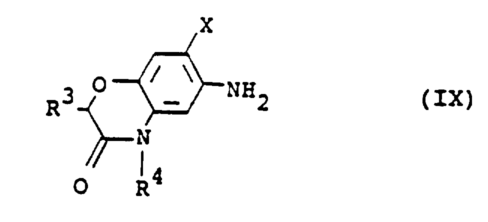 dans laquelle R³, R⁴ et X possèdent chacun les significations qui leur ont précédemment attribuées,
avec un composé de la formule XIII 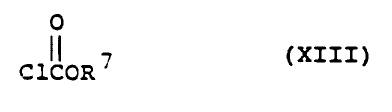 dans laquelle R⁷ représente un radical alkyle en C₁ à C₆.

12. Procédé de préparation d'un composé de la formule X 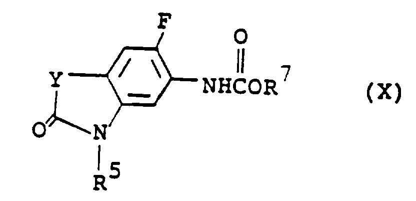 dans laquelle R⁵ représente un radical alkyle en C₁ à C₇, un radical alcényle en C₃ à C₇, un radical alcynyle en C₃ à C₇, un radical haloalkyle en C₁ à C₆, un radical haloalcényle en C₃ à C₆ ou un radical alcoxy(C₁-C₄)alkyle(C₁-C₃), R⁷ représente un radical alkyle en C₁ à C₆ et Y représente un atome d'oxygène ou un atome de soufre,
caractérisé en ce que l'on fait réagir le composé de la formule XI 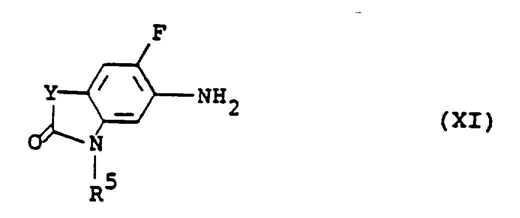 dans laquelle R⁵ et Y possèdent chacun les significations qui leur ont été précédemment attribuées,avec un composé de la formule XIII 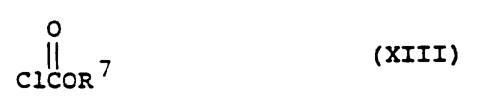 dans laquelle R⁷ représente un radical alkyle en C₁ à C₆.
